(19) Europäisches Patentamt
European Patent Office
Office européen des brevets

(11) **EP 3 970 606 A1**

(12) **EUROPEAN PATENT APPLICATION**
published in accordance with Art. 153(4) EPC

(43) Date of publication:
**23.03.2022 Bulletin 2022/12**

(21) Application number: **20805845.3**

(22) Date of filing: **13.05.2020**

(51) International Patent Classification (IPC):
*A61B 5/00* (2006.01)     *G16B 25/10* (2019.01)
*G16B 40/00* (2019.01)     *G16B 50/00* (2019.01)
*G16H 50/20* (2018.01)

(52) Cooperative Patent Classification (CPC):
**A61B 5/00; G16B 25/10; G16B 40/00; G16B 50/00;
G16H 50/20**

(86) International application number:
**PCT/KR2020/006305**

(87) International publication number:
**WO 2020/231184 (19.11.2020 Gazette 2020/47)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **14.05.2019   KR 20190056105
08.05.2020   KR 20200055087**

(71) Applicant: **Industry-University Cooperation
Foundation
Hanyang University
Seoul 04763 (KR)**

(72) Inventors:
• **KIM, Jin Hyuk**
  **Seongnam-si Gyeonggi-do 13589 (KR)**
• **KIM, Hye Young**
  **Seongnam-si Gyeonggi-do 13597 (KR)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(54) **SAMPLE ANALYSIS METHOD AND DEVICE BASED ON KERNEL MODULE IN GENOME MODULE NETWORK**

(57)     Disclosed is a sample analysis method using a kernel module in a genomic module network. The method includes a step in which an analysis device utilizes gene expression data of a sample to construct a genomic module network based on entropy and a step in which the analysis device analyzes the sample, using a kernel module of a reference genomic module network and a kernel module of the genomic module network of the sample. The kernel module is a module that is lower in entropy by a reference value or greater than the other modules in the corresponding genomic module network. The entropy represents relations between multiple genes on the basis of probabilities of transcriptional states of the multiple genes.

[FIG. 58]

**Description**

**Technical Field**

The technology described below relates to a sample analysis technique based on the kernel module of a genomic module network.

**Background Art**

The etiology of diseases such as malignant tumors is conventionally presumed to be in genomes. Thus, most studies associated with malignant tumors focus on the genome. With the advancement of molecular biology, molecular-targeted therapies have developed for selectively killing cancer cells and reducing the side effects of conventional anticancer chemotherapy. However, studies on cancer treatment are yet incomplete due to a lack of understanding of functions and mechanisms of the genome. The conventional genome studies dependent on biochemical techniques have limits on expanding the understanding of the genome, beyond the chemical reactions and structures.

**Disclosure**

**Technical Problem**

Conventional molecular biological tumor diagnosis predicts the possibility of tumor development by detecting gene mutations. However, it has not yet been proven that mutations are the cause of tumorigenesis, and there is no gene mutation that can be applied to all tumors. On the other hand, pathological tumor diagnosis is based on the morphological characteristics of tumor cells. Therefore, the effectiveness thereof has been reduced in terms of prediction of tumor development.

The technique described below is intended to provide a sample analysis technique based on the kernel module of a genomic module network constructed with a gene expression data set.

**Technical Solution**

On one aspect, there is provided a sample analysis method performed, via a sample analysis device, on the basis of a kernel module of a genomic module network, the method including: constructing a genomic module network for a sample on the basis of entropy, using gene expression data of the sample; and analyzing the sample on the basis of a reference kernel module of a reference genomic module network and a kernel module of the genomic module network of the sample.

In another aspect, there is provided a sample analysis method performed, via a sample analysis device, on the basis of a kernel module of a genomic module network, in which the sample analysis device performs the steps of: constructing a genomic module network on the basis of entropy, using gene expression data in which reference gene expression data and sample gene expression data are combined; and performing analysis on a sample on the basis of the kernel module of the genomic module network.

The reference genomic module network may be constructed in advance using at least one of a normal tissue gene expression data set and a tumor tissue gene expression data set. The kernel module may be a module having an entropy level that is lower by a reference value or more than those of other modules in the genomic module network, and the entropy level indicates relations between a plurality of genes on the basis of probabilities of transcriptional states for the plurality of genes.

In a further aspect, there is provided a sample analysis device including: an input device configured to receive reference data and gene expression data of a sample; a storage device configured to store a program for analyzing data on the basis of a kernel module of a genomic module network constructed with a gene expression data set; and a computing device configured to construct the genomic module network using the gene expression data of the sample and the program and to analyze the sample on the basis of information on genes constituting the kernel module of the constructed genomic module network.

The reference data may be at least one of a normal tissue gene expression data set and a tumor tissue gene expression data set or may be data of a reference genomic module network constructed with at least one of the normal tissue gene expression data set and the tumor tissue gene expression data set. The kernel module may be a module having an entropy level that is lower, by a reference value or more, than other modules in the genomic module network. The entropy may indicate relations between each of a plurality of genes on the basis of the probabilities of transcriptional states for the plurality of genes.

**Advantageous Effects**

The technology described below investigates the difference between normal tissue and tumor tissue, using the kernel module of the genomic module network, thereby enabling biological analysis or diagnosis for a sample on the basis of the investigation results.

**Description of Drawings**

FIG. 1 illustrates an example of a genomic module network;
FIG. 2 illustrates an example of the basis states of a genome;
FIG. 3 illustrates an example of a density matrix in a genomic space;
FIG. 4 illustrates an example of genomic modules in a matrix of basis states;
FIG. 5 illustrates an example of a method of measuring gene expression in a genomic space and a sample space;
FIG. 6 illustrates an example of a density matrix for an arbitrary module in a sample space;
FIG. 7 illustrates an example of module perturbation attributable to removal of gene i in a genetic network of an arbitrary module;
FIG. 8 illustrates an example of intermodular networks of eight different tissue types, which are constructed on the basis of a TCGA data set;
FIG. 9 illustrates an example of an intermodular networks of BRNO at various cutoffs;
FIG. 10 illustrates an example of an intermodular networks of BRNO mapped to a different type of tissue;
FIG. 11 illustrates an example of a kernel module of each of eight different tissue types;
FIG. 12 illustrates an example of the mapping of the kernel module of a normal breast tissue (BRNO) to a different type of tissue;
FIG. 13 illustrates an example of the mapping of genomic modules in a cell cycle and DNA repair (CCDR) domain of BRNO to a different type of tissue;
FIG. 14 illustrates an example of a flowchart for a genomic module network construction process;
FIG. 15 illustrates an exemplary process of generating analysis indices for sample data, using a genomic module network;
FIG. 16 illustrates an example of sample probabilities (SPs) of a plurality of tumor tissue samples, which are calculated using normal tissue genomic modules;
FIG. 17 illustrates an example of the comparison of tumor tissue samples classified on the basis of sample probabilities (SPs);
FIG. 18 illustrates an example of tumor tissue samples classified on the basis of modular sample probabilities (MSPs);
FIG. 19 illustrates an example of the average MSP of tumor sample groups for each genomic module depicted in a genomic module network of a normal tissue;
FIG. 20 illustrates an example of a density matrix of a specific gene group in a genetic space and an example of the probability of each analysis target (sample) in the specific gene group;
FIG. 21 illustrates an example of log odds ratios (LORs) of genes in each tumor tissue sample, in which the LORs are calculated using a density matrix of a specific gene group of a normal tissue;
FIG. 22 illustrates an example of the LOR calculated for each tumor tissue sample, using a genomic module of a normal tissue;
FIG. 23 illustrates an example of a flowchart for a sample data analysis method based on a filtering-based genomic module network;
FIG. 24 illustrates an example of a sample data analysis index calculation process using a filtering-based genomic module network;
FIG. 25 illustrates an example of filtering gene expression data;
FIG. 26 illustrates an example of a genomic module network generated using a filtered BRCA data set;
FIG. 27 illustrates an example of samples classified on the basis of MSPs in a specific module of the genomic module network in FIG. 26;
FIG. 28 illustrates another example of samples classified on the basis of MSPs in a specific module of the genomic module network of FIG. 26;
FIG. 29 illustrates a survival curve of a specific sample group;
FIG. 30 illustrates an example of clustering on the basis of the relatively entropy of each of eight tissue types with respect to CGX;
FIG. 31 illustrates an example of clustering on the basis of the relative entropy of each of 8 tissue types with respect to CG;

FIG. 32 illustrates an example of calculation of $MSP_{GC}$ and $MSP_{CGX}$ for BRCA samples and illustrates the distribution of $MSP_{CG}$ and $MSP_{CGX}$ according to the expression of a receptor;

FIG. 33 illustrates an example of BRNO samples clustering on the basis of the $MSP_{CG}$'s and the $MSP_{CGX}$'s;

FIG. 34 illustrates an example of the relative entropy of each of genomic modules with respect to the kernel module in an intermodular network of BRNO;

FIG. 35 illustrates an example of the representation of the relative entropy of each of genomic modules with respect to a kernel module for several samples in the intermodular network of BRNO;

FIG. 36 illustrates an example of the representation of the relative entropy of each of genomic modules with respect to the CGX of the kernel module for several samples in the intermodular network of BRNO;

FIG. 37 illustrates an example of the representation of the relative entropy of each of genomic modules with respect to CG of the kernel module for several samples in an intermodular network of BRNO;

FIG. 38 illustrates an example of clustering on the basis of MSPs of BRNO and BRCA samples, in which the MSPs are calculated with respect to CG and CGX of BRNO;

FIG. 39 illustrates an example of a level plot of clustering on the basis of MPs of BRNO and BRCA samples, in which the MPs are calculated for all genomic modules of BRNO;

FIG. 40 illustrates an example of a module with the lowest MSP depicted in a BRNO intermodular network, among the MSPs of 12 BRNO samples with respect to all BRNO genomic modules;

FIG. 41 illustrates an example of a level plot of clustering on the basis of the relative entropy of each of BN2211 modules with respect to respective BRNO modules mapped to BN2211 modules;

FIG. 42 illustrates a distribution of the MSPs of BRNO samples for modules 21, 30, 39, and 81 of BN2211.

FIG. 43 illustrates an example of a level plot of clustering on the basis of the relative entropy of each of BNRF modules with respect to BN2211 modules mapped to BNRF modules;

FIG. 44 illustrates a result of LOR calculation for the genes of modules 21, 30, 39 and 81 of BN2211;

FIG. 45 illustrates an example of a level plot of clustering on the basis of the relative entropy of each of BN2211 modules with respect to BRCA modules mapped to BN2211 modules;

FIG. 46 illustrates an example of a level plot of clustering on the basis of the relative entropy of each of BRCA modules with respect to BN2211 modules mapped to BRCA modules;

FIG. 47 illustrates an example of clustering based on the MSPs of BRCA samples for some BN2211 modules;

FIG. 48 illustrates a survival curve of each sample group of BRCA samples classified on the basis of $MSP_{CG}$ and $MSP_{CGX}$;

FIG. 49 illustrates an example of a level plot of clustering on the basis of the relative entropy of each of BAHL modules with respect to BRNO mapped to BAHL;

FIG. 50 illustrates an example of a distribution of MSPs of BRCA samples with respect to a BAHL module;

FIG. 51 illustrates an example of MSPs of BRNO samples with respect to BAHL modules 26, 32, 43, 53 and 60;

FIG. 52 illustrates a result of clustering on the basis of the MSPs of BRCA samples with respect to BAHL modules 26, 32, 43, 53 and 60;

FIG. 53 illustrates BRCA samples clustering on the basis of $MSP_{CG}$ and $MSP_{CGX}$ thereof;

FIG. 54 shows the results of linear regression analysis between the median of the mutation frequency in a BRCA sample group and the relative entropy of CG and CGX of BRCA with respect to CG and CGX of BRNO;

FIG. 55 is an example of a BRNO intermodular network in which the results of linear regression analysis on the mutation frequency of a BRCA sample group in each BRNO module are depicted;

FIG. 56 illustrates the relative entropy of each of four BRCA sample groups classified by the frequency of mutation, with respect to a BRNO module;

FIG. 57 illustrates a result of linear regression analysis on the mutation frequency and the relative entropy of BRCA with respect to BRNO modules 43 and 51;

FIG. 58 illustrates an example of a process of analyzing a kernel module of a genomic module network;

FIG. 59 illustrates an example of a process of analyzing a sample on the basis of a kernel module of a genomic module network;

FIG. 60 illustrates an example of a system for analyzing a sample, using a kernel module of a genomic module network; and

FIG. 61 illustrates an example of an analysis device that analyzes a sample, using a kernel module of a genomic module network.

**Best Mode**

Specific embodiments will be shown in the accompanying drawings and be described in detail below because the following description may be variously modified and have several example embodiments. It should be understood, however, that there is no intent to limit the following description to the particular forms disclosed, but on the contrary, the invention is to cover all modifications, equivalents, and alternatives falling within the spirit and scope of the following description.

Terms to be used in the following description will be defined first.

The term "sample" as used herein can refer to an individual living organism, and the term "individual living organism" may correspond to a person, an animal, a microorganism, or the like. However, in the following description, it is assumed that the sample is a human subject. The sample may represent a sample obtained from a subject to be analyzed. Thus, a sample has such meanings as an individual, an individual's tissue, an individual's cell set, and the like.

The term "sample data" refers to gene expression data of a sample.

The term "gene expression" as used herein refers to the transcription of a gene into an RNA product.

The term "gene expression data" as used herein refers to a set of data representing the gene expression levels of a plurality of genes of a sample, and the gene expression data may be derived via a technique such as microarrays, next generation sequencing (NGS), and the like.

The term "genomic module" or "module" as used herein refer to a group of genes of the genome of a high multicellular eukaryotic organism. One genomic module consists of a plurality of genes.

The term "modularization" as used herein refers to a process of finding a plurality of genomic modules using a gene expression data set.

The term "intermodular network" as used herein refers to a network in which a plurality of genomic modules are connected with each other by edges. In a graph data structure, a genomic module corresponds to a node, and an edge refers to a link connecting nodes.

The term "edge" as used herein refers to a connection between genomic modules in an intermodular network, and the edge can be called a channel via which the genomic modules transfer or exchange information to or with each other.

The term "genetic network" refers to a network in which genes constituting a genomic module are connected by edges. A genetic network is a network of genes within a genomic module.

The term "genomic module network" refers to a network including an intermodular network and a genetic network.

The term "domain" or "genomic module domain" as used herein refers to a specific region being composed of a plurality of genomic modules in an intermodular network.

The term "mapping" as used herein refers to an operation that overwrites data of a plurality of genes in a first genomic module composed of a first gene expression data set or in a specific module of a first genomic module network to a second gene expression data set and executes specific analysis of the genes on the basis of the second gene expression data set. In other words, some items of data of the gene expression data set to be currently analyzed (the second gene expression data set) is extracted from another gene expression data set (the first gene expression data set) and analyzed. Here, the specific analysis may mean entropy calculation, genomic module network reconstruction, and the like.

The term "genomic space" refers to a Hilbert space having a basis state vector of a genome as a coordinate axis.

The term "sample space" refers to an m-dimensional space with m samples as coordinate axes in a given data set.

The term "sample probability" (hereinafter referred to as SP) refers to the probability of each sample for a plurality of genes in the entire genomic module network. For example, the sample probability may correspond to a quantified value indicating the degree of transformation of a specific sample with respect to a normal tissue for all genes of the sample.

The term "modular sample probability" (hereinafter referred to as MSP) refers to sample probability for a plurality of genes belonging to a specific genomic module.

The term "domain sample probability" (hereinafter referred to as DSP) refers to sample probability for a plurality of genes belonging to a specific domain.

The term "log odds ratio" (hereinafter referred to as LOR) is the log value of the ratio of a first probability for a case where a specific gene is present in a genomic module to a second probability for a case where the specific gene is not present in the genomic module. The LOR can indicate the effect of a particular gene on a genomic module (genomic system).

The term "$LOR_{MSP}$" is the negative logarithm of the ratio of the MSP of a specific genomic module when a specific gene is present in a specific sample and the MSP of the specific genomic module when the specific gene is not present in the specific sample. The LOR MSP indicates the effect of a specific gene on a specific genomic module.

The term "$LOR_{DSP}$" refers to the negative logarithm of the ratio of the DSP of a specific domain when a specific gene is present in a specific sample and the DSP of the specific domain when the specific gene is not present in the specific sample. The $LOR_{DSP}$ indicates the effect of a specific gene on a specific domain.

The term "$LOR_{SP}$" refers to the negative logarithm of the ratio of the SP of a sample when a specific gene is present in a specific sample and the SP of the specific sample when the specific gene is not present in the specific sample. The $LOR_{SP}$ indicates the effect of a specific gene on a specific sample.

The term "principal eigenvector" refers to an eigenvector having the largest eigenvalue as the result of singular value decomposition (SVD).

The term "kernel module" or "kernel" refers to a module having a low entropy level than other modules, not only in the original tissue but also in other types of tissue to which the original tissue is mapped.

The entropy level indicates the degree of functional unity of multiple genes and the activity of properties.

Genomic module network construction and sample analysis based on the genomic module network may be performed in a computer. The term "computer" refers to a device having a computing ability to process input data in a predetermined way. For example, the computer may be any one of devices such as a personal computer (PC), a smart phone, a server, and a chipset in which a program is embedded. The genomic module network construction and the sample analysis based on the constructed genomic module network may be performed in one single device. Alternatively, the construction of the genomic module network and the analysis based on the constructed genomic module network may be performed in separate devices, respectively. Hereinafter, a computer that constructs a genomic module network and/or performs analysis based on the constructed genomic module network is referred to as an analysis device.

The techniques described below can elucidate the relationship between a genomic module network and a phenotype. Researchers may explore the flow of information on the transcriptional activity of a gene in a genome and investigate the gene in terms of the phenotype. The genomic module network can be utilized in various applications. Researchers may construct a genomic module network using gene expression data for a specific sample and analyze the sample using the constructed genomic module network. For example, the sample analysis may involve various test items such as whether a disease has developed, the likelihood that a disease will develop, a survival rate, a survival period, a customized treatment method for a disease, and the like.

Most conventional studies related to specific diseases (for example, malignant tumors) are based on biochemical techniques. The technology to be described below has a completely different point of view from the biochemical technique and is a technique that analyzes a sample while considering a living organism as a system.

Living organisms evolved from prokaryotes to eukaryotes and from unicellular organisms to multicellular organisms, whereby the living organisms developed into a complex structure, vertically and horizontally. Vertically, a multilayered structure was formed, and horizontally, it developed into an evolved biological system by forming complicated connections between multiple components. In general, a system is a collection of components connected to each other in an organized way. A single component or a set of some components of a system influences the characteristics of the system. Ackhoff (1972) and Checkland (1981) stated that a system expresses the characteristics of the system itself rather than the characteristics of each component or part. Similarly, in the case of a biological system, it can be said that the characteristics of the biological system itself are expressed rather than that the characteristics of proteins and genes, which are components of the biological system, are expressed. The expression of the characteristics of the biological system itself results in a phenotype. Proteins and genes can affect the phenotypes of an organism, but it is difficult to see proteins and genes as elements that build the living system itself.

The biological system expresses an appropriate phenotype to respond to internal and external environmental changes. It is only in the DNA chain that these response scenarios of the biological system be encoded. Therefore, it can be said that the whole biological system is specified by information of genes. The technique described below constructs a genomic module network using a gene expression data set of a sample and enables analysis of the sample through a system called a genomic module network.

**Construction of Genomic Module Network**

First, the process of constructing a genomic module network will be described.

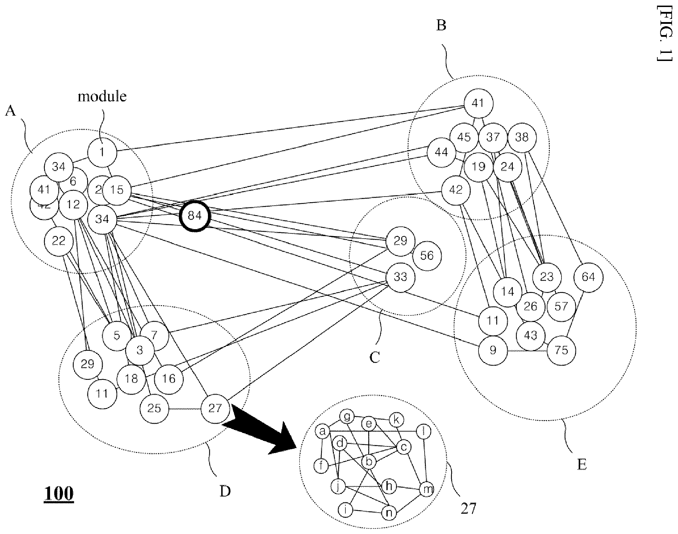

FIG. 1 illustrates an example of a genomic module network 100. Before describing the process of constructing the genomic module network 100, the structure of the genomic module network 100 will be described first. The genomic module network 100 includes an intermodular network and a genetic network. The intermodular network includes edges that connect a plurality of genomic modules to each other.

Each module of the genomic module network 100 includes predetermined genes. In FIG. 1, the module is shown by a solid-line circle. The number marked on the module is an example of an identifier used to identify the module. Genes belonging to one module correspond to genes associated with the expression of a particular phenotype. A plurality of modules may be divided into an arbitrary number of module groups each of which performs a specific function in relation to a phenotype. In FIG. 1, the module groups are indicated by respective dotted circles. The modules in one module group are related to a specific function. A domain is a group of modules related to a specific function. The domains are denoted by A, B, C, D, and E in FIG. 1. On the other hand, a module 84 serves as a relay connecting the domain A and the domain C. Domains may be associated with functions such as cell cycle, DNA damage control, epithelial tissue, extracellular matrix, immunity, angiogenesis, and the like.

FIG. 1 illustrates a state in which two modules are connected by an edge. It means that two modules connected by an edge have a certain relation with each other. Here, the edge can be considered as a channel for transferring or exchanging information between modules. When two modules are connected by an edge, it means that one of the two edgeconnected modules is related to the function of the other module. When modules respectively belonging to different domains are connected, it can be interpreted that one domain affects the function of another domain. For example, a specific phenotype can be expressed by one module, or a specific phenotype can be expressed by direct or indirect involvement of a plurality of modules.

An enlarged representation of a module 27 is illustrated at the bottom of FIG. 1. As described above, each module is composed of a plurality of genes. The genes belonging to the module 27 are marked alphabetically. The genes belonging to one module constitute a genetic network. A genetic network is a form in which genes in a module are connected by edges, similarly to a network of modules.

Hereinafter, the process of constructing a genomic module network includes a step of modularizing a genome, a step of constructing a network of genomic modules, and a step of constructing a genetic network within each module. Hereinafter, the genomic module network construction process will be described step by step.

**State of Genome**

The concept that defines the state of a gene will be explained first. The state of a gene will be described at the level of a quantum system. The quantum system is expressed in the form of a density matrix.

A gene has two basis states, active or inactive. The active state means that a gene is active in the transcription process. At a particular point in time, a gene is either active or inactive. Therefore, the basis states are mutually exclusive and mathematically orthonormal in vector space. The active state may be expressed as "1" or "on", and the inactive state may be expressed as "0" or "off". For convenience of description, active and inactive states are expressed as basis state vectors |1> and |0>, respectively. The transcriptional state vector |t> of one gene corresponds to a linear combination of two basic states as shown in Equation 1 below.

[Equation 1]

$$|t\rangle = a_0|0\rangle + a_1|1\rangle$$

In Equation 1, $a_0$ is a coefficient for an inactive state, and $a_1$ is a coefficient for an active state. The amount of mRNA produced by a gene is determined by $a_1$. The basis state vectors |1> and |0> are orthonormal.

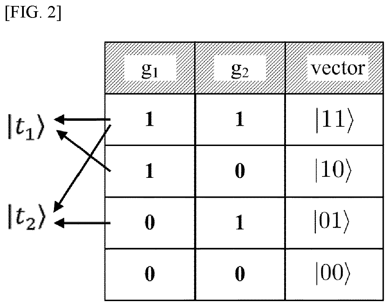

FIG. 2 illustrates an example of the basis states of genes. FIG. 2 illustrates the basis states of two genes g1 and g2. The two genes can be in four basis states. In FIG. 2, the transcriptional state vectors of the two genes g1 and g2 are expressed as $|t_1\rangle$ and $|t_2\rangle$

A probability distribution for the states of genes in a genome represents characteristic relationships between the genes. When the genes have a uniform distribution of states, it means that the genes have random activity without relation with each other. However, as the association between genes increases, the degree of nonuniformity in the distribution of states increases. Therefore, the nonuniformity of the probability distribution of gene states can be interpreted as information indicating the degree of association of genes in a genome.

A genome with n genes has a total of $2^n$ basis states. Each of the n genes has basis state vectors with orthogonal canonical properties. Therefore, the basis state vectors of a genome can be expressed as $|j_1 \cdots j_i \cdots j_n\rangle$ where $j_i \in \{0, 1\}$ and i = 1, 2 ,..., n. After all, a genome composed of n genes has $2^n$ basis state vectors $\left\{ \underbrace{|0\cdots 0\rangle, ..., |1\cdots 1\rangle}_{n} \right\}$ with orthogonal canonical properties. That is, the number of new vector spaces increases according to the number of genes. The space defined by the basis state vector of a genome is the Hilbert space. The space defined by the basis state vector of a genome is named genomic space.

The $\alpha$-th basis state among all the basis states |Ψ> is denoted by $|\Psi_\alpha\rangle$. $|\Psi_\alpha\rangle$ is expressed as $|j_{1\alpha}\cdots j_{i\alpha}\cdots j_{n\alpha}\rangle \in \left\{ \underbrace{|0\cdots 0\rangle, ..., |1\cdots 1\rangle}_{n} \right\}$ wherein $j_{i,\alpha} \in \{0,1\}$. All basis states $|\Psi_\alpha\rangle$ are orthogonal to each other. Therefore, the transcriptional state vector of a gene i can be expressed as in Equation 2 below.

[Equation 2]

$$|t_i\rangle = \sum_{\alpha=1}^{2^n} a_{i\alpha}|\psi_\alpha\rangle$$

The dyad $|t_i><t_i|$ normalized so that the diagonal trace is 1 is called a density matrix $\rho_i$ of a gene i. Since $\rho^2_i$ is equal to $\rho_i$, the density matrix represents the pure state of a genome. In a quantum system, a pure state means a state in which the state is accurately known. Considering the probabilistic characteristics of the genomic system, it is useful to describe a mixed state of a genome with an ensemble of pure states using a density matrix. Therefore, $\rho_i$ can be expressed as in Equation 3 below.

[Equation 3]

$$\rho_i = |t_i\rangle\langle t_i| = \sum_{\alpha=1}^{2^n} a_{i\alpha}^2 |\psi_\alpha\rangle\langle\psi_\alpha|$$

Thus, the mixed state density matrix $\rho$ of a genome corresponds to a combination of $\rho_i$. That is, p is $\sum_{i=1}^{n} w_i \rho_i$ where $w_i$ is the probability of $\rho_i$. When $w_i$ are all equal to 1/n, p can be expressed as $\frac{1}{n}\sum_{i=1}^{n}|t_i\rangle\langle t_i|$. Since a genomic space is a Hilbert space, the probability of a unit vector $|u>$ for a density matrix $\rho$ can be defined according to Gleason's theorem as shown in Equation 4 below.

[Equation 4]

$$\mathbf{Tr(\rho|u\rangle\langle u|)} = \langle u|\rho|u\rangle)$$

The probability that a genome stays in the $\alpha$-th basis state is $\langle \Psi_\alpha|\rho|\Psi_\alpha\rangle$. The probability that a genome is in a particular basis state lies on the diagonal of the genome's density matrix. The density matrix for a genome consisting of n genes is a square matrix with a size $2^n \times 2^n$. This density matrix has $2^n$ eigenvectors and eigenvalues. The eigen vectors represent respective eigenstates, and the eigenvalues represent the probabilities for respective states.

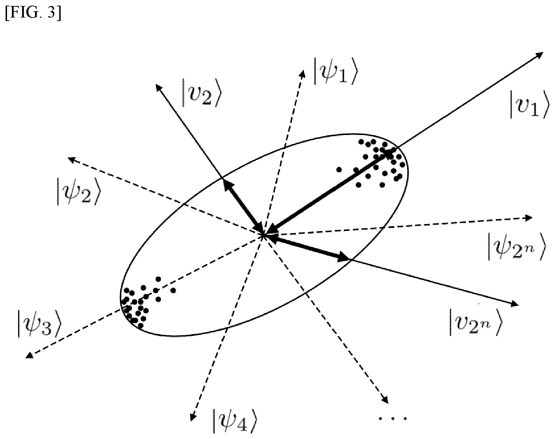

The probabilities that a genomic system will stay in respective eigenstates are not uniform. In the genomic system, this non-uniformity falls within genetic information. FIG. 3 illustrates an example of a density matrix in a genomic space. The density matrix has an elliptical shape in a twodimensional genomic space. The $2^n$ axes $|\Psi\rangle$ indicated by dotted arrows represent the basis state vectors of the genome. The $2^n$ axes $|v\rangle$ indicated by solid arrows represent eigenvectors. The length of a bold arrow indicates the probability of the eigenvector $|v\rangle$. Black dots represent respective genes.

The eigenvectors of the mixed state density matrix $\rho$ of a genome represent emergent features of a genomic system, and the eigenvalues of the eigenvectors determine the probability of feature expression. The nonuniformity can be expressed as entropy S(p). A genome has a high entropy value when the genome does not activate genes during any interaction or when the genome activates multiple genes simultaneously during many interactions. As entropy increases in a genomic space, the ellipse of the density matrix loses directionality thereof and takes on a circular shape. Conversely, when only a small number of specific targets in a genome are active, the genome has a low entropy value. Genetic information generated in a genomic space is transferred to a protein network in a real space. mRNA corresponds to a channel connecting the genomic space and the protein space.

**Genome Modularization**

Higher eukaryotes run different protein networks simultaneously, even in a single cell. It is assumed that genes involved in a particular action belong to the group. Genes belonging to a specific group work in association with each other to produce proteins that exhibit phenotypes associated with specific interactions. Therefore, genes belonging to the specific group can be defined as one module. A module is a collection of genes involved in the production of proteins related to a specific phenotype. Gene analysis for the entire genome shows that a genome can be divided into a plurality of modules. Genes belonging to a module may be directly involved in the production of proteins for a particular phenotype. In addition, genes belonging to a module may be indirectly involved in the process of producing a specific protein.

Researchers classify the entire genome into independent modules as much as possible and analyze the linkage between each of the independent modules to identify the linkages (edges) between the modules. By constructing a genomic module network for a specific genome, the inventors of the present application aim at analyzing a genome at a genomic module network level.

A plurality of modules may be cooperatively involved in the expression of a particular phenotype. From this, it is considered that a plurality of modules perform constant communication through an edge between each of the modules.

In principle, the isolation of genomic modules is possible through proper alignment of gene indices and basis states. The probability that the genome stays in a basis state is close to zero and will fluctuate in a genomic module domain.

In unicellular organisms, a single gene can only fulfill one role because it cannot have multiple different states at the same time. In other words, since mRNA maintains one level in a physically continuous space, one gene needs to be included in one genomic module.

On the other hand, in multicellular organisms, genes are expressed in physically separate spaces, respectively. Like multitasking through time division of a central processing unit (CPU) of a computer, a gene in a nucleated organism can perform multitasking through space division. This suggests one ground for the evolution of a nucleated organism into a multicellular organism.

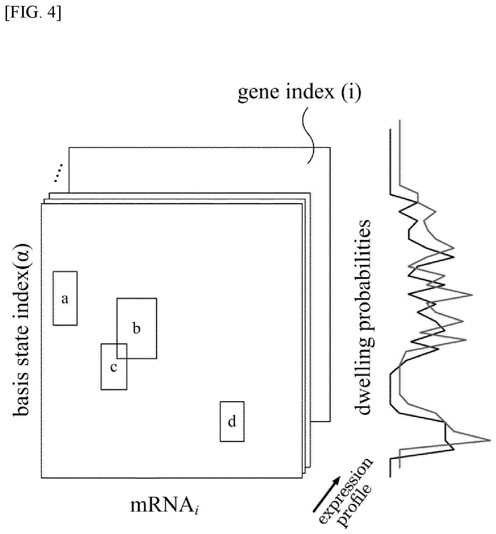

FIG. 4 illustrates an example of a genomic module located in a matrix of basis states of a genome. The vertical axis represents a basis state index, and the horizontal axis represents a gene index. A module c and a module b partially overlap.

Modules a, b, and d or modules a, c, and d can be activated in one cell (i.e., one genomic space). However, modules b and c need to be activated in different cells (multi-genomic spaces) because they share some genes.

Modules a and b partially overlap in terms of basis states, but the eigenvectors of the two modules have different directions. Thus, the two modules a and b are involved in different protein networks and phenotypes. Mutual information | (a:b) for the two modules a and b is expressed as $S(\rho_a) + S(\rho_b) - S(\rho_{ab})$. Mutual information refers to the interdependence between two modules. When the number of the basis states shared by two modules increases, $S(\rho_{ab})$ decreases, and the amount of the mutual information increases. The number of basis states shared between two modules can affect the degree of connectivity between genomic modules. However, when each genomic module has an enough complexity to express its own characteristics, the connectivity functions as a parameter in the execution of the genomic module.

A real space and a genomic space are fundamentally different from each other. The genomic space is a $2^n$-dimensional space in which the basis states of a genome are defined by a unit vector. The real space is a three-dimensional space in the actual chemical reactions such as the production of specific proteins occur through gene activity in living organisms. Therefore, it is difficult to directly access the genomic space to determine the activity of the genome. Therefore, for genome analysis, there is a need for a method of converting the genomic space into a sample space for gene expression data. The sample space is an m-dimensional space in which each sample is defined as a unit vector.

The measurement of gene expression of m samples translates information carried on mRNA in a genomic space into information in a m-dimensional sample space. On the other hand, technologies such as cDNA microarrays can measure expression levels of tens of thousands of genes at the same time.

In a genome with n genes, the transcriptional state vector $|t_i>$ of gene i constitutes a transcriptional state $n{\times}2^n$ matrix T . The pure state vector $|g_1>$ of a gene i in n gene expression data sets measured from m samples constitutes a matrix $\overset{n{\times}m}{\mathbf{G}}$ . The relationship between the two matrices is shown in Equation 5 below.

$$[\text{Equation 5}]$$

$$\overset{n{\times}m}{\mathbf{G}} = \overset{n{\times}2^n}{\mathbf{T}}\overset{2^n{\times}m}{\mathcal{M}}$$

Here, $\overset{2^n{\times}m}{\mathcal{M}}$ is a transformation matrix for transforming a genomic space into a sample space.

According to Equation 3 above, the density matrix ρ of the entire genome and the transcription state matrix T have a relationship of $\overset{2^n{\times}2^n}{\rho} = \overset{2^n{\times}n}{\mathbf{T}^{\top}}\overset{n{\times}2^n}{\mathbf{T}}$ . Since the density matrix ρ is a real symmetric matrix, the result $\rho = \mathbf{Q}{\wedge}\mathbf{Q}^{\top}$ can be obtained through eigen decomposition. In the result, Q is an orthogonal normal matrix in which each column is each eigenvector of the density matrix p, and ∧ is a diagonal matrix in which each diagonal component is an eigenvalue of the density matrix p. Therefore, when $\mathbf{G}^{\top}\mathbf{G}$ is deployed from Equation 5, $\mathbf{G}^{\top}\mathbf{G} = M^{\top}(\mathbf{T}^{\top}\mathbf{T})M = M^{\top}\rho M = M^{\top}(\mathbf{Q}{\wedge}\mathbf{Q}^{\top})M$ is established.

In addition, when a transformation matrix is decomposed into SVD, the result is $\overset{2^n{\times}m}{\mathcal{M}} = \overset{2^n{\times}2^n}{\mathbf{U}}\,\overset{2^n{\times}m}{\mathbf{\Sigma}}\,\overset{m{\times}m}{\mathbf{V}^{\top}}$ wherein $\overset{2^n{\times}2^n}{\mathbf{U}}$ a left singular vector matrix, $\overset{2^n{\times}m}{\mathbf{\Sigma}}$ is a singular value matrix, and $\overset{m{\times}m}{\mathbf{V}^{\top}}$ is a right singular vector matrix. Therefore, $\mathbf{G}^{\top}\mathbf{G} = \mathbf{V}\Sigma^{\top}\mathbf{U}^{\top}(\mathbf{Q}{\wedge}\mathbf{Q}^{\top})\mathbf{U}\Sigma\mathbf{V}^{\top} = \mathbf{V}\Sigma^{\top}\mathbf{Q}'{\wedge}\mathbf{Q}'^{\top}\Sigma\mathbf{V}^{\top}$ is established. Since $\mathbf{Q}' = \mathbf{U}^{\top}\mathbf{Q}$, Q' is a new matrix of eigenvectors obtained by rotating each eigenvector of the density matrix ρ of the entire genome with the left singular vector matrix of the transformation matrix *M.*

On the other hand, when the proposition that von Neumann entropy is invariant is applied to unitary transformation, $S(\mathbf{VE}^{\top}\mathbf{Q}'{\wedge}\mathbf{Q}'^{\top}\mathbf{EV}^{\top}) = S(\Sigma^{\top}\mathbf{Q}'{\wedge}\mathbf{Q}'^{\top}\Sigma)$ is established.

That is, the entropy of $\mathbf{G}^{\mathsf{T}}\mathbf{G}$, $S\left(\overset{m \times n}{\mathbf{G}^{\mathsf{T}}} \overset{n \times m}{\mathbf{G}}\right) = S\left(\overset{m \times 2^n}{\mathbf{\Sigma}^{\mathsf{T}}} \overset{2^n \times 2^n}{\mathbf{Q}'} \overset{2^n \times 2^n}{\mathbf{\Lambda}} \overset{2^n \times 2^n}{\mathbf{Q}'^{\mathsf{T}}} \overset{2^n \times m}{\mathbf{\Sigma}}\right)$ is established. Here, $\mathbf{Q}'\Lambda\mathbf{Q}'^{\mathsf{T}}$ means that the density matrix $\rho$ only rotates and the eigenvalue matrix $\Lambda$ remains unchanged. The diagonal component "$\Lambda$", that is, the eigenvalue of p is $\lambda_1 > \lambda_2 > \cdots > \lambda_{2^n} > 0$, and when the entropy is low, the eigenvalue decreases rapidly. That is, $\sum_{\alpha=m+1}^{2^n} \lambda_\alpha \approx 0$ can be assumed. When a diagonal matrix $\overset{m \times m}{\mathbf{\Lambda}'}$ is created with only the first m eigenvalues of $\Lambda$, then $\overset{2^n \times 2^n}{\mathbf{Q}'} \overset{2^n \times 2^n}{\mathbf{\Lambda}} \overset{2^n \times 2^n}{\mathbf{Q}'^{\mathsf{T}}} \approx \overset{2^n \times m}{\mathbf{Q}''} \overset{m \times m}{\mathbf{\Lambda}'} \overset{m \times 2^n}{\mathbf{Q}''^{\mathsf{T}}}$ is established. Here, $\overset{2^n \times m}{\mathbf{Q}''}$ is a matrix composed of only the first m eigenvectors among the $2^n$ eigenvectors in $\overset{2^n \times 2^n}{\mathbf{Q}'}$.

When all samples are obtained from the same type of tissue, the first m rows in the singular value matrix $\overset{2^n \times m}{\mathbf{\Sigma}}$ of the transformation matrix $\boldsymbol{M}$ are filled with 1's and the remainder is filled with 0's. Therefore, $\overset{m \times 2^n}{\mathbf{\Sigma}^{\mathsf{T}}} \overset{2^n \times 2^n}{\mathbf{Q}'} \overset{2^n \times 2^n}{\mathbf{\Lambda}} \overset{2^n \times 2^n}{\mathbf{Q}'^{\mathsf{T}}} \overset{2^n \times m}{\mathbf{\Sigma}} \approx \overset{m \times 2^n}{\mathbf{\Sigma}^{\mathsf{T}}} \overset{2^n \times m}{\mathbf{Q}''} \overset{m \times m}{\mathbf{\Lambda}'} \overset{m \times 2^n}{\mathbf{Q}''^{\mathsf{T}}} \overset{2^n \times m}{\mathbf{\Sigma}} = \overset{m \times m}{\mathbf{W}} \overset{m \times m}{\mathbf{\Lambda}'} \overset{m \times m}{\mathbf{W}^{\mathsf{T}}}$ is established. Here, W is a matrix created by extracting the first m rows from the matrix $\overset{2^n \times m}{\mathbf{Q}''}$.

On the other hand, since $\overset{2^n \times 2^n}{\mathbf{Q}'} = \overset{2^n \times 2^n}{\mathbf{U}^{\mathsf{T}}} \overset{2^n \times 2^n}{\mathbf{Q}}$, $|q_i'\rangle = \mathbf{U}^{\mathsf{T}}|q_i\rangle$ is established between the column vectors $|q_i'\rangle$ and $|q_i\rangle$ that constitute Q' and Q, respectively. Here, Q' is a matrix obtained by rotating Q with respect to U. Therefore, the orthonormality between each eigenvector is maintained. Therefore, when i = j, $\langle q_i'|q_j'\rangle$ is 1 whereas when i $\neq$ j, $\langle q_i'|q_j'\rangle = \langle q_i|\mathbf{U}\mathbf{U}^{\mathsf{T}}|q_j\rangle$ is established. In constructing the vector $\overset{2^n \times m}{\mathbf{U}'}$ using the first m singular vectors selected from among the $2^n$ singular vectors constituting the matrix $\overset{2^n \times m}{\mathbf{U}'}$, since the most important singular vector of the transformation vector $\boldsymbol{M}$ are used, $\langle q_i'|q_j'\rangle \approx \langle q_i|\mathbf{U}'\mathbf{U}'^{\mathsf{T}}|q_j\rangle$ is established. Here, since $\mathbf{U}'^{\mathsf{T}}|q_j\rangle$ is the same as the column vector $|w_j\rangle$ of the matrix W, $\langle q_i'|q_j'\rangle$ is established. Therefore, the matrix W can be considered as an orthogonal normal matrix.

In addition, the sum of the diagonal components of $\Lambda'$ (i.e., the first m eigenvalues of $\Lambda$) approaches 1. That is, $\mathbf{W}\Lambda'\mathbf{W}^{\mathsf{T}}$ is considered a density matrix obtained by transforming the density matrix $\rho$ from the $2^n$-dimensional genomic space to the m-dimensional sample space. Therefore, an approximate value of the entropy of $\mathbf{G}^{\mathsf{T}}\mathbf{G}$ is expressed as in Equation 6 below.

[Equation 6]

$$S(\mathbf{G}^{\mathsf{T}}\mathbf{G}) \approx S(\mathbf{W}\Lambda'\mathbf{W}^{\mathsf{T}})$$

Here, $S(\mathbf{W}\Lambda'\mathbf{W}^{\mathsf{T}}) = -\sum_{\alpha=1}^{m} \lambda_\alpha \log \lambda_\alpha$ and it approximates $-\sum_{\alpha=1}^{2^n} \lambda_\alpha \log \lambda_\alpha$. That is, Equation 7 shown below can be obtained.

[Equation 7]

$$S(\mathbf{G}^{\mathsf{T}}\mathbf{G}) \approx S(\mathbf{T}^{\mathsf{T}}\mathbf{T})$$

Equation 7 means that the entropy calculated from data obtained by measuring gene expression is almost identical to the entropy of the transcriptional state of the genome.

From Equation 4, the probability of the gene i for the entire genome in the genomic space is $\langle t_i|\mathbf{T}^{\mathsf{T}}\mathbf{T}|t_i\rangle$. However, the density matrix $\rho$ of the genomic system and the transcriptional state of the gene i cannot be directly confirmed, and only the gene expression data G is known. Therefore, finding a way to calculate the probability from G is an important process. First, the eigenvalue decomposition of $\mathbf{T}^\top\mathbf{T}$ is performed, $\langle t_i|\mathbf{T}^\top\mathbf{T}|t_i\rangle = \langle t_i|\mathbf{Q}\Lambda\mathbf{Q}^\top|t_i\rangle$ is established. Here, $\mathbf{Q}^\top|t_i\rangle$ is a vector obtained by transforming the gene transcription state vector $|t_i\rangle$ into a coordinate system using the eigenvector of the density matrix of the genome as the basis vector and can be substituted with $|t_i^*\rangle$. Therefore, $\langle t_i|\mathbf{T}^\top\mathbf{T}|t_i\rangle = \langle t_i^*|\Lambda|t_i^*\rangle = \sum_{\alpha=1}^{2^n} t_{i\alpha}^* \lambda_\alpha t_{i\alpha}^*$ is established.

On the other hand, the pure state of the gene in the sample space is as shown in Equation 8 below.

[Equation 8]

$$|g_i\rangle = \mathcal{M}^\top|t_i\rangle$$

In the sample space, the probability of each gene for $\mathbf{G}^\top\mathbf{G}$ is $\langle g_i|\mathbf{G}^\top\mathbf{G}|g_i\rangle$, whereby $\langle g_i|\mathbf{G}^\top\mathbf{G}|g_i\rangle = \langle t_i|\mathbf{M}\mathbf{M}^\top\mathbf{T}^\top\mathbf{T}\mathbf{M}\mathbf{M}^\top|t_i\rangle$ is established from Equation 5. Here, when the eigenvalue decomposition ( $\mathbf{T}^\top\mathbf{T} = \mathbf{Q}\Lambda\mathbf{Q}^\top$ ) of $\mathbf{T}^\top\mathbf{T}$ is applied, and the decomposition ( $\mathbf{M} = \mathbf{U}\Sigma\mathbf{V}^\top$ )) of the transformation matrix $\mathbf{M}$ is performed using the SVD, $\langle g_i|\mathbf{G}^\top\mathbf{G}|g_i\rangle = \langle t_i|\mathbf{U}\Sigma\mathbf{V}^\top\mathbf{V}\Sigma^\top\mathbf{U}^\top\mathbf{Q}\Lambda\mathbf{Q}^\top\mathbf{U}\Sigma\mathbf{V}^\top\mathbf{V}\Sigma^\top\mathbf{U}^\top|t_i\rangle$ is established. Here, since the singular vector of the transformation matrix $\mathbf{M}$ is orthogonally normal, the equations $\mathbf{V}^\top\mathbf{V} = \mathbf{I}$ and $\Sigma^\top\mathbf{U}^\top\mathbf{Q}\Lambda\mathbf{Q}^\top\mathbf{U}\Sigma \approx \mathbf{W}\Lambda'\mathbf{W}^\top$ are applied, $\langle g_i|\mathbf{G}^\top\mathbf{G}|g_i\rangle \approx \langle t_i|\mathbf{U}\Sigma\mathbf{W}\Lambda'\mathbf{W}^\top\Sigma^\top\mathbf{U}^\top|t_i\rangle$ is established. In addition, $\Sigma^\top\mathbf{U}^\top|t_i\rangle$ is a vector obtained when $|t_i\rangle$ (dimension $2^n \times 1$) undergoes transformation into a coordinate system using the singular vector U of the transformation vector $\mathbf{M}$ as a basis vector and then undergoes dimensionality reduction using $\Sigma^\top$ (dimension $m \times 2^n$), and $\Sigma^\top\mathbf{U}^\top|t_i\rangle$ can be substituted with $|t_i'\rangle$ (dimension $m \times 1$). Therefore, in the sample space, the probability of each gene for $\mathbf{G}^\top\mathbf{G}$ is as shown in Equation 9 below.

[Equation 9]

$$\langle g_i|\mathbf{G}^\top\mathbf{G}|g_i\rangle \approx \langle t_i'|\mathbf{W}\Lambda'\mathbf{W}^\top|t_i'\rangle$$

Here, $\langle t_i'|\mathbf{W}\Lambda'\mathbf{W}^\top|t_i'\rangle = \sum_{\alpha=1}^{m} t_{i\alpha}' \lambda_\alpha t_{i\alpha}'$, and the value approximates $\sum_{\alpha=1}^{2^n} t_{i\alpha}^* \lambda_\alpha t_{i\alpha}^*$. In conclusion, Equation 10 shown below can be obtained.

[Equation 10]

$$\langle g_i|\mathbf{G}^\top\mathbf{G}|g_i\rangle \approx \langle t_i|\mathbf{T}^\top\mathbf{T}|t_i\rangle$$

Equation 10 means that the probability of a gene calculated from data obtained by measuring gene expression is almost identical to the probability calculated from the density matrix of the genome and the transcriptional state of the gene.

To determine the state of the genome or gene directly from the measured expression level $|g_i\rangle$, $\mathbf{M}$ is necessary.

On the other hand, in the process of measuring gene expression, selection of a sample in time or sample space, measurement method, data processing, etc. are factors influencing the process. Therefore, the transformation matrix $M$ can be affected by many factors, and even under the same conditions, the influence on each gene is different. In conclusion, $|g_i\rangle$ is affected by the experimental conditions or environment. This means that gene expression data are in principle inconsistent. There is a limit to overcoming the vulnerability of these data using statistical or experimental methods.

Equation 4, Equation 8, and Equation 10 shown above mean the following fact. As to the probability of a gene i for a genomic module, represented by the density matrix p, the probability $\langle t_i|\rho|t_i\rangle$ in the genomic space is equal to the probability $\langle g_i|\mathbf{G}^\top\mathbf{G}|g_i\rangle$ in the sample space. Furthermore, the entropy S(p) in the genomic space becomes equal to the entropy $S(\mathbf{G}^\top\mathbf{G})$ in the sample space. This proves that the probability and the entropy are parameters that are not affected by the measurement environment depending on the difference in gene expression level. It is difficult to obtain a perfect transformation matrix $M$ for measuring the genome of eukaryotes, but the entropy and probability can be obtained without considering the measurement process.

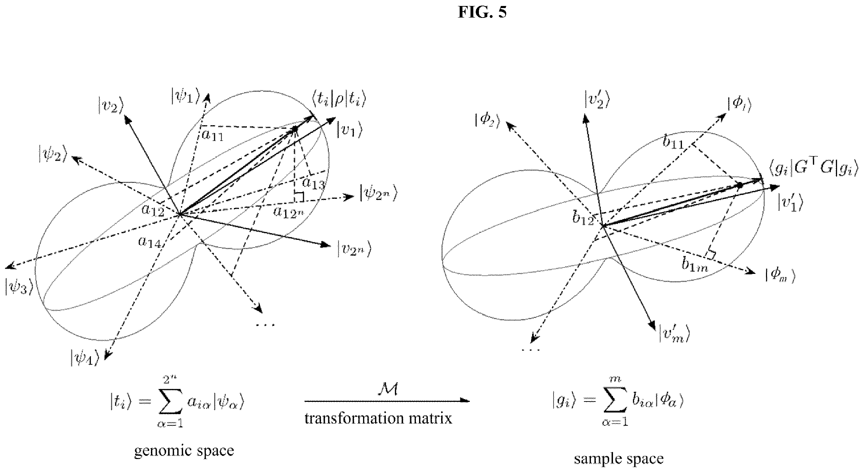

FIG. 5 illustrates an example of measurement of gene expression shown in a genomic space in a sample space. This is an example in which a density matrix $\rho$ of a genomic module and a gene transcription state vector $\rho$ in a $2^n$-dimensional genomic space composed of basis vectors $|\Psi\rangle$ are respectively transformed into $\mathbf{G}^\mathrm{T}\mathbf{G}$ and gene expression vectors $|g_i\rangle$ and a density matrix $\rho$ in an m-dimensional sample space composed of basis vectors $|\phi\rangle$. Here, since $\langle t_i|\rho|t_i\rangle = \langle g_i|\mathbf{G}^\mathrm{T}\mathbf{G}|g_i\rangle$ is established, the probability of a gene for a module is not affected by the transformation from the genomic space to the sample space for the measurement of gene expression.

When the vectors of all genes in the sample space have the same direction, the entropy is zero. This means that the elliptical density matrix is a straight line that coincides with the first eigenvector. When the probability of a gene is the same for all eigenvectors and thus the density matrix is a perfect circle (or sphere), the entropy has a maximum value.

Hereinafter, an example of a process of constructing the genomic module network using actual gene expression data will be described. A tumor is assumed to be a small, independent system that resides in a large host system. Therefore, genomic module network will be constructed using genetic information about tumors.

A gene expression data set included data of six types of tumor tissue, including breast cancer (BRCA), colon adenocarcinoma (COAD), rectal adenocarcinoma (READ), lung adenocarcinoma (LUAD), lung squamous cell carcinoma (LUSC), and ovarian cancer (OV) and data of two types of normal tissue including normal breast tissue (BRNO) and normal colon tissue (CONO). The gene expression data set also includes the arbitrary mixes (X6CA) of the data of the six types of tumor tissue, mixes (X2NO) of the data of the two types of normal tissue, and arbitrary mixes (X6C2N) of the data of the six types of tumor tissue and the data of the two types of normal tissue. BRCA, etc. refers to a data set that is publicly available for academic research and is obtained by measuring the amount of gene expression in the corresponding tissue, in which the measurement is performed by the Cancer Genome Atlas (TCGA). To reduce the computation time, 36 samples were randomly selected from each of BRCA, COAD, LUSC, and OV, each of which included more than 36 samples. Genomic modules were isolated using the data set.

The process of constructing a genomic module with the prepared gene expression data set will be described. Among the above-mentioned contents, the contents necessary for modularization will be briefly described. In n completely independent (with no connectivity at all) modules, respective density matrices are represented by $\rho_1,......,$ and $\rho_n$. Since each space is a Hilbert space, the overall density matrix is $\rho = \rho_1 \otimes \cdots \otimes \rho_n$. Accordingly, the total entropy is equal to the sum of the entropy of each module. That is, $S(\rho) = S(\rho_1 \otimes \cdots \otimes \rho_n) = \sum_{i=1}^{n} S(\rho_i)$.

However, when n modules are not independent of each other (i.e., when connectivity between modules exists), the overall entropy becomes smaller than the sum of the entropy of each module. That is, $S(\rho) < \sum_{i=1}^{n} S(\rho_i)$.

One module affects other modules, and certain information is exchanged between modules. One module contains genes, and there may be a case where different modules share the same gene. Therefore, it is difficult for each module to function completely independently in a genomic module network. In this case, the criterion for module separation is to find a module that minimizes the difference between the sum of the entropy of each module and the overall entropy.

However, an analysis device has no idea about the actual number of modules that are activated in the genome, no idea about the range of participating genes, and no idea about genes participating in multiple modules at the same time. Therefore, it is impossible to find a combination of modules in the method described above. As a solution to this problem, the analysis device may identify the local optimal points of a true module that exists and construct a module around them to complete an estimation module. In this process, the analysis device generates estimation modules close to the intrinsic module by preventing the transition to another local optimal point. The outer limit of the estimation modules overlapping each other to a considerable extent coincides with a domain, which is a connected group of intrinsic modules expressing phenotypes in a large category. This is because a domain that regulates a large category of phenotypes has only a small number of information exchange channels with other domains (max-flow min-cut).

Table 1 below is an example of a pseudo code for an algorithm for finding a local optimal point for a genomic module. Genes are divided into an arbitrary number of sets, and genes are removed one by one from each set (module) of genes to reduce the entropy to a target value, thereby finding the local optimum point. The entropy target is set low enough because it is necessary to find the local optimum point that exists in the actual module. In Table 1, "th" corresponds to a threshold value that is the target value. In Table 1, the backslash "\" operation means an operation that removes a right element from a left set.

[Table 1]

---

**Exploration of a local optimum for a genomic module**

---

**Require:** a gene set $M_i = \{1_i, \ldots, u_i\}$, where $1_i, \ldots, u_i \in \{1, \ldots, n\}$

**Require:** $th$

  **for** gene $j_i \in M_i$ **do**

    **if** $\mathcal{S}(\rho(M_i \setminus j_i)) > th$ **then**

      $M_i = M_i \setminus j_i$

    **end if**

  **end for**

  **return** $M_i$

---

The genomic module is finally determined using the local optimum found through the process in Table 1 above. Table 2 below is an example of the algorithm for the process of completing the genomic module.

[Table 2]

---

**Buildup of a genomic module**

---

**Require:** a gene set $M_i = \{1_i, \ldots, u_i\}$, where $1_i, \ldots, u_i \in \{1, \ldots, n\}$

**Require:** $th$

  **for** gene $j \notin M_i$ **do**

    **if** $\mathcal{S}(\rho(M_i \cup j)) \leq \mathcal{S}(\rho(M_i))$ and $\mathrm{v}_1(\rho(M_i \cup j)) \cdot \mathrm{v}_1(\rho(M_i)) \leq th$ **then**

      $M_i = M_i \cup j$

    **end if**

  **end for**

  **return** $M_i$

---

Table 2 expands the module by adding foreign genes j one by one under the condition that entropy does not increase. To prevent the center of the module from moving during the module expansion process, the fluctuation in the direction of the principal eigenvector $v_1$ is limited. In Table 2, "th" means a threshold value for the variation angle of the principal eigenvector.

In this case, the optimal parameters such as the target value of entropy in Table 1 and the fluctuation angle of the principal vector in Table 2 depend on the characteristics of the gene expression data. Therefore, it may be necessary to construct a genomic module network and identify a domain on the basis of the results obtained with various parameters, thereby determining the optimal parameters showing consistent results.

In general, low entropy refers to a system that focuses on a specific target represented by the first eigenvector of a density matrix. In the genomic system of a eukaryotic cell, it is known that a genomic module with low entropy generates information for expressing a specific phenotype.

Some of the genes constituting a genomic module also may appear in other modules. This is because the condition was set such that the change in the first eigenvector change was lower than a certain threshold when constructing the genomic module.

When the analysis device performs the operations described in Tables 1 and 2 on the basis of the input gene expression data set, thereby constructing genomic modules.

The process of constructing a genetic network in a genomic module will be described first, and then the process of constructing an intermodular network by connecting the edges of genomic modules will be described.

**Construction of Genetic Network**

As described above, the genomic module is composed of a plurality of genes. Genes present in one module constitute a network for exchanging information. This is named a genetic network in the following description.

A genomic module represents a program unit in a eukaryotic genome. As described above, a module constitutes a specific unit in the entire program for living things. Here, the program refers to the process required to run the system called a living organism. The genetic network present in a genomic module is an element that connects genes in a specific point of view.

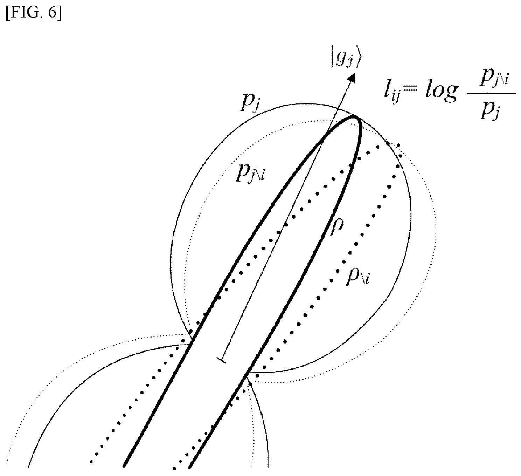

FIG. 6 illustrates an example of a density matrix for an arbitrary module in a sample space. FIG. 6 shows the density matrix ρ of a certain module and the expression vector $|g_j\rangle$ of a certain gene j contained in the module. The thick solid line represents the density matrix of the module. The plain (thin) solid line represents the probability trajectory of the unit vector for the density matrix. The dotted line represents the perturbation attributable to the exclusion of a gene i. The term "perturbation" refers to a motion that occurs in a mechanical system as the motion caused by the action of a major force is disturbed by the effect of a secondary force. Since $|g_j\rangle$ is the normalized expression vector of a gene j, the probabilities for the density matrix are on the corresponding loci.

A certain module is perturbed by the exclusion of a gene i. The density matrix of the perturbed module is represented by $\rho_{\backslash i}$. When the gene i is excluded and the module is perturbed, the density matrix rotates slightly in a sample space, and the elliptical shape narrows or widens slightly. The probability of another gene j in the density matrix changes from $P_j$ to $P_{j\backslash i}$. When the gene j is strongly linked to the gene i, the probability of the gene j is greatly reduced by the perturbation.

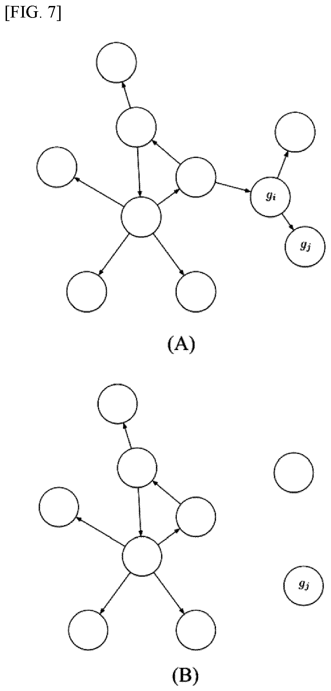

FIG. 7 illustrates an example of module perturbation caused by the exclusion of a gene i in a genetic network of a certain module. 7A shows a genetic network before the exclusion of a gene i in a certain module, and 7B shows genetic network after the exclusion of the gene i. When the gene i is removed from a module, a gene j linked only to the gene i is isolated from the module. Therefore, the decrease in the probability of the gene j increases in the module where the gene i is removed. On the other hand, when the gene is not linked to the gene i, or when the gene j is linked to another gene aside from the gene I, the decrease in the probability of the gene j decreases. Therefore, the linkage between the gene i and the gene j can be estimated on the basis of the LOR between the probability of the gene j before the removal of the gene i and the probability of the gene j after the removal of the gene i.

The odds ratio of probabilities can quantitatively express the influence between two genes. The log odds ratio (LOR) of the probability is equal to the difference in information content. Equation 11 below indicates the degree of probability change ($l_{ij}$) of the gene j belonging to the same module when the gene i is excluded from the module.

[Equation 11]

$$l_{ij} = \log \frac{p_{j\backslash i}}{p_j}$$

The $l_{ij}$ for all possible gene pairs in the genomic module is calculated. When the $l_{ij}$ for any two genes (i and j) exceeds a certain threshold, it is determined that the gene i and the gene j have strong connectivity. Genes with strong connectivity are connected by edges. In this way, a genetic network can be constructed by calculating the above-described $l_{ij}$ among all the genes present in the genomic module. For example, FIG. 6 shows kernel modules for eight tissue types, and a genetic network for each module is completed using the above-described method.

Table 3 below is a pseudocode for the process of constructing a genetic network in a module. Briefly describing, an LOR is calculated for each gene pair belonging to an arbitrary module, and an adjacency matrix is generated based on the result of the calculation. The LOR between the gene i and each of all other genes is extracted from the adjacency matrix to calculate the quartile, and the internal threshold value $th_i$ of the gene i is calculated using the cutoff value. For each gene, the process of assigning an edge to a gene pair having an LOR greater than or equal to the internal threshold is repeated.

[Table 3]

---

**Reconstruction of genetic network within a genomic module**

---

Require: isolated gene set $\{g_i\}$ as a genomic module, where $i = 1, \ldots, n$
Require: the log odds ratio matrix L
  for $i \leq n$ do
    for $j \leq n$ do

      Probability of gene j for the modular system: $p_j = \langle g_j|\rho|g_j\rangle$
      Probability of gene j for the modular system without gene i: $p_{j\backslash i} =$
      $\langle g_{j\backslash i}|\rho_{\backslash i}|g_{j\backslash i}\rangle$
      Log odds ratio: $l_{ij} = log\ \dfrac{p_{j\backslash i}}{p_j}$   {$l_{ij}$ is an element of the matrix L}
    end for
  end for
Require: adjacency matrix A, cutoff value c
  for $i \leq n$ do
    $q_i$ = quartile $(l_{i\cdot})$
    $th_i = q_{i,50\%} - c \cdot (q_{i,75\%} - q_{i,25\%})$
    for $j \leq n$ do
      if $l_{ij} \leq th_i$ then
        $a_{ij} = l_{ij}$   {$a_{ij}$ is an element of the matrix A}
      else if $l_{ij} > th_i$ then
        $a_{ij} = 0$
      end if
    end for
  end for
  return A

---

**Intermodular Network**

When a genetic network is a program operated by genes, the program structure of an organism can be represented as a network between modules. As described above, there is an edge between modules in a network of genomic modules. Here, the edge means that the modules have a certain association or connection with each other. The edge can also be seen as a channel through which modules transmit or exchange certain information.

The process of configuring an intermodular network will be described. The relative entropy is measured for all possible module pairs extracted from the genetic data set. The description will be made using a module i and a module j as exemplary modules. The relative entropy means the information gain that module i has with respect to module j. The relative entropy can be expressed as $S(\rho_i\|\rho_j) = Tr(\rho_i ln\rho) - Tr(\rho_i ln\rho_j)$ where $\rho_i$ and $\rho_j$ denote the density matrices for the module i and the module j, respectively. The relative entropy is always non-negative and non-transmutable. That is, $S(\rho_i\|\rho_j) \neq S(\rho_j\|\rho_i)$ is established. The relative entropy is used as information to construct an intermodular network. When two density matrices are the same, the relative entropy is zero. When the difference between two density matrices is large, the relative entropy value is also large.

In addition, the relative entropy is also used in the process of identifying similarities between modules isolated from different tissue types. Modules isolated from different tissues cannot be directly compared because the sample spaces thereof are completely different. The relative entropy calculated by mapping modules of one tissue to modules of another tissue represents the difference between the density matrices in the same sample space.

When the module i has a low information gain compared to the module j, it can be said that the module i and the module j are highly correlated with each other. In this case, a network is established by connecting the module i and the module j with an edge.

To increase the resolution of the relative entropy at a low level, a negative log may be applied to the relative entropy. The logarithmic relative entropy is $nlr_{ij} = -log(r_{ij})$. Here, $r_{ij} = S(\rho_i\|\rho_j)$, $r_{ij} > 0$, and $i \neq j$. Table 4 below is an example of a pseudocode for an algorithm for building an intermodular network.

[Table 4]

| Reconstruction of intermodular network |
| --- |

```
Require: genomic module set {Mᵢ}, where i = 1, ... , n
Require: the negative log relative entropy matrix N
    for i ≤ n do
        Density matrix of module i: ρᵢ = ρ(Mᵢ)
        for j ≤ n do
            Density matrix of module j: ρⱼ = ρ(Mⱼ)
            Relative entropy: rᵢⱼ = Tr(ρᵢ ln ρᵢ) - Tr(ρⱼ ln ρⱼ)
            Negative log relative entropy: nlrᵢⱼ = -log(rᵢⱼ) {nlrᵢⱼ is an element of
            the matrix N}
        end for
    end for
Require: adjacency matrix A, cutoff value c
    for i ≤ n do
        qᵢ = quartile(nlrᵢ.)
        thᵢ = qᵢ,₅₀% + c · (qᵢ,₇₅% - qᵢ,₂₅%)
        for j ≤ n do
            if nlrᵢⱼ > thᵢ and nlrᵢⱼ > 0 then
                aᵢⱼ = nlrᵢⱼ {aᵢⱼ is an element of the matrix A}
            else if lᵢⱼ ≤ thᵢ or nlrᵢⱼ ≤ 0 then
                aᵢⱼ = 0
            end if
        end for
    end for
    return A
```

For a given module i, a predetermined threshold is used to determine the association with another counterpart module j. An edge between the module i and the module j connects to the module i and the module j when the $nlr_{ij}$ between the module i and the module j does not exceed a predetermined threshold. With respect to the cutoff $C_f$, an appropriate threshold value to determine an intermodular edge needs to be found. For example, as shown in Table 4, the first quartile Q1, the second quartile Q2, and the third quartile Q3 of the $nlr_{ij}$ may be used.

An information exchange pattern between genomic modules may be represented as an intermodular network. As described above, the relative entropy between the genomic modules measured in the sample space can be used to determine connection between modules.

The relative entropies may be measured between all possible genomic modules, and an adjacency matrix consisting of relative entropy values that do not exceed a threshold value calculated using the cutoff may be prepared. An intermodular network can be constructed using the adjacency matrix. When constructing an intermodular network using the adjacency matrix calculated by reducing the cutoff, the module connection order depends on the tissue type.

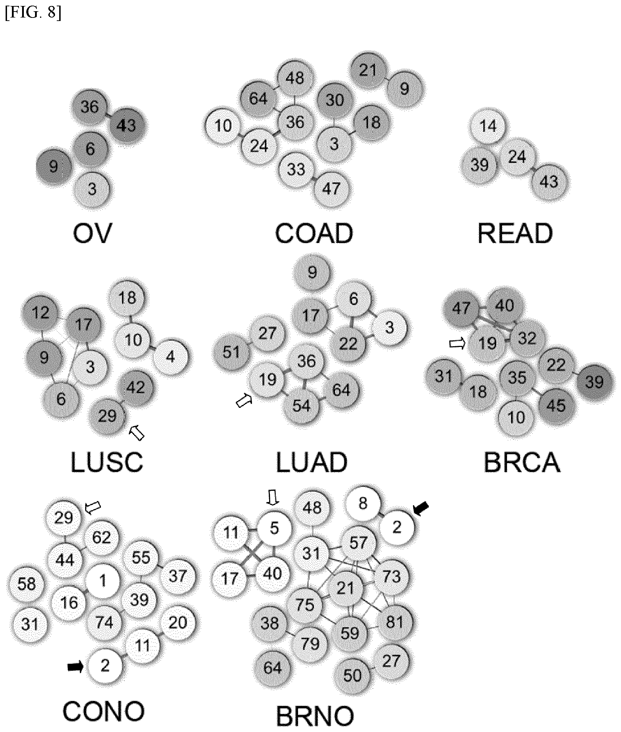

Early-linked modules constitute a seed in a region constituting an intermodular network. FIG. 8 illustrates an example of an intermodular network constructed with 8 different tissues based on TCGA data sets. A black arrow indicates a seed of a kernel domain, a white arrow indicates a seed of a cell cycle and DNA repair (CCDR) domain. The entropy of the module is represented in gray scale. A brighter color indicates a lower entropy level. In FIG. 8, each node represents a single module, and each module is identified by a node number.

For BRNO and CONO data sets, the seed of the kernel domain appears at cutoff ($C_f$) of 4.0. However, the first edge in the kernel domain of BRCA does not appear until the $C_f$ 2.2. The intermodular networks of LUAD, COAD and READ data sets shows the first edge of the kernel domain at $C_f$ = 3.0, 2.8, and 3.0, respectively. In the case of LUSC and OV, the first edge of the kernel domain appears at $C_f$ 1.9. These results suggest that the intermodular networks of tumor tissues may differ from those of normal tissues with respect to the kernel domain.

The intermodular network was reconstructed while experimentally varying $C_f$ for the TCGA data set. The total number of edges and the number of edges per module in a normal tissue were larger than those in a tumor tissue. This implies that the genomic system of the tumor is simpler than that of the normal tissue.

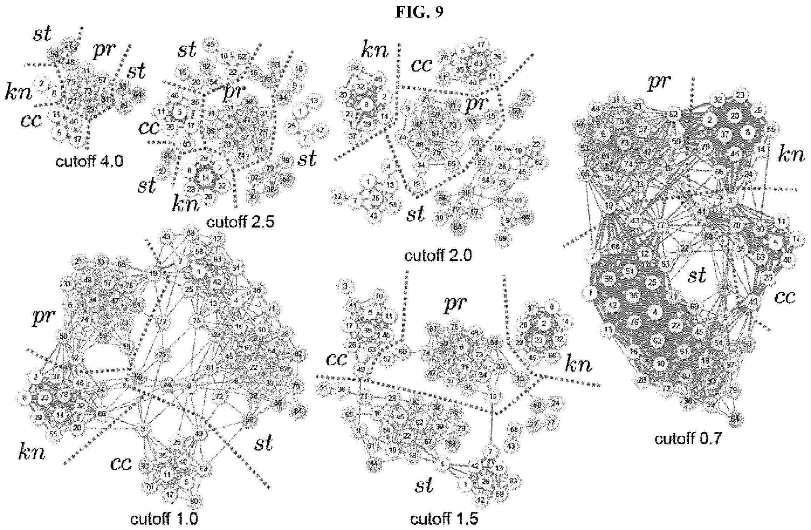

FIG. 9 illustrates an example of an intermodular network of BRNO at various cutoffs. In FIG. 9, kn denotes a kernel domain, cc denotes a CCDR domain, pr denotes a parenchyma domain, and st denotes a stroma domain. In FIG. 9, each node has a gray scale value. A brighter color indicates a lower entropy. When the cutoff $C_f$ decreases, the number of modules having an edge increases, and network disruption is reduced. The intermodular network exhibited complete connections between domains before the $C_f$ reaches 1.0. The seeds of all domains kn, cc, pr, and st are identifiable at $C_f$ 4.0.

By mapping modules between gene expression data sets of different tissues and searching functions of elementary genes from gene ontology, the specific biological function of each region demarcated by an intermodular network can be inferred. The intermodular network of BRNO constructed at $C_f$ 1.0 may clearly explain the relationship between domains. The kernel domain kn may control the parenchyma module pr that performs an actual function through modules m52 and m60. A module m3 relays an information flow between the kernel domain and the CCDR domain.

A module of the st region may play a role in the stroma function of a normal breast. At $C_f$ 4.0, the st region is divided into two regions. The region containing m38, m64 and m79 may be associated with adipocytes, and the region containing m27 and m50 may relay information between the stroma domain and the kernel domain.

The stroma domain st has 6 seeds at $C_f$ 2.5. Through the analysis of the module, it is assumed that angiogenesis, immune function (macrophage), extracellular matrix formation, and actions on adipocytes are performed. In addition, the stroma domain st is also assumed to serves as a relay between the kernel domain and the CCDR domain.

Functions of domains and modules can be estimated on the basis of various intermodular networks determined by the $C_f$ value.

Several modules located in the central are of the intermodular network connect all domains of the genomic system. Those modules may be regarded a kind of meta-program. The modules suggest that the extracellular matrix and a vasculature are regulated by communication between modules belonging to the stroma, parenchyma, and kernel domains. In a normal breast tissue, the genomic system related to immune function seems to be suppressed by other systems.

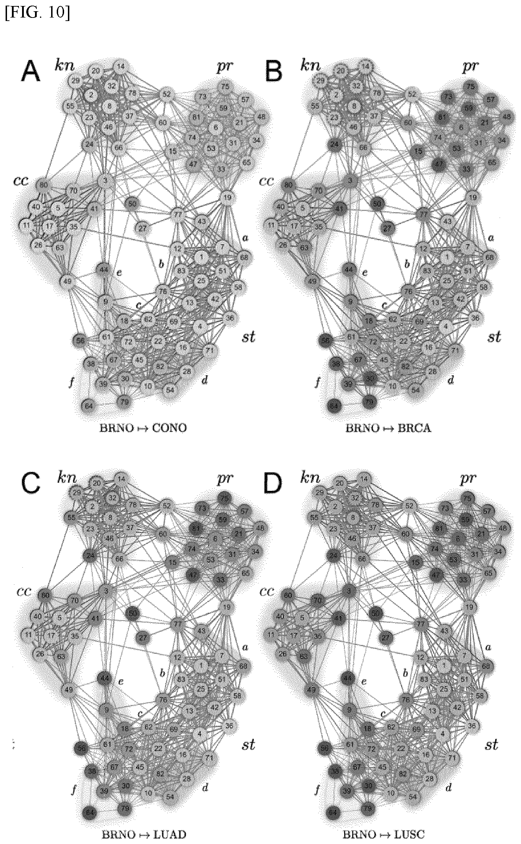

FIG. 10 shows an example of an intramodular network of BRNO mapped to other types of tissue. A node color indicates a variation in entropy in the genomic module of BRNO. Each node has a grayscale value. A brighter color indicates that the entropy is almost the same in BRNO and other tissues. A darker color indicate that the entropy is higher in other tissues than in BRNO.

FIG. 10A shows an example in which BRNO is mapped into CONO, FIG. 10B shows an example in which BRNO is mapped into BRCA, FIG. 10C shows an example in which BRNO is mapped into LUAD, and FIG. 10D shows an example in which BRNO is mapped into LUSC. FIG. 10A is an example of mapping to another normal tissue, and FIGS. 10B to 10D are examples of mapping to a tumor tissue. The intermodular network is constructed based on a domain type and entropy difference between modules in the genomic module network. In FIGS. 10A to 10D, f denotes an adipose tissue domain.

Referring to FIG. 10A, when the intermodular network of BRNO is mapped into CONO, most modules in the kernel domain kn shows mapped entropies of 0.091 nats to 0.182 nats similar to 0.017 nats to 0.109 nats which are entropies of the primary modules in BRNO. However, the kernel of CONO mapped to BRNO yields mapped entropies of 0.144 nats to 0.289 nats, which are slightly higher than the entropies of 0.016 nats to 0.043 nats of CONO itself. This difference may be due to the slightly wider kernel domain of CONO relative to that of BRNO. Accordingly, it may be inferred that a colonic tissue is composed of more cell types than a breast tissue. In other domains, a few modules in the parenchyma (pr) tissue and the adipose tissue (f) of normal breast reveal mapped entropies that increased somewhat. Accordingly, a substantial proportion of the biological program of a normal breast tissue should also be active in a normal colon, but the degree of their functional activities may be altered according to parametric inputs from the environments and other modules. Almost all of the mapped modules of CONO to BRNO aside from the kernel domain are much less active than the corresponding primary modules of CONO. This result implies that the genomic system of a normal colon is more complex than that of a normal breast.

FIGS. 10B to 10D show results of mapping the intermodular network of BRNO to BRCA, LUAD, and LUSC, which are tumor data sets, respectively. Although the type of cancer differs, the distribution patterns of entropy in mapped intermodular networks are similar for all three tumor data sets.

When all the modules included in the parenchyma domain pr of BRNO are mapped to other tumor tissues, the entropies of all the modules were 0.890 nats to 1.493 nats. These entropies are much higher than an original entropy of 0.109 nats in BRNO and a mapped entropy of 0.263 nats when BRNO is mapped into CONO. Accordingly, it is considered that the parenchyma domain was altered in tumor tissues to the extent not to normally function.

The CCDR domain showed mapped entropies ranging from 0.754 nats to 1.507 nats. This implies that different breakage patterns are shown for different tumor types.

In particular, meta-modules that connect domains are deactivated in a tumor tissue. The meta-module refers to a module for serving to connect different domains. When the intermodular networks of BRNO were mapped into LUSC, the entropy of the module m3 was in a range of 0.795 nats to 1.407 nats. That is, the second highest disintegration next to the CCDR domain was shown.

The kernel, CCDR, and parenchyma domains of the genomic system can send predetermined information to the stroma domain to control extracellular matrix formation including angiogenesis (c), immune function (d), and formation of adipose tissue (f). Regions a and e for connecting the parenchyma domain pr, the kernel domain kn, and the CCDR domain cc to the stroma domain st are very weakened in a tumor tissue. This implies that it is difficult in the tumor tissue that the stroma domain communicates with the other domains. That is, the action in which the stroma domain transfers information to other domains to influence a certain function cannot be performed. This is consistent with uncontrolled abnormal stroma construction in a tumor tissue.

The genomic modules obtained from 6 types of tumor tissue gene expression data BRCA, COAD, READ, LUAD, LUSC, and OV, 2 types of normal tissue gene expression data BRNO and CONO and 3 types of mixed data X6CA, X2NO, and X6C2N have various entropy levels. The experiment shows that the module with extremely low entropy is isolated. The module with the lowest entropy is: (i) the second module m2 in most tissues and; the first module m1 in breast tumor tissue (BRCA), normal colon tissue (CONO), and ovarian tumor tissue (OV). As described above, a genomic module having a lower entropy compared to other modules corresponds to a kernel module.

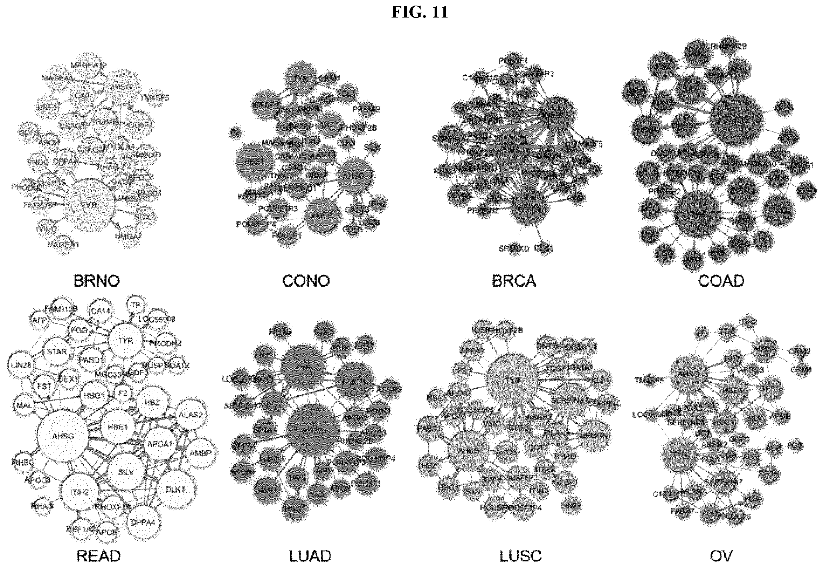

FIG. 11 illustrates an example of the kernel modules of 8 tissues. The TCGA data sets for the eight tissues are BRNO, CONO, BRCA, COAD, READ, LUAD, LUSC, and OV as described above.

As described above, each module includes a plurality of genes. Genes belonging to a module constitute a genetic network. In FIG. 11, the size of each node is proportional to the number of edges between genes. As can be seen from FIG. 11, in the modules of different tissues, major genes are common. For example, TYR, HBE1, F2, GDF3 and AHSG are commonly present in all tissues, and the remaining genes are commonly present in more than half of the tissues. In addition, most of the tissues are common in that genetic each of the networks is constructed around TYR and AHSG. Although the respective genetic networks of different tissues are not composed of perfectly identical genes, the modules and the genes constituting each of the modules are considerably common in different tissues.

The exceptionally low entropy of a particular module in all tissues means that the particular module is activated in all cells regardless of phenotype and function, meaning that the module performs a common function in all types of cells and can be considered a key component in the eukaryotic genomic system. A module whose entropy is extremely low and which is composed of genes common in all tissues is called a kernel module. A plurality of kernel modules may exist, and a set of kernel modules is called a kernel domain. The kernel module is presumed to play a significant role in the activation of the genomic system by generating gene expression by-products such as non-coding RNA rather than by generating proteins related to a specific protein network.

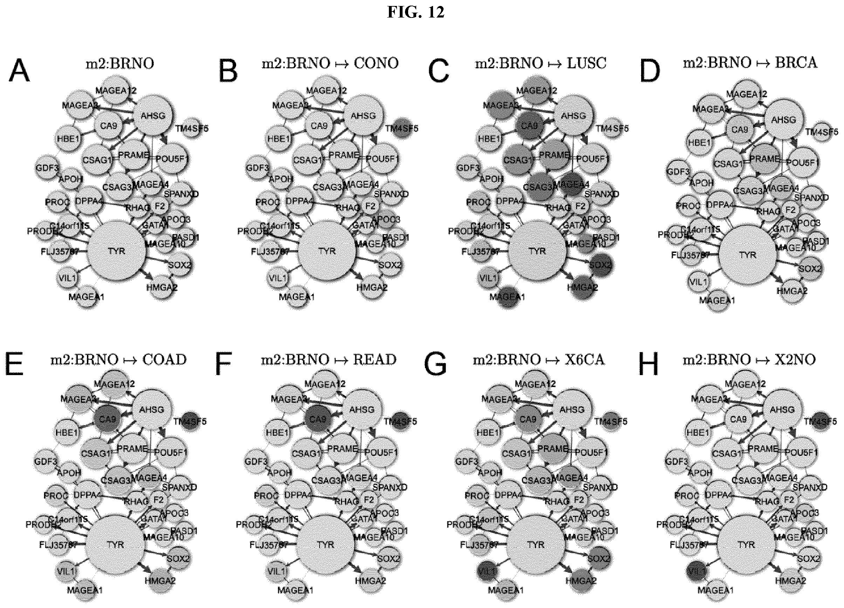

To experimentally check whether the kernel modules are common among different tissues, the kernel modules are mapped to different tissues. FIG. 12 illustrates an example of mapping the kernel module of BRNO to another tissue. Here, mapping refers to a process of isolating data of genes belonging to a given module from gene expression data of a different tissue and performing the same calculation on the basis of the isolated data.

FIG. 12A is the basic kernel module of BRNO. FIG. 12B is the result of mapping BRNO to CONO. FIG. 12C is the result of mapping BRNO to LUSC. FIG. 12D the result of mapping BRNO to BRCA. FIG. 12E is the result of mapping BRNO to COAD. FIG. 12F is the result of mapping BRNO to READ. FIG. 12G is the result of mapping BRNO to X6CA. FIG. 12H is the result of mapping BRNO to XNO. Nodes in the genetic network are represented in grayscale. A bright node means that the LOR value of the gene is close to 0, and a dark node means that the LOR value of the gene is a negative value. The brighter the color, the closer to 0 the LOR value. The darker the color, the greater the negative value.

When mapping the kernel of BRNO to another tissue, the entropy increases if the mapped gene does not exist in another kernel region or if the complexity of the kernel region of the tissue to be mapped is low. Mapping the BRNO kernel to another tissue means the process of isolating the gene data existing in the BRNO kernel from the gene expression data of other tissues and performing the necessary calculations on the basis of the isolated gene data. When the BRNO kernel is mapped to CONO, the calculated mapped entropy is 0.091 nats. This is much lower than 0.515 nats which is the entropy of the gene randomly selected from CONO (normal colon tissue). This implies that the kernel modules of the two different tissues are quite similar. Referring to FIG. 12B, when the kernel module of BRNO is mapped to CONO, the LOR is close to 0 for most genes. On the other hand, when the kernel module of BRNO is mapped to the tumor data (BRCA, COAD, READ, LUAD, LUSC, OV), the mapped entropy was as high as 0.224 nats to 0.601 nats. Therefore, the fact that the mapped entropy is high when BRNO (normal breast tissue) is mapped to tumor tissue means that the characteristic directionality of the tumor kernel area is dispersed compared to that of normal tissue. Furthermore, equivalent results are obtained when the kernel module of CONO is mapped to other data sets.

A domain related to the cell cycle and DNA repair (hereinafter referred to as CCDR), which is important in relation to a tumor, will be described.

Cell division is an essential process for the development of multicellular organisms from a fertilized egg to a somatic cell and for an increase in the population of unicellular organisms. Cell division is delicately regulated through cell cycle arrest and DNA damage repair, and dysregulation can result in abnormal cell growth.

The CCDR domain is composed of a plurality of modules in normal breast tissue. Twelve modules constituting the CCDR domain are composed of genes that participate in cell division (for example, BUB1) and have been shown to be strongly linked to each other via edges. Many modules composed of these genes were also found in other normal tissues (CONO and X2NO) whereas few modules composed of those genes were found in tumor tissues.

When 12 CCDR modules of normal breast tissue are mapped to other normal tissues, the mapped entropy values are overall similar to the original entropy values. In contrast, when the CCDR module is mapped to a tumor data set, the mapped entropy value increases to the level of random entropy calculated in each data set.

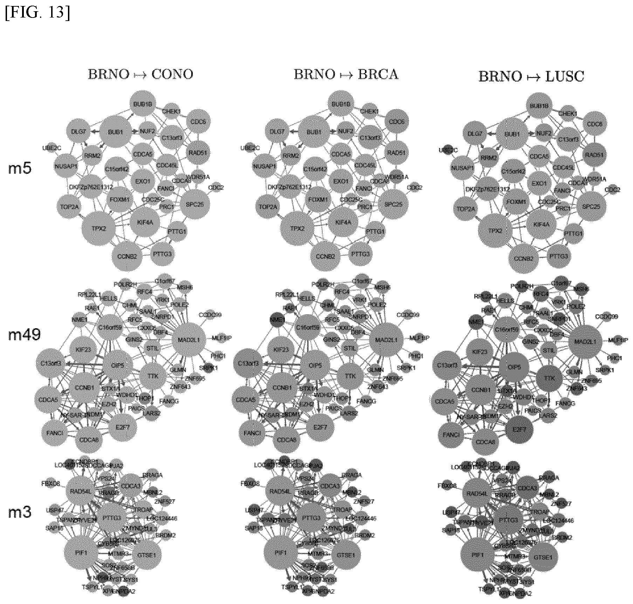

FIG. 13 shows an example of mapping the modules of the CCDR domain of BRNO to the modules of another tissue. FIG. 13 shows genetic networks for several modules belonging to the CCDR domain. FIG. 13 shows modules m3, m41, and m49 among the modules of the CCDR domain. FIG. 13 is a result of mapping the modules of the CCDR domain of BRNO to each of CONO, BRCA, and LUSC. The meaning of the colors of the nodes are the same as described with reference to FIG. 12.

FIG. 13 shows the genetic networks within the modules constituting the CCDR domain of a normal breast. The probability of a module contributing to the expression of a gene indicates how the gene relates to the module. When the modules of the CCDR domain of a normal breasts were mapped to normal colon (CONO), most genes showed high probability with respect to a corresponding one of the modules. In contrast, the probability decreased significantly when the modules of the CCDR domain was mapped to a cancer data set. When the modules m3, m41, and m49 were mapped to the tumor data sets, the mapped entropy values exceeded 1.0 nats. This means that collapse or alteration of the modules of the CCDR domain in tumors not only results in cancer cell proliferation but also being uncontrollable by the kernel domain, thereby being out of balance with cellular events in the parenchyma and stroma.

When combining the results obtained by mapping the CCDR module of normal breast tissue to other normal and tumor tissues, normal cells are under the strict control of the CCDR program, but in tumor tissues, due to the collapse or alteration of the CCDR module, the cell cycle continues in cells where DNA damage has occurred. When BRNO is mapped to LUAD, the entropy value is more than twice the entropy value of the case where BRNO is mapped to LUSC. This is consistent with the results of previous studies showing that LUSC has a faster rate of cancer progression and a higher mutation rate than LUAD.

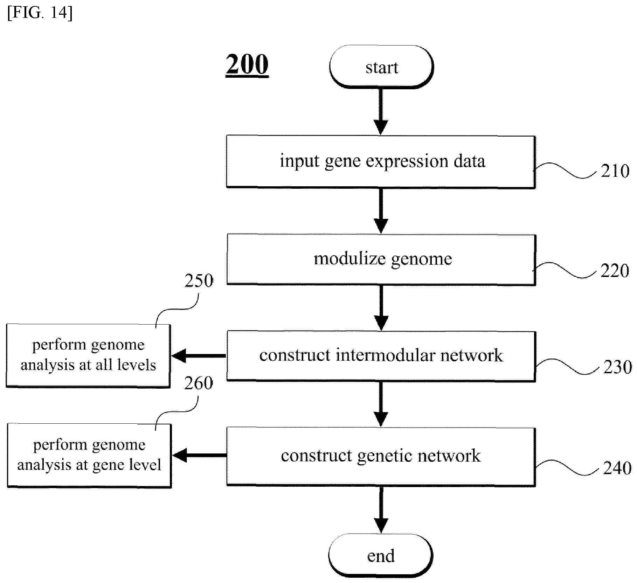

The above-described genomic module network construction process will be summarized. FIG. 14 is an example of a flowchart 200 for a genomic module network construction process. The genomic module network constructed in the same manner as in FIG. 14 is called a basic genomic module network. The basic genomic module network can be constructed by the following process.

The analysis device receives gene expression data (210). Here, the gene expression data may be gene expression data for a normal tissue. The gene expression data is preferably data isolated from a plurality of samples. The gene expression data is data obtained using a technique such as cDNA microarray. Thereafter, the analysis device divides (i.e., modularizes) the genes into specific modules using the gene expression data (220). This is the process of interpreting the gene expression data to classify the genes constituting the genome into specific modules. The analysis device establishes an intermodular network with a plurality of modules (230). In addition, the analysis device constructs a genetic network with a plurality of genes belonging to a module (240). Genetic network construction needs to be performed after the modularization. The analysis device may analyze the intermodular network to analyze the genome at all levels (250). The analysis device may identify the relationship between the modules on the basis of the intermodular network. In addition, by mapping the intermodular network of one sample to the intermodular network of another sample, it is possible to identify the relationship between different samples. As described above, it is confirmed that the activity of a specific module or a specific domain is weakened in the tumor tissue as compared to the normal tissue.

Furthermore, the analysis device may analyze the genome at the gene level (260). Genetic networks can be used to identify the relationship between genes. Furthermore, by mapping genetic networks of different samples to each other, it is also possible to identify the gene functions for a specific sample. For example, analysis for determining activation of a function of a specific gene, inactivation of a specific gene, detection of a gene associated with a tumor, etc. may be performed on a tumor patient. By using this, genes (markers) related to specific diseases can be identified.

In FIG. 14, the order of the intermodular network construction 230 and the genetic network construction 240 is not fixed. Therefore, unlike FIG. 14, the analysis device may first construct a genetic network after the modularization 220 of the genome.

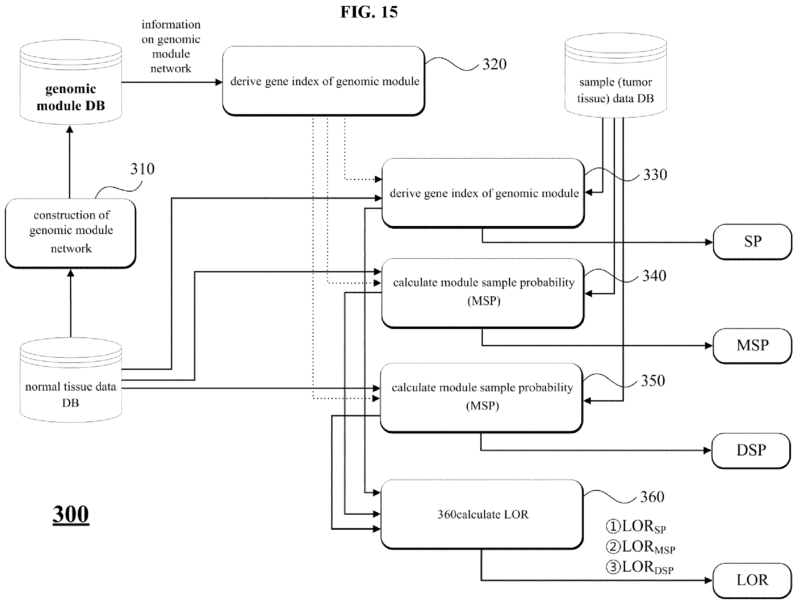

FIG. 15 is an example of a process 300 of calculating an analysis index for sample data using a genomic module network. The analysis index includes at least one of the SP, MSP, DSP, and LOR.

FIG. 15 shows three DBs. A normal tissue data DB stores gene expression information (corresponding to the first gene expression data described above) for a plurality of normal tissues. A genomic module DB stores information generated after the construction of the genomic module network. A sample data DB stores gene expression information (second gene expression data) of an analysis target. The sample data DB may store gene expression information of tumor tissues. The sample data DB may store gene expression information of a plurality of tumor tissues and individual characteristic information of a corresponding sample. Hereinafter, it is assumed that the sample data DB stores gene expression information of tumor patients. Although FIG. 15 shows three DBs separately, the three DBs may be in a physically integrated single storage device.

The analysis device acquires the gene expression vector of a tumor sample from the tumor tissue data DB. An expression vector refers to a one-dimensional array constructed with expression data extracted from the entire genome or with a part of a gene isolated from a specific sample. The analysis device extracts gene expression data consisting of specific genes isolated from all samples registered in the normal tissue data DB, and uses Equation 3 above to obtain a density matrix (p(s)) in a genetic space. In addition, the probability of a specific sample for the density matrix in the genetic space is calculated using Equation 4. The analysis device may acquire gene expression data of a tumor tissue sample and generate an expression vector therefrom. The analysis device may generate a predetermined density matrix from the gene expression data.

The analysis device acquires first gene expression data for normal tissue from the normal tissue data DB. The analysis device builds a genomic module network based on the first gene expression data (310). The genomic module DB stores information on the constructed genomic module network.

The analysis device may derive an index of a specific gene from the genomic module DB to identify a gene belonging to a target module of the genomic module network **(320)**. The genomic module DB provides information about which genes a specific module is composed of or information about which module a specific domain is composed of. In addition, the genomic module DB may provide information about to which module or domain a specific gene belongs. The genomic module DB may contain module identifiers, domain identifiers, gene identifiers, a module-to-gen matching table, a domain-to-module matching table, a domain-to-gene matching table, and the like.

The analysis device analyzes a sample by comparing normal tissues and tumor tissues, using information provided by the normal tissue data DB, the sample data DB, and the genomic module DB. The analysis device may generate various indices to quantify the transformation of the tumor tissue compared to the normal tissue. FIG. 15 shows an example in which the analysis device calculates a sample probability (SP), a module sample probability (MSP), a domain sample probability (DSP), and a log odds ratio (LOR).

The gene expression data for a normal tissue used for the computation of the indices may be gene expression data used to construct a genomic module network or gene expression data extracted from another normal tissue.

The analysis device may calculate SP (330). SP is a value obtained by quantifying the degree of transformation of the genomic system from a normal state in a sample of an individual cancer patient to be analyzed, for all genes included in the whole genomic module. SP represents the degree of transformation of the currently input sample, for the whole module. That is, SP corresponds to an analysis value for all genes included in the whole genomic module. To this end, the analysis device extracts the indices of all genes included in one or more modules among the entire genomic module, obtains a density matrix from normal tissues, constructs an expression vector with the relative genes extracted from specific sample data, and calculates SP. The SP is expressed as a certain probability with respect to sample data to be analyzed. The probability of a sample i with respect to the corresponding gene set may be expressed as in Equation 12 below. This is calculated using the density matrix (p) in the genetic space defined by the corresponding genes using Equation 3 and the expression vector ($s_i$) composed of the expression data of the corresponding gene in the sample i.

[Equation 12]

$$P_i = \langle s_i | \rho | s_i \rangle$$

In fact, the degree of transformation of the genomic system of a specific sample i can be determined based on the expression data of the genes included in all modules of a normal tissue. Therefore, SP can be expressed as Equation 13 below. That is, SP is the same as Pi calculated by Equation 13.

[Equation 13]

$$P_i = P(s_i | G_M) = \sigma_{iM}^\top \rho_M^{(s)} \sigma_{iM},$$

$$\rho_M^{(s)} = \frac{\mathbf{G}_M \mathbf{G}_M^\top}{tr(\mathbf{G}_M \mathbf{G}_M^\top)},$$

$$\sigma_{iM} = \frac{\mathbf{s}_{iM}}{\|\mathbf{s}_{iM}\|}$$

In Equation 13, $G_M$ denotes an expression matrix of all gene sets included in one or more modules among all modules of a normal tissue, and $s_{iM}$ denotes an expression vector constructed by identifying the corresponding gene from specific sample data $s_i$.

In order to calculate SP, the analysis device identifies all genes belonging to one or more modules among the genomic modules of a normal tissue, extracts expression data of the corresponding gene from all samples in the normal tissue reference data DB to construct a density matrix, extracts expression data of the corresponding gene from the tumor tissue reference data to construct an expression vector, and calculates SPs.

The analysis device may calculate MSPs (340). MSP means a sample probability for each module. While the SP represents a sample probability that quantified the degree of transformation of the genomic system from a normal state based on the whole genomic module, the MSP represents a sample probability calculated based on one module. To this end, the analysis device extracts the gene index included in a specific genomic module, obtains a density matrix from a normal tissue, and constructs an expression vector with the corresponding gene obtained from specific sample data to calculate the MSP. The MSP represents the degree of transformation of a specific sample for a specific module of a normal tissue. That is, the MSP is a value that quantifies the degree of transformation in the genomic system for each module in a specific sample. Depending on the disease (a specific tumor, etc.), a large degree of transformation may appear first in a specific module. Therefore, MSP analysis can be used as a meaningful indicator for disease diagnosis or prediction. Further, as will be described later, MSP is also used to uniformly classify samples. MSP can be expressed as in Equation 14 below.

[Equation 14]

$$P(s_i | G_\alpha) = \sigma_{i\alpha}^\top \rho_\alpha^{(s)} \sigma_{i\alpha},$$

$$\rho_\alpha^{(s)} = \frac{\mathbf{G}_\alpha \mathbf{G}_\alpha^\top}{tr(\mathbf{G}_\alpha \mathbf{G}_\alpha^\top)},$$

$$\sigma_{i\alpha} = \frac{\mathbf{s}_{i\alpha}}{||\mathbf{s}_{i\alpha}||}$$

In Equation 14, $G_\alpha$ denotes an expression matrix of a gene set included in a specific module $\alpha$ of a normal tissue. $s_{i\alpha}$ refers to an expression vector of a gene included in a specific module $\alpha$ in specific sample data $s_i$. That is, MSP represents the degree of the genomic system transformation of a specific sample tissue, investigated for a specific module of the normal tissue. Therefore, to calculate the MSP, a genomic module network must be established in advance. This is because the modules containing genes must be determined.

On the other hand, the analysis device may calculate the DSP **(350)**. A genomic module domain is a collection of genomic modules having similar biological functions and is composed of modules that are adjacent to each other in a genomic module network. DSP represents a sample probability calculated for all genes included in one or more modules among modules belonging to a specific domain. To this end, the analysis device extracts the indices of all genes included in one or more modules among the modules belonging to a specific domain, obtains a density matrix from normal tissue data, constructs an expression vector with the corresponding genes from the sample data to be analyzed, and calculates DSP. The DSP indicates the degree of transformation of a sample (analysis target) with respect to a specific genomic module domain of a normal tissue. In other words, the DSP is a value obtained by quantifying the degree of transformation of the genomic system for each domain in the sample to be analyzed. When the DSP is described by Equation 14, $G_\alpha$ means the expression matrix of a gene set included in a module belonging to a specific domain of a normal tissue, and $s_{i\alpha}$ is a gene expression data constructed with genes extracted from the sample data $s_i$.

The analysis device may calculate a log odds ratio (LOR) of a specific gene with respect to the sample probability (360). LOR is a generalized term meaning the log ratio of the probability according to the presence or absence of a specific condition. The LOR refers to the degree of change in the probability of a gene with respect to a genomic module according to the presence or absence of a specific gene in one genomic module and is a value quantifying the connectivity between genes. On the other hand, changes in in sample probabilities SP, MSP, and DSP according to the presence or absence of a specific gene in one sample are also LORs. That is, the LOR of a specific gene with respect to the sample probability is a value that quantifies the effect of a gene on the transformation in a genomic system in one sample. The LOR is the result of analysis of one gene unit. The analysis device may calculate the LOR based on several units. (1) $LOR_{SP}$ is a value that quantifies the degree of influence of a specific gene on the sample probability (SP) for the entire genomic module in the sample to be analyzed. (2) $LOR_{MSP}$ is a value that quantifies the degree of influence of a specific gene on the sample probability (MSP) for a specific genomic module in the sample to be analyzed. (3) $LOR_{DSP}$

is a value that quantifies the degree of influence of a specific gene on the sample probability (DSP) for a plurality of genomic modules belonging to a specific domain in a sample to be analyzed.

FIG. 15 is an example of constructing a genomic module network with normal tissue gene expression data, and then estimating information on transformation of a sample with respect to a normal tissue. Unlike FIG. 15, a genomic module network may be initially constructed with tumor tissue gene expression data, and then the sample and tumor tissue may be compared. In this case, the sample may be analyzed by determining how different or similar the sample is to the tumor tissue.

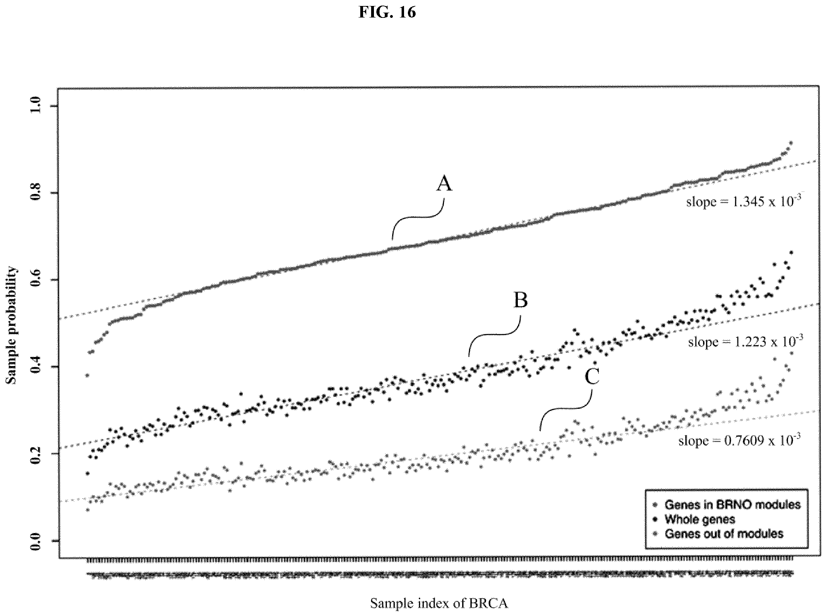

FIG. 16 illustrates an example of calculating the sample probability (SP) of a plurality of tumor tissue samples by using the genomic modules of a normal tissue. In FIG. 16, each dot represents the SP of each sample. FIG. 16 shows SPs of 248 breast tumor tissue (BRCA) samples. In FIG. 16, A shows the SP of each breast cancer sample for all genes included in the modules of a normal breast tissue (BRNO). That is, it corresponds to the SP defined by Equation 13 above. In FIG. 16, B represents the probability of each breast cancer sample for all genes, and C represents the probability for all genes not included in any module. As described above, the SP indicates the degree of transformation in the genomic system of the sample. A sample having a greater transformation in the genomic system has a lower SP value and is located on the left side of the graph of FIG. 16. When compared with the sample probability for all genes included in the BRNO module in FIG. 16, the sample probability for all genes is overall low, but the trend (slope) of the probabilities depending on the samples are similar. On the other hand, for the sample probability for all genes that are not included in any module, the slope is about half that of the former case. That is, the difference in the probability depending on the sample is not significant. The graph of FIG. 16 shows that genes not included in any module of each tumor sample do not exhibit the transformation in the genomic system well. It is the genes belonging to a module that drive the transformation in the genomic system. FIG. 16 shows that the sample probability (i.e., SP) using genes included in the genomic modules of a normal tissue reflects the transformation of the genomic system of each tumor sample well.

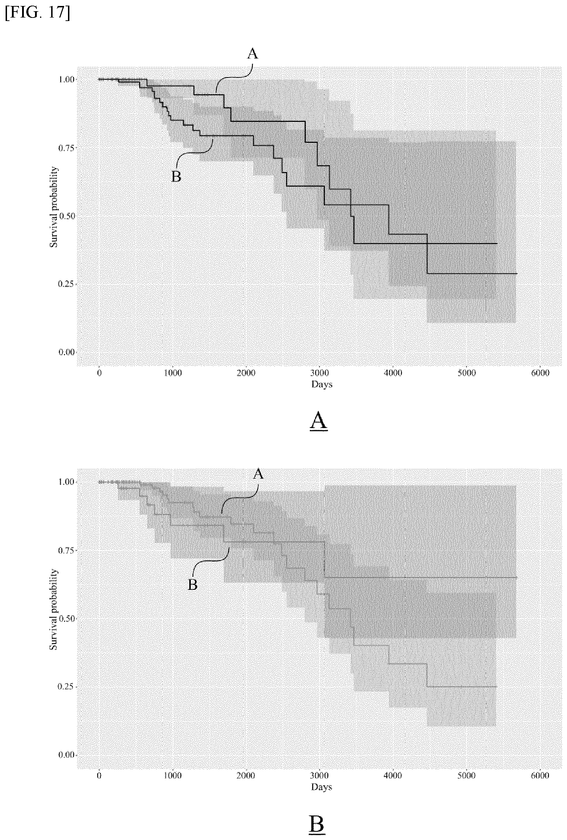

FIG. 17 shows an example of comparing tumor tissue sample groups classified according to SP. FIG. 17 is a result of performing a Kaplan-Meier survival analysis using information on whether each patient died and the time of death stored together with the gene expression data of 248 breast cancer patients in the tumor tissue reference data DB. FIG. 17A shows a result of survival analysis performed by calculating SP for each sample and dividing the samples into a high-SP sample group A with an SP value of 0.746 or more and a low-SP sample group B with an SP of less than 0.746. The area around the survival curve means a confidence interval with a confidence level of 95%. FIG. 17A shows that the high-SP sample group having an SP of 0.746 or higher has a significantly higher probability of surviving for about 1,700 days (p-value<0.05) than the low-SP sample group having an SP of less than 0.746. FIG. 17B is a result of survival analysis of each sample group by classifying the samples according to the presence or absence of expression of estrogen receptor (ER), which is commonly used as a standard for conventional breast cancer treatment. FIG. 17B is a result of survival analysis of a sample group A in which ER expression is present and a sample group B in which ER expression is not present in a breast cancer sample. FIG. 17B shows that the presence or absence of ER expression is independent of the survival rate of breast cancer patients. FIG. 17 shows that it is possible to predict the survival of a patient for a specific period by calculating the SP of a new cancer patient sample using the genomic module of a normal tissue.

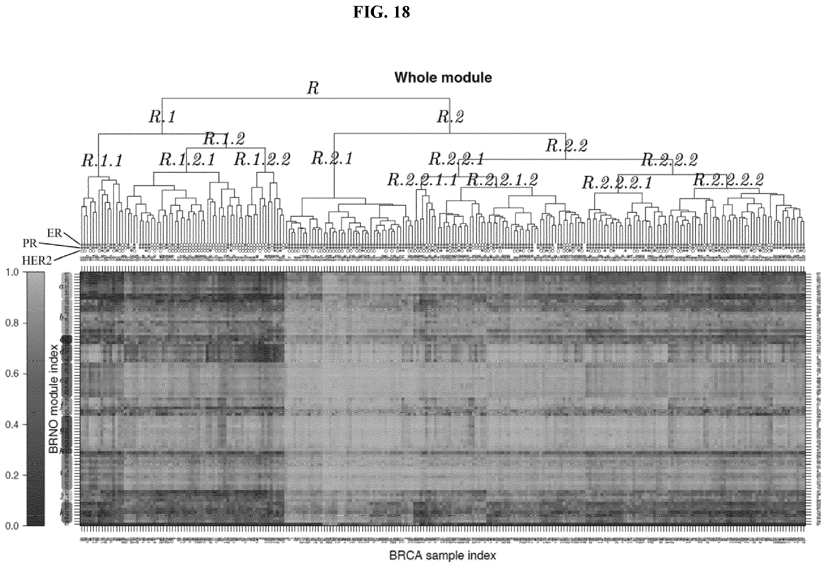

The analysis device may classify the samples of the tumor tissue reference data using the sample probability. FIG. 18 is an example of the classification of tumor tissue samples according to MSP. FIG. 18 illustrates an example of classifying samples by calculating the MSP of tissue samples (BRCA) of 248 breast cancer patients for 85 genomic modules isolated from normal breast tissue (BRNO). FIG. 18 illustrates an example of classifying samples by hierarchically clustering the MSPs of the breast cancer samples with respect to the genomic module of the normal breast tissue. The classification of the samples may be performed using all or part of the MSPs for the genomic module of a normal tissue or using DSP instead of MSP.

A heat map at the bottom of FIG. 18 shows the result of calculating the MSP for each module of each sample. In FIG. 18, the horizontal axis of the heat map at the bottom corresponds to breast cancer samples arranged through clustering, and the vertical axis represents the genomic modules of normal breast tissue classified according to domains. A dendrogram at the upper part of FIG. 18 shows the results of clustering of the breast cancer samples according to the MSPs for 85 modules of normal breast tissue. In FIG. 18, a mark starting with R means a sample group according to hierarchical classification. Considering the variation in the number of samples, the samples were classified into a total of 8 breast cancer sample groups (R.1.1, R.1.2.1, R.1,2,2, R.2.1, R.2.2.1.1, R.2.2.1.2, R. 2.2.2.1, R.2.2.2.2). In the heat map of FIG. 18, R.2.1 indicates that the sample group was composed of samples with a higher MSP than other sample groups.

Three columns of dots at the bottom of the dendrogram of FIG. 18 indicate the presence or absence of expression of an estrogen receptor ER, a progesterone receptor PR, and an HER2 receptor in this order from the upper side. The presence or absence of expression of these receptors is information used in conventional breast cancer diagnosis and drug treatment policy, and the information is extracted from sample information that is provided along with breast cancer gene expression data. A black dot indicates that the receptor is expressed in the sample, and a white indicates that the receptor is not expressed. In FIG. 18, the sample clustering based on MSPs shows that the sample group is highly correlated with tumor characteristics and progression. For example, the result shows that the sample groups R.1.2.1 and R.1.2.2 have significantly more triple negative samples (which means no expression of ER, PR, and HER2) compared to the rest of the sample groups. In conventional breast cancer diagnosis, the triple negative sample is classified as a patient with the highest risk, and when ER and PR are expressed, the sample is classified as a patient with a insignificant risk.

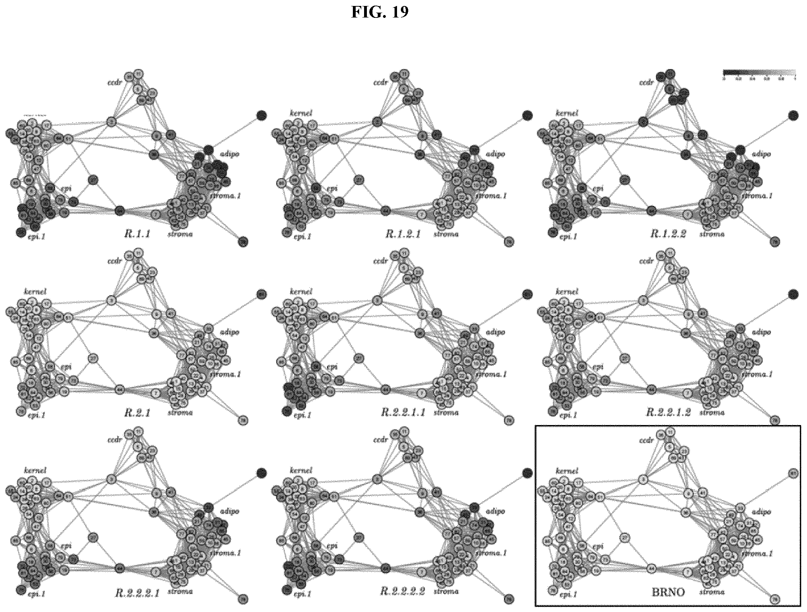

FIG. 19 illustrates an example in which the average MSP for each module of the tumor tissue sample group is marked on the genomic module networks of a normal tissue. That is, on the genomic module networks of a normal breast tissue of FIG. 19, the average MSP for each module of the 8 breast cancer sample groups classified as in FIG. 18 is marked. The lower right part of FIG. 19 illustrates the genomic module network of the normal breast tissue, on which the average MSP for each module of the whole normal breast tissue (BRNO) sample is marked. In FIG. 19, a lighter color represents that the average MSP for each genomic module is closer to 1 and a darker color represents that the average MSP for each genomic module is closer to 1. That is, FIG. 19 shows the modules and domains in which the transformation of the genomic system intensively occurs, in each breast cancer sample group. Referring to FIG. 19, the domain in which the transformation intensively occurs vary depending on the sample group. For example, in the sample group R.1.2.2, the module transformation of the CCDR domain severely occurs. As described above, the CCDR domain is a domain involved in cell cycle and DNA repair. Therefore, when a specific patient's sample belongs to the sample group R.1.2.2, drug treatment targeting cell cycle regulation is not expected to be effective. This is because there is a remarkably high possibility that the mechanism associated with the cell cycle is not working properly. On the other hand, in the case of the sample group R.2.1, all the modules except for the module 61 show MSP levels close to that of the normal breast tissue. Therefore, patients belonging to the sample group R.2.1 may be early breast cancer patients for which various treatments are expected to be effective because their state is close to that of the normal group. As such, the MSP and the MSP-based sample classification can be used as a basis for patient-specific treatment. Furthermore, it is inferred that the classification of modules and domains of the constructed genomic module network is a biologically significant.

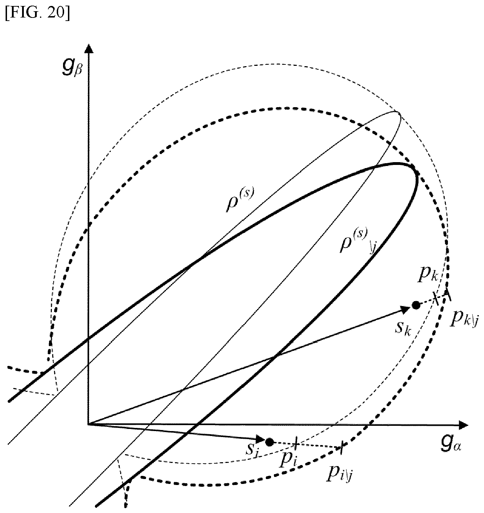

FIG. 20 illustrates an example in which the density matrix of a specific gene group is defined in a genetic space. FIG. 20 also shows the probability of an analysis target of the specific gene group. FIG. 20 is used to describe variables for LOR computation. In FIG. 20, a normal (thin) solid line ellipse represents the density matrix p(S) of the specific gene group, and a thick solid line ellipse represents the density matrix $\rho^{(s)}{}_{\backslash j}$ for the case where a gene j is removed from the specific gene group. In FIG. 20, a dotted line represents the probability trajectory of a sample for each density matrix. The LOR can be expressed as in Equation 15 below and may be computed for a specific gene belonging to a specific module.

[Equation 15]

$$r_{ij} = - \log \frac{p_{i \backslash j}}{p_i}$$

The LOR is a value that quantifies the effect of a gene j in sample data $s_i$ on the sample probability, i.e., SP, MSP, or DSP. The sample probability is a value that quantifies the degree of transformation of the genomic system of an analysis target sample, and the LOR is a value that quantifies the degree of influence of a specific gene on the sample probability in the sample. In the case of genes that promote transformation of the genomic system, the LOR has a negative value, whereas in the case of genes that inhibit transformation, the LOR has a positive value.

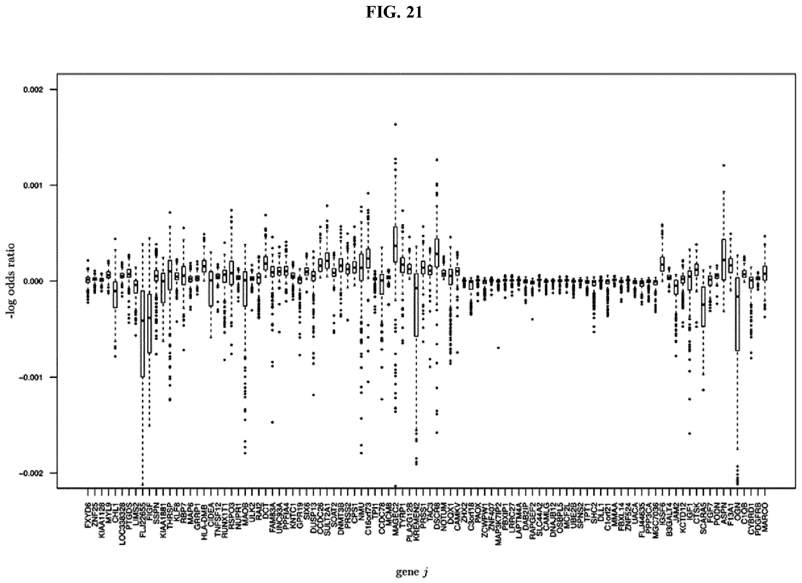

FIG. 21 illustrates an example in which the LOR of a specific gene for each tumor tissue sample is calculated using the density matrix of the counterpart gene group of the normal tissue. FIG. 21 illustrates an example in which LORs are calculated using the density matrix of a specific gene group derived from the gene expression data of normal breast tissue, after sequentially restoring and extracting genes belonging to the counterpart gene group of each sample of 248 breast tumor tissues. Referring to FIG. 21, each gene exhibits a variable distribution of LORs in 248 breast cancer samples. FIG. 21 shows that the degree of influence of each gene on the transformation of the genomic system varies depending on breast cancer patient. In FIG. 21, a breast cancer sample having a specific gene exhibiting an LOR that is significantly deviated from 0 means that the breast cancer sample is highly influenced by the specific gene in terms of the genomic system transformation.

In Equation 15, (1) as to $LOR_{SP}$, $P_i$ represents the value of the SP of an analysis target sample $s_i$ as defined in Equation 13, and $P_{i\backslash j}$ represents the value of the SP of the sample $s_i$ for a gene j for the case where the gene j of the genes belonging to the whole genomic module is removed. (2) As to $LOR_{MSP}$, $P_i$ represents the value of the MSP of an analysis target sample $s_i$ with respect to a specific module $\alpha$ as defined in Equation 14, and $P_{i\backslash j}$ represents the value of the MSP of the sample $s_i$ for a gene j for the case where the gene j of the genes belonging to the specific module $\alpha$ is

eliminated. (3) As to LOR$_{DSP}$, P$_i$ represents the value of the DSP of an analysis target sample s$_i$ for a gene j included in one or more modules among all modules belonging to a specific domain, and P$_{i\backslash j}$ represents the value of the DSP of the sample s$_i$ for the case where a gene j of eliminated from the genes.

To calculate the LOR, the analysis device first obtains the sample probability using a density matrix calculated from the expression data of a specific combination of genes of normal tissues and an expression vector constructed from the counterpart genes of an analysis target sample, and then obtains the sample probability for the case where a specific gene is eliminated.

In addition, the analysis device can perform analysis on a gene belonging to a specific module. Therefore, the analysis device can identify a gene belonging to a specific module by referring to the genomic module DB and can compute the LOR for the gene.

When describing the LOR, an example of calculating the LOR of each gene of an analysis target sample using normal tissues has been used. In some cases, the LOR of each gene of an analysis target sample may be computed using tumor tissues. In such cases, the LOR of each gene can be computed by obtaining a density matrix from a genomic module isolated from the gene expression data of a tumor tissue and then constructing the expression vector of the analysis target sample.

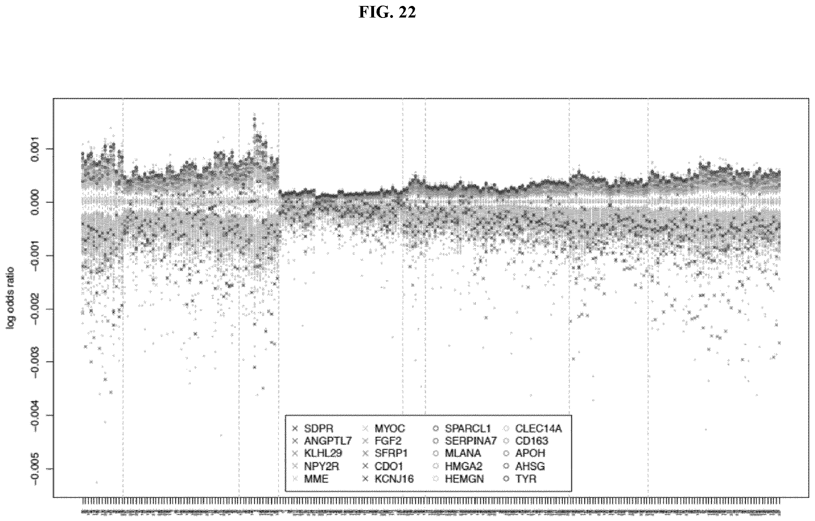

FIG. 22 is an example in which the LOR of a gene is computed for each tumor tissue sample, using the genomic modules of a normal tissue. Specifically, referring to FIG. 22, the LORs of respective genes for each of 248 breast tumor tissue samples (BRCA) are computed using the genomic modules of normal breast tissues (BRNO). In FIG. 22, the horizontal axis represents breast cancer samples arranged through the sample classification according to the MSPs of FIG. 18. That is, FIG. 22 is a dot plot showing the LORs of some genes for each breast tumor tissue sample. In FIG. 22, the LORs of 20 genes isolated from breast cancer patients exhibiting a specific pattern are illustrated. All the genes marked with "×" in the graph have negative LOR values. That is, the genes are considered to promote development of breast cancer. On the other hand, genes marked with "○" have positive LOR values. That is, these genes are considered to inhibit development of breast cancer. As such, the LORs of some genes even in a patient with a specific disease such as cancer have values with a specific tendency. Therefore, LOR can be used as a specific indicator for genetic analysis.

On the other hand, a genomic module network can be constructed by other approaches aside from the basic genomic module network described above. Gene expression data may be filtered in a predetermined way, and a genomic module network may be built based on the filtered data. A genomic module network based on the filtered data is called a filtering-based genomic module network.

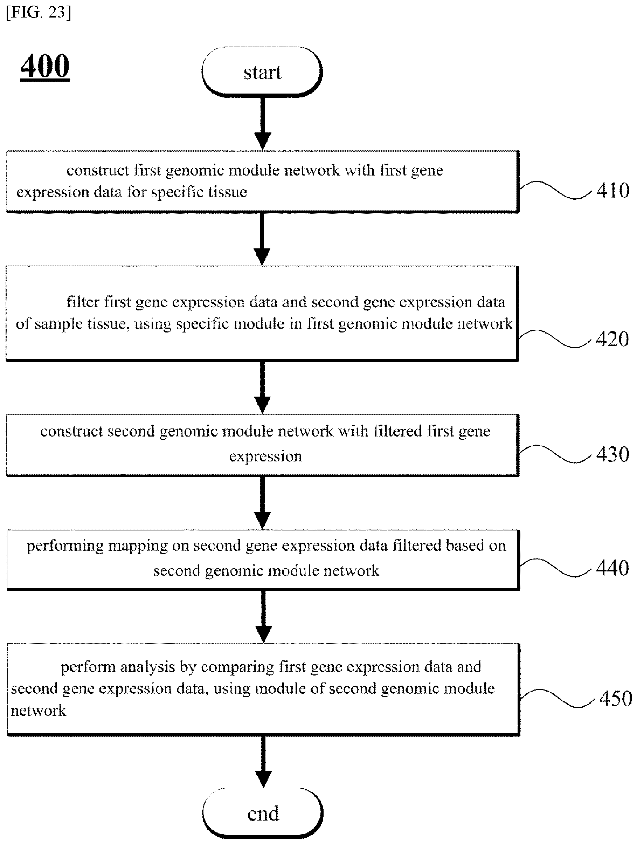

FIG. 23 illustrates an exemplary flowchart for a sample data analysis method 400 based on a filtering-based genomic module network.

An analysis device uses two types of gene expression data. First type of data is referred to as first gene expression data herein and is gene expression data for a specific tissue which is used as a reference for constructing a reference genomic module network. The specific tissue may be a normal tissue or a tumor tissue.

Second type of data is referred to as second gene expression data herein and is gene expression data for a sample tissue which will be analyzed. The type of the analysis target tissue is a tumor tissue occurring at the same position as the normal tissue.

The analysis device constructs a first genomic module network (reference genomic module network) based on the first gene expression data for a normal tissue or a tumor tissue (410). The first genomic module network is constructed based on the gene expression data of a normal tissue or a tumor tissue. The first genomic module network may be composed of module identifiers, identifiers of genes belonging to a module, connectivity information between modules, domain identifiers, identifiers of modules constituting a domain, identifiers of genes constituting a domain, connectivity information (a genetic network) between genes belonging to one module, etc. After constructing the genomic module network, genes are matched with a corresponding module to which the genes belong. In addition, connectivity of the genes with the modules, and the connectivity between the genes in the same module are determined.

The analysis device performs filtering on the first gene expression data and the second gene expression data, using a specific module belonging to the first genomic module network (220). The filtering process will be further detailed latter. The first genomic module network is composed of a plurality of modules, and a single module has connectivity with at least one of the other modules. That is, a single module transfers predetermined information to at least one of the other modules. The filtering is the process of blocking (filtering) the transfer of information to another module (or multiple modules in some cases) that transfers a large volume of information. The specific module to which the transfer of information is blocked may be called a kernel module. The kernel module has a low entropy level than other modules and is highly likely to participate in various biological processes. A kernel domain may include a plurality of kernel modules. In this case, the filtering may be performed on at least one of the multiple kernel modules. For example, the filtering may be performed on the kernel module with the lowest entropy level.

The specific module to be filtered may be a module with an entropy level that is lower than a predetermined reference level. The reference level may vary depending on the type of an analysis target tissue, the type of disease, and data collection environment.

The analysis device constructs a second genomic network using the filtered first gene expression data (430). The process of constructing the second genomic network is the same as that described above. The second genomic module network is constructed based on the data filtered in a predetermined way.

The analysis device performs mapping on the filtered second gene expression data of the sample tissue, using the constructed second genomic module network (440). That is, the analysis device identifies to which module the second gene expression data of the sample tissue belongs, using the identifiers of the genes belonging to a specific module in the second genomic module network.

The analysis device performs analysis by comparing the first gene expression data and the second gene expression data, using the constructed second genomic module network (450). The analysis device performs analysis on all modules within the genomic module network, a plurality of modules within the genomic module network, or a single module (target module) within the genomic module network.

The analysis device compares a difference of the first gene expression data belonging to a target module with a difference of the second gene expression data belonging to the same target module. Thus, the analysis device may quantitatively determine the transformation of the sample tissue compared with the normal tissues or the tumor tissues. The analysis device may analyze the transformation of the sample tissue, using the non-filtered first gene expression data and the non-filtered second gene expression data. Alternatively, the analysis device may analyze the transformation of the sample tissue, using the filtered first gene expression data and the filtered second gene expression data.

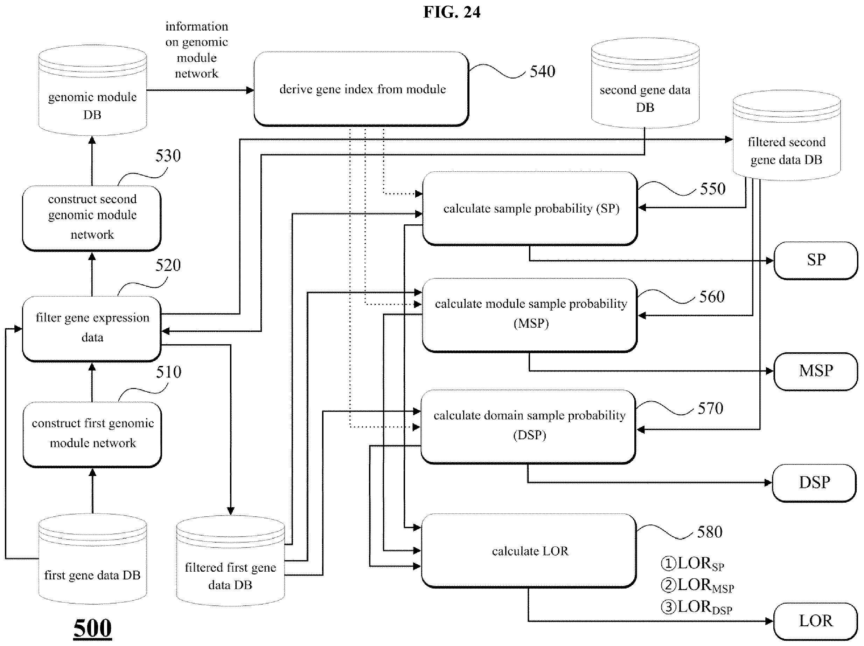

FIG. 24 illustrates an exemplary process 500 of calculating an analysis index for sample data, using a filtering-based genomic module network.

FIG. 24 shows five databases (DBs). A first gene data DB stores gene expression information for a plurality of normal or tumor tissues. The first gene data DB stores the first gene expression data described above. A first gene filtration data DB stores data obtained by uniformly filtering the data stored in the first gene data DB.

A genomic module DB stores information generated after the construction of genomic module networks. A second gene data DB stores gene expression information for a sample tissue to be analyzed. The second gene data DB stores the second gene expression data described above. The second gene data DB may store gene expression information for a tumor tissue. The second gene data DB may store gene expression information on a plurality of tumor tissues and characteristic information for the sample. Hereinafter, it is assumed that the second gene data DB stores gene expression information of a cancer patient. A second gene filtration data DB stores data obtained by uniformly filtering the data stored in the second gene data DB.

Although FIG. 24 illustrates five separate databases (DBs), the multiple DBs may be stored in a physically integrated single storage device.

The analysis device acquires the first gene expression data for normal tissues or tumor tissues from the first gene data DB. As described above, the analysis device constructs a first genomic module network based on the first gene expression data (510).

The analysis device filters the first gene expression data and the second gene expression data, using a specific module belonging to the first genomic module network (**520**). The first gene filtration data DB stores filtered first gene expression data. The second gene filtration data DB stores filtered second gene expression data.

The analysis device constructs a second genomic module network based on the filtered first gene expression data (530). The genomic module DB stores information on the constructed second genomic module network.

The analysis device derives the index of a specific gene from the genomic module DB to identify a gene belonging to a target module in the second genomic module network (540). The genomic module DB may include module identifiers, domain identifiers, gene identifiers, a modules-to-genes matching table, a domains-to-modules matching table, a domains-to-genes matching table, etc.

The analysis device analyzes a transformation of a sample of a tumor tissue on the basis of information provided by the second genomic module network, using the first gene filtration data DB, the second gene filtration data DB, and the genomic module DB. The analysis device may generate various indices to quantify a transformation of a tumor tissue relative to multiple normal tissues and tumor tissues. In this process, the analysis generates indices based on the second genomic module network.

The gene expression data used for the index computation process may be the gene expression data used for the construction of the genomic module network or the gene expression data extracted from another tissue. The gene expression data used for the index computation process may be filtered gene expression data or non-filtered gene expression data.

The analysis device may compute an SP (530). The SP is a value quantifying the degree of transformation of a genomic system of a sample (analysis target) of a patient, relative to genes included all the genomic modules of a normal or tumor tissue. The SP indicates the degree of transformation of a currently input sample relative to all the genomic modules. To compute the SP, the analysis device extracts indices of all genes included in one or more modules selected from among all the genomic modules, determines a density matrix based on a normal tissue or a tumor tissue, and constructs an expression vector based on a corresponding gene of specific sample data. The SP is represented as a certain probability with respect to sample data. The probability of a sample i with respect to a corresponding gene set may be represented by Equation 12 below. The analysis device computes the SP based on the second genomic module network. Alternatively, the analysis device may compute the SP based on filtered data.

To compute the SP, the analysis device identifies all genes belonging to one or more modules among all the genomic modules of the second genomic module network, extracts gene expression data of the identified genes from all the samples stored in the first gene filtration data DB to construct a density matrix, and extracts the gene expression data of the identified genes from the filtered second gene data to construct an expression vector.

The analysis device may compute an MSP (540) . The MSP refers to a sample probability for each module. While the above-described SP is a sample probability quantifying the degree of transformation of a sample from a normal tissue, for all the genes included in all the genomic modules, the MSP refers to a sample probability calculated on the basis of one module. To compute the MSP, the analysis device extracts indices of genes included in a specific genomic module, determines a density matrix based on a normal tissue or a tumor tissue, and constructs an expression vector based on the corresponding genes from specific sample data. The MSP refers to the degree of transformation of a genomic system of a specific sample for each module. Depending on disease (e.g., a specific tumor), a large degree of transformation may appear in a specific module. Accordingly, the MSP is a meaningful index for diagnosing or predicting a disease. Further, as will be described later, the MSP may be represented by Equation 14 below. The analysis device computes the MSP based on the second genomic module network. The analysis device may compute the MSP based on filtered data.

The analysis device may compute a DSP (570). A genomic module domain is a set of genomic modules having similar biological functions and consists of modules adjacent to each other in the genomic module network. The DSP refers to a sample probability calculated for all genes included in one or more modules included in a specific domain. To compute the DSP, the analysis device extracts the indices of all genes included in one or more modules included in a specific domain, determines a density matrix based on the normal tissue data or the tumor tissue data, and constructs an expression vector based on the corresponding genes among the genes of the analysis target sample. The DSP refers to the degree of transformation of a sample from a specific genomic module domain of a normal or tumor tissue. That is, the DSP is a value quantifying the degree of transformation of the genomic system of the sample for each domain. The DSP will be described using Equation 14. In Equation 14, $G_\alpha$ denotes an expression matrix for a set of genes included in modules belonging to a specific domain $\alpha$ of a normal or tumor tissue, and $s_{i\alpha}$ denotes a gene expression vector configured by extracting data of the corresponding genes from the sample data $s_i$. The analysis device may compute the DSP based on the second genomic module network. Alternatively, the analysis device may compute the DSP, using filtered data.

The analysis device may compute a log odds ratio (LOR) of a specific gene with respect to a sample probability (**580**). The LOR refers to a degree of fluctuation in a sample probability in a genomic module depending on the presence or absence of a specific gene in one genomic module. The LOR is a value quantifying connectivity between the genes. The fluctuation in the sample probability (SP, MSP, and DSP) depending on the presence or absence of a specific gene in a sample is also a kind of LOR. That is, the LOR of a specific gene for a sample probability is a value quantifying the influence of the specific gene on the transformation of the genomic system of the sample. The LOR is an analysis result per gene. The analysis device may compute several LORs per different units. (1) $LOR_{SP}$ is a value quantifying the degree of influence of a specific gene within an analysis target sample on a sample probability (SP) for all genomic modules in the analysis target sample. (2) $LOR_{MSP}$ is a value quantifying the degree of influence of a specific gene within an analysis target sample on a sample probability (MSP) for a specific genomic module in the analysis target sample. (3) $LOR_{DSP}$ is a value quantifying the degree of influence of a specific gene on a sample probability (DSP) for a plurality of genomic modules belonging to a specific domain, in an analysis target sample. The analysis device may compute the LORs based on the second genomic module network. The analysis device may compute the LORs, using filtered data.

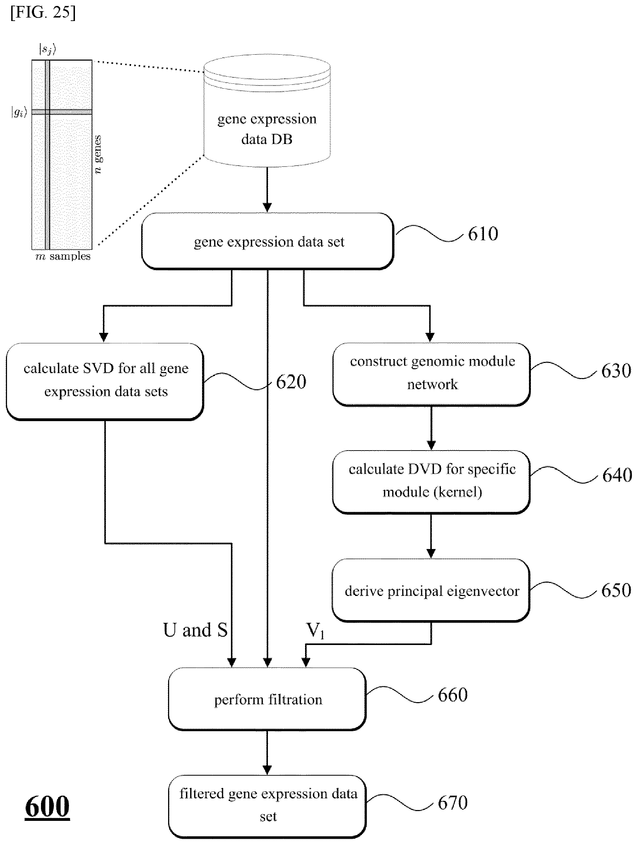

FIG. 25 illustrates an exemplary filtering process (600) for gene expression data set. The gene expression data DB stores gene expression data to be filtered. The analysis device may filter the first gene expression data and the second gene expression data. The analysis device eliminates a specific component of a linear combination from the gene expression data to be filtered. Various techniques may be used to eliminate the specific component of the linear combination. For convenience of description, it is assumed that singular value decomposition (hereinafter referred to as SVD) is used.

As shown in FIG. 25, the gene expression data DB contains expression data for n genes for each of m samples. The analysis device removes the specific component of the linear combination from the gene expression data in the form of a two-dimensional matrix. The SVD decomposes a m×n matrix A as expressed in Equation 16 below. U is a left singular vector matrix, S is a singular value matrix, and V is a right singular vector matrix. The SVD is a technique well known in the art, and thus a detailed description thereof will be omitted.

[Equation 16]

$$A = USV^T$$

The analysis device receives a gene expression data set (610). The analysis device performs the SVD on the entire gene expression data set (620).

The analysis device constructs a genomic module network using gene expression data of a normal tissue (630). The analysis device selects a specific module to serve as a reference for filtering among the modules belonging to the constructed genomic module network and performs the SVD computation on the specific module (640). The analysis device performs the SVD computation on gene expression data belonging to the specific module. For convenience of description, it is assumed that the specific module is a kernel module. The analysis device extracts a principal eigenvector (a column vector) of V (right singular vector matrix) from the SVD result for the kernel module (750).

The analysis device selects U (left singular vector matrix) and S (singular value matrix) for the entire gene expression data. In addition, the analysis device selects a principal eigenvector $V_1$ of V (right singular vector matrix) from the SVD result for the kernel module.

The analysis device performs filtering, using U (left singular vector matrix) and S (singular value matrix) extracted from the SVD results for the entire gene expression data and using the principal eigenvector $V_1$ of V (right singular vector matrix) extracted from the SVD result for the kernel module (660). Thus, the analysis device prepares uniformly filtered gene expression data sets (670).

[Table 5]

| Calculate filtered expression data $G'$ |
| --- |
| 1: $G_{(1)} = U_{(1)}S_{(1)}V_{(1)}^\top$: apply SVD on the first module of kernel domain |
| 2: $\lvert v \rangle \leftarrow$ extract the first column vector of $V_{(1)}$ |
| 3: $G = USV^\top$: apply SVD on whole expression data |
| 4: $\lvert f \rangle = US\lvert v \rangle$ |
| 5: normalize $\lvert f \rangle$ |
| 6: $G' = G - \lvert f \rangle$: subtract $\lvert f \rangle$ from each column vector of $G$ |
| 7: normalize $G'$ |

Table 5 above shows pseudocode for the filtering process. able 5 is an example of a filtering process performed on the basis of the first kernel module of the kernel domain. The analysis device generates a filter value vector $\lvert f \rangle$ by multiplying the first eigenvector $V_1$ of V (right singular vector matrix) extracted from the SVD result for the kernel module by U (left singular vector matrix) and S (singular value matrix) extracted from the SVD results for the entire gene expression data. In some cases, the analysis device normalizes the filter value vector. Finally, the analysis device generates G' obtained by subtracting the filter value vector from each piece of column data of the entire gene expression data G. G' corresponds to the filtered gene expression data. In some cases, the analysis device may normalize G'.

Hereinafter, an example of the genomic module network constructed based on the filtered data will be described. Originally, the gene expression data is a value obtained through linear combination. When a data filtering process for removing a specific component is performed, it is possible to determine primary features hidden by the specific component. A new module resulting from the filtering is referred to as a latent genomic module. It will be shown that the genomic module network constructed based on the filtered data is also meaningful for analysis.

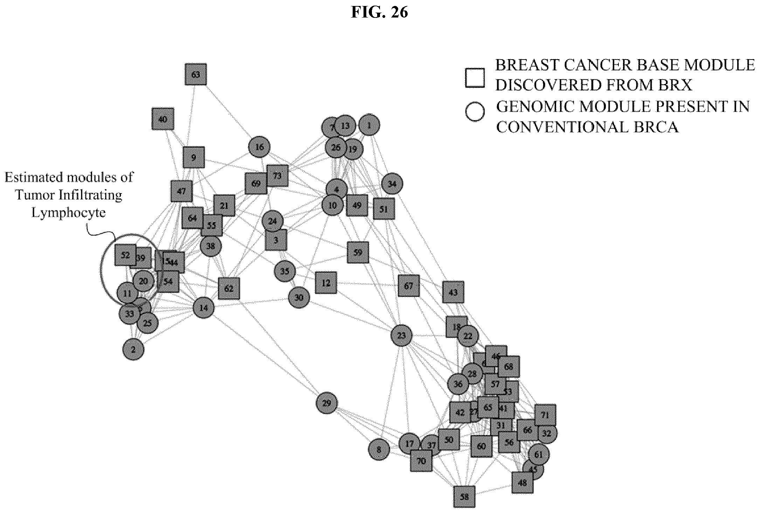

FIG. 26 illustrates an example of a genomic module network constructed based on filtered BRCA data. Herein, the filtered BRCA data is referred to as BRX. The BRX is obtained by filtering BRCA using a principal eigenvector of the kernel module. FIG. 26 is an exemplary genomic module network constructed based on BRX. The genomic module network constructed based BRX is hereinafter referred to as a BRX genomic module network.

In FIG. 26, modules which are present in a genomic module network constructed with BRCA (unfiltered data) and present in the BRX genomic module network are represented by a circle. In FIG. 26, modules that are present only in the BRX genomic module network are represented by a quadrangle. There are latent genomic modules that have not been identified before the filtering. As will be described below, the activity of a module related to a tumor-infiltrating lymphocyte (TIL) among modules of the BRX genomic module network is identified. For example, module #11 (BRX#11), module #20 (BRX#20), module #39 (BRX#39), and module #52 (BRX#52) of the BRX genomic module network contain a large number of various chemokine ligands necessary for lymphocyte migration, interleukin receptors involved in

EP 3 970 606 A1

immune responses, interferons, etc. Among the genomic modules, BRX#11 and BRX#20 are genomic modules that are present in the BRCA genomic module network constructed with non-filtered data as well as in the BRX genomic module network, and BRX#39 and BRX#52 are latent genomic modules that are newly found after the filtering.

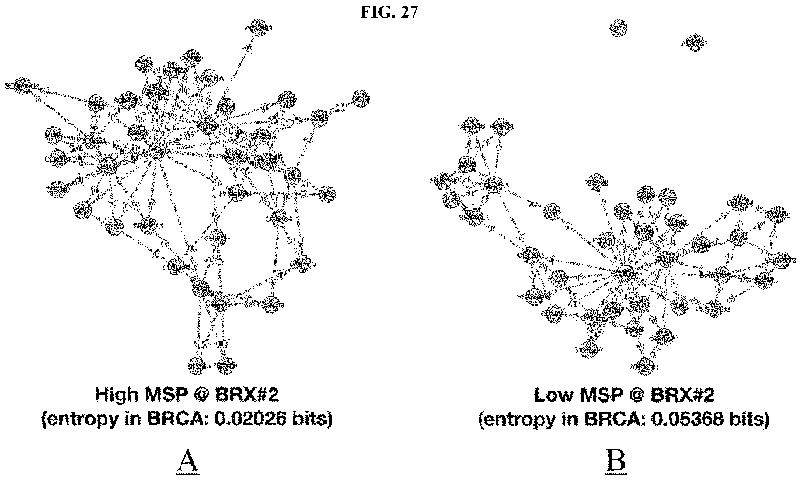

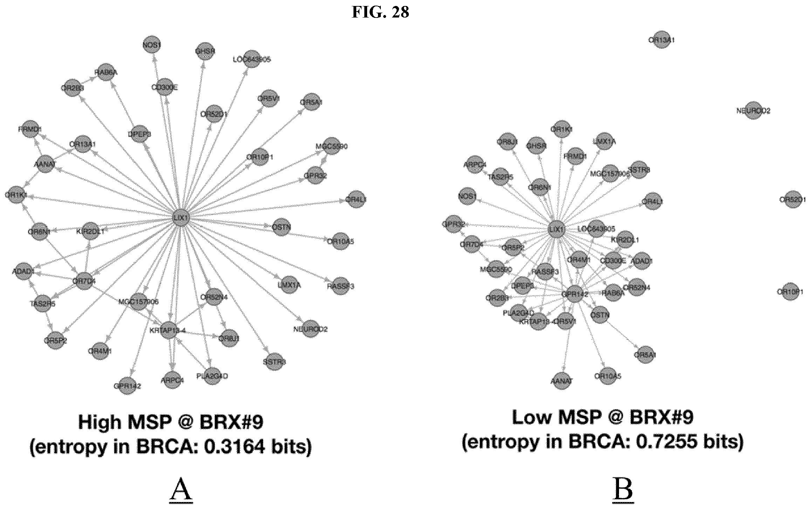

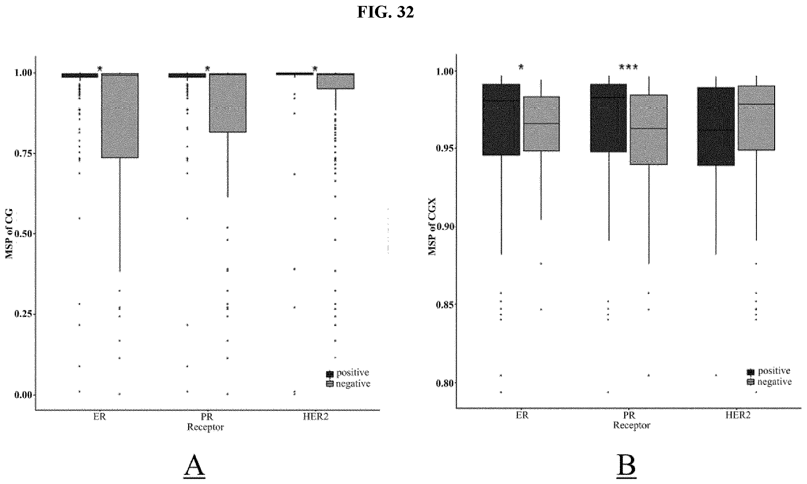

FIGS. 27 and 28 illustrates a sample classification example in which samples are classified based on an MSP at a specific module of the genomic module network. FIGS. 27 and 28 illustrate an example in which when samples are classified based on an MSP at a specific module of the genomic module network, the samples are classified into a high-MSP sample group and a low-MSP sample group that are distinguished with the MSP that maximizes a hazard ratio in Cox proportional hazard model survival analysis as a reference point. FIG. 27 illustrates an example in which samples are classified with an MSP for module #2 (BRX#2) of the BRX genomic module network as a reference point. FIG. 27 is a diagram illustrating a genetic network of BRX#2 constructed using a high-MSP sample group A and a low-MSP sample group B for BRX#2. When the entropy of BRX#2 is calculated using the unfiltered data of each sample group, it is confirmed that the entropies of both of the sample groups are very low. This proves that BRX#2 is a genomic module that is also present in the genomic module network constructed before the filtering. FIG. 28 is an example in which samples are classified based on an MSP for module #9 (BRX#9) of the BRX genomic module network. FIG. 32 shows a genetic network of BRX#9 constructed using a high-MSP sample group A and a low-MSP sample group B for BRX#9. When the entropy of BRX#9 is calculated using the unfiltered BRCA data of each sample group, it is confirmed that the entropy of the low-MSP sample group is two times or more higher than that of the high-MSP sample group. This proves that BRX#9 is a latent genomic module that is not found from the BRCA genomic module before the filtering. FIG. 28 shows that the genetic network B of BRX#9 in the low-MSP sample group has better connectivity than the genetic network A in the high-MSP sample group.

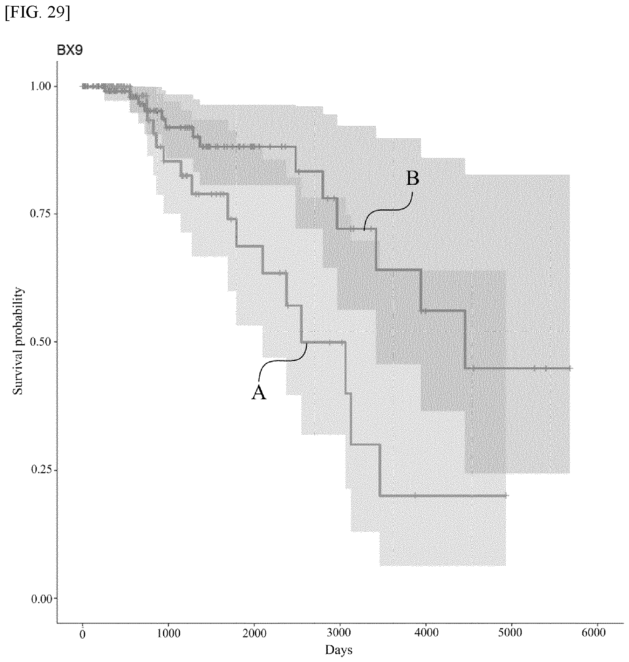

FIG. 29 illustrates an example of a survival curve for a specific sample group. FIG. 29 is an example in which Kaplan-Meier survival analysis is performed on breast cancer patient groups classified using BRX#9. In FIG. 29, A denotes a survival curve at a confidence level of 99% for patients in a high-MSP sample group, and B denotes a survival curve at a confidence level of 99% for patients in a low-MSP sample group. The biological characteristic of BRX#9 derived from the BRX genomic module network have not been fully examined. However, the result of the Cox proportional hazard model survival analysis shows that the survival rate of the patients in the low-MSP sample group was 2.4 times higher than that of the patients in the high-MSP sample group (p-value <0.05). Therefore, BRX#9 is an important genomic module for classification of breast cancer samples with respect to breast cancer progression. FIG. 29 suggests that a sample with a low MSP for a BRX latent genomic module is not related with breast cancer, i.e., the sample is close to a normal tissue. As a result of classifying patients using MSPs for several different breast cancer latent genomic modules, the survival rate of the patients in the low-MSP sample group was observed to be high.

**Sample Analysis Based on Kernel Module**

Gene expression data sets used by the researcher of the present disclosure will be described. The utilized gene expression data sets are similar to the data used in the above-described experimental procedure. To describe an example of sample analysis based on a genomic module network, the gene expression data sets will be described again. Eight TCGA gene expression data sets are shown in Table 6 below. Data for 2 normal tissues and data for 6 tumor tissues were used as follows.

[Table 6]

| Tissue | Gene expression data set |
|---|---|
| Normal breast | BRNO |
| Normal colon | CONO |
| Breast cancer | BRCA |
| Colon adenocarcinoma | COAD |
| Rectal adenocarcinoma | READ |
| Lung adenocarcinoma | LUAD |
| Lung squamous cell carcinoma | LUSC |
| Ovarian cancer | OV |

Furthermore, mixed data sets obtained by extracting some data sets from the eight gene expression data sets

28

and combining the extracted data sets were also used. The used mixed data sets are shown in Table 7 below. X2NO is a mixed data set of normal tissue data sets, X6CA is a mixed data set of 6 tumor tissue data sets, and X6C2N is a mixed data set of a total of 8 data sets.

[Table 7]

| Data sets | Mixed data sets |
|---|---|
| BRNO + CONO | X2NO |
| BRCA + COAD + READ + LUAD + LUSC + OV | X6CA |
| Mixture of all of eight data sets | X6C2N |

The researcher isolated genomic modules using 8 gene expression data sets and 3 mixed data sets (a total of 11 gene expression data sets). The construction of genomic modules is performed according to the genomic module network generation method described above (in the experiment, a basic genomic module network generation technique is used).

**Discovery of Tumor-specific Genomic System**

Genomic module networks were constructed for the respective 11 gene expression data sets. For each of the 11 genomic module networks, modules with low entropy compared to other modules were identified. That is, for each of the 11 genomic module networks, a kernel module was determined. As described above, the kernel module corresponds to a module having entropy lower than a reference value. Meanwhile, a reference value serving as the criterion to determine the kernel module may be a variable value that is adaptively changeable depending on a sample or may be a fixed value. From each of the 11 gene expression data sets, a kernel module formed around TYR and AHSG was isolated.

Genes that are present in the kernel modules of the respective normal tissues but not present in the kernel modules of the tumor tissues were identified from the 11 genomic module networks. A total of seven genes were experimentally identified. All the seven genes were genes found in cancer testis (CT) antigen genes. Specifically, the seven genes were MAGEA1, MAGEA3, MAGEA4, MAGEA10, MAGEA12, CSAG1, and CSAG3A. As described above, genes strongly presumed to be highly related to tumors as the result of the genomic module network-based sample analysis specifically using the kernel modules are called critical genes (CGs). Genes other than the CGs among the genes belonging to the kernel module are called CGX. Table 8 below shows the entropy for the kernel module (Kernel), CGX, and CG extracted from 11 the gene expression data sets.

[Table 8]

| Tissue | Entropy (bits) | | |
|---|---|---|---|
| | Kernel | CGX | CG |
| BRNO | 0.0249 | 0.0235 | 0.0195 |
| CONO | 0.0225 | 0.0218 | 0.0216 |
| BRCA | 0.1072 | 0.1072 | 0.2464 |
| COAD | 0.0546 | 0.0545 | 0.3056 |
| READ | 0.0460 | 0.0460 | 0.1449 |
| LUAD | 0.0563 | 0.0563 | 0.2361 |
| LUSC | 0.0762 | 0.0762 | 1.0368 |
| OV | 0.1030 | 0.1030 | 0.5460 |
| X2NO | 0.0421 | 0.0431 | 0.0246 |
| X6CA | 0.0873 | 0.0873 | 0.4880 |
| X6C2N | 0.1088 | 0.1088 | 0.4128 |

Except for BRCA, OV, and X6C2N, the entropy of the kernel module in each of the remaining data sets was all less than 0.1 bits.

A transcriptional state matrix $\mathbf{T}^{n \times 2^n}$ and a gene expression data set $\mathbf{G}^{n \times m}$ are in a relationship of

$$\overset{n \times m}{\mathbf{G}} = \overset{n \times 2^n}{\mathbf{T}} \overset{2^n \times m}{\mathcal{M}}.$$ $\overset{2^n \times m}{\mathcal{M}}$ is a factor related to the characteristics of individual samples for m samples. Therefore, $\overset{2^n \times m}{\mathcal{M}}$ contains all the different properties, including the deviation attributable to the measurement process of individual samples for $2^n$ transcription states. When $\overset{2^n \times m}{\mathcal{M}}$ is factorized by singular value decomposition (SVD), $\overset{2^n \times m}{\mathcal{M}} = \overset{2^n \times m}{\mathbf{U}} \overset{m \times m}{\mathbf{\Sigma}} \overset{m \times m}{\mathbf{V}^{\top}}$ is obtained. Here, when the samples included in a diagonal matrix are not homogeneous, $\overset{m \times m}{\mathbf{\Sigma}}$ cannot be an identity matrix I. Therefore, the von Neumann entropy $S(\mathbf{G}^{\top}\mathbf{G})$ calculated from the gene expression data becomes larger than the entropy of the transcriptional state $S(\mathbf{T}^{\top}\mathbf{T})$. This may occur for two reasons. First, when an intentional sample selection is performed for a homogeneous sample group or when there is a problem in the sample handling process, it is caused due to the bias of $\mathcal{M}$. Second, when diverse types of samples are mixed, it is possible to escape from the assumption of a single T.

In the case of X6C2N, since data of normal tissues and data of tumor tissues are mixed, the entropy of the kernel module increases because the heterogeneity of T is large. Based on these theories and examples, it is natural that the entropy of the kernel is high in BRCA and OV in which various subtypes are assumed to be included.

On the other hand, since the kernel modules extracted from all the tumor tissues do not contain CG, the entropy of the kernel module and the entropy of CGX are inevitably the same. On the other hand, the entropy of the kernel module and the entropy of CGX were almost the same in the BRNO, CONO, and X2NO. In the kernel module and CGX, the entropy of normal tissue was lower than that of tumor tissue, but the difference was small. On the other hand, there was a significant difference between the entropy of CG in normal tissue and the entropy of CG in tumor tissue. The entropy of CG in tumor tissue was 10 or more times higher than the entropy of CG in normal tissue. This can be interpreted that in the tumor tissue, the CG is isolated from the kernel module and in severe cases (for example, LUSC) completely collapse.

Comparing the kernel module, CGX, and CG in BRNO, CONO, and X2NO, the entropy of the kernel module and CGX in X2NO, which is the mixed data of BRNO and CONO, increased significantly about 1.7 to 2.0 times. The increase in the entropy of the kernel modules is due to CGX, which occupies a significant part in the kernel module. As mentioned above, the entropy increases as samples with different transcription states T are mixed. Therefore, it means that the CGX of BRNO and the CGX of CONO are in different transcriptional states, and it is the starting point of differences in the biological properties of the two tissues.

On the other hand, the entropy of the CG of the X2NO was insignificant compared to that of the BRNO and the CONO. The fact that the increase in entropy of the CG was negligible despite the mixing of several types of samples (i.e., BRNO and CONO) suggests that the CG is a functioning genomic system that functions before the biological difference between the two tissues is generated. Comparing the entropies of CGX and CG of tumor tissue are compared with those of normal tissue, the increase in the entropy of the CG is significantly higher than the increase in the entropy of the CGX. This suggests that the development of tumor tissue is due to the collapse of CG.

To measure the degree of deviation of CG in tumor tissue, relative entropy and angular divergence (AD) were measured. The measured relative entropy and angular divergence are shown in Table 9 below. In the tumor tissue, to calculate the relative entropy of CGX and the relative entropy of CG under the same conditions as in normal tissue, CG was added to the gene set of the kernel module isolated from the tumor tissue to construct a tumor tissue kernel.

[Table 9]

| Tissues | Relative entropy (bits) | | | Angular divergence (degree) | | |
|---------|---------|---------|---------|---------|---------|---------|
| | $S_{CGX\|kr}$ | $S_{CG\|kr}$ | $S_{CG\|CGX}$ | $AD_{CGX,kr}$ | $AD_{CG,kr}$ | $AD_{CG,CGX}$ |
| BRNO | 0.00074 | 0.00789 | 0.02513 | 0.4 | 1.3 | 1.6 |
| CONO | 0.00032 | 0.00888 | 0.01346 | 0.2 | 1.4 | 1.6 |
| BRCA | 0.02625 | 0.48848 | 4.65419 | 2.0 | 15.0 | 17.0 |
| COAD | 0.04417 | 0.64751 | 4.47601 | 2.7 | 20.2 | 22.9 |
| READ | 0.02651 | 0.42065 | 1.41374 | 2.2 | 14.6 | 16.9 |
| LUAD | 0.06173 | 0.83547 | 2.89381 | 3.6 | 23.6 | 27.2 |
| LUSC | 0.06396 | 1.82855 | 14.44333 | 1.9 | 37.5 | 39.4 |
| OV | 0.01333 | 0.62025 | 5.17747 | 1.3 | 13.2 | 14.6 |
| X2NO | 0.00051 | 0.01003 | 0.02347 | 0.3 | 1.3 | 1.6 |
| X6CA | 0.04913 | 1.00963 | 3.08814 | 2.6 | 24.5 | 27.1 |
| X6C2N | 0.03758 | 0.79521 | 2.60642 | 2.2 | 21.3 | 23.6 |

In Table 9, kr denotes a kernel module, $S_{A\|B}$ denotes a relative entropy $S(\rho_A\|\rho_B)$, $AD_{A,B}$ is equal to

$$cos^{-1}(\langle v_1^{\rho A}|v_1^{\rho B}\rangle) \times$$ 180/π denotes a relative entropy. $|v_1^A\rangle$ denotes a first eigenvector of A.

The relative entropy of CGX $S(\rho_{CGX}\|\rho_{kr})$ and the relative entropy of CG $S(\rho_{CG}\|\rho_{kr})$ with respect to the kernel module of the tumor tissue and the kernel module of the normal tissue generated in this way were calculated, and the relative entropies were both increased in tumor tissue. In addition, the relative entropy of CG with respect to CGX $S(\rho_{CG}\|\rho_{CGX})$ was increased in the tumor tissue to the same extent.

In addition, the angular divergence between the first eigenvector $|v_1^{\rho kr}\rangle$ of the density matrix of the kernel module and the first eigenvector $|v_1^{\rho CG}\rangle$ of the density matrix of the CG (AD$_{CG,kr}$), the angular divergence between the first eigenvector $|v_1^{\rho kr}\rangle$ of the density matrix of the kernel module and the first eigenvector $|v_1^{\rho CGX}\rangle$ of the density matrix of CGX (AD$_{CGX,kr}$), and the angular divergence between the first eigenvector $|v_1^{\rho CG}\rangle$ of the density matrix of CG and the first eigenvector $|v_1^{\rho CGX}\rangle$ of the density matrix of CGX (AD$_{CG,CGX}$) were measured. Since the number of genes in CG is smaller than that of CGX, the effect of CG added to the kernel module of the tumor tissue will be limited.

In fact, in tumor tissues, AD$_{CGX,kr}$ was smaller than AD$_{CG,kr}$. That is, AD$_{CG,kr}$ was significantly greater in tumor tissues than in normal tissues (ANOVA test: p = 0.001). In addition, AD$_{CG, CGX}$ was significantly higher in tumor tissues (ANOVA test: p = 0.001). These results indicate that CGX and CG are closely related to each other in normal tissues, whereas in tumor tissues, CGX and CG are separated, and CG tends to collapse.

To determine the degree and direction of transformation of the gene sets CGX and CG as a genomic system in normal and tumor tissues, the relative entropy between the tissues of CGX and CG was calculated. First, to calculate the relative entropy between the tissues of CGX, in a genetic space composed of genes of CGX of a tissue A, the relative entropy $S(\rho_B\|\rho_A)$ of the density matrix $\rho_B$ of a sample group of a tissue B with respect to the density matrix $\rho_A$ of a sample group of the tissue A was calculated. The results of calculation of the relative entropy of CGX between tissues are shown in Table 10 below.

[Table 10]

|  | BRNO | CONO | BRCA | COAD | READ | LUAD | LUSC | OV | X2NO | X6CA | X6C2N |
|---|---|---|---|---|---|---|---|---|---|---|---|
| BRNO | 0.000 | 2.577 | 0.274 | 2.655 | 2.646 | 1.038 | 2.283 | 1.084 | 0.826 | 1.348 | 1.012 |
| CONO | 4.508 | 0.000 | 3.346 | 0.500 | 0.506 | 2.557 | 4.403 | 2.478 | 1.935 | 2.012 | 1.889 |
| BRCA | 0.113 | 1.168 | 0.000 | 0.759 | 0.782 | 0.155 | 0.336 | 0.098 | 0.466 | 0.237 | 0.277 |
| COAD | 0.900 | 0.267 | 0.912 | 0.000 | 0.030 | 0.837 | 0.472 | 0.633 | 0.476 | 0.301 | 0.349 |
| READ | 0.781 | 0.357 | 0.424 | 0.025 | 0.000 | 0.824 | 0.748 | 0.903 | 0.553 | 0.343 | 0.332 |
| LUAD | 0.405 | 4.435 | 0.738 | 2.444 | 2.463 | 0.000 | 0.865 | 0.332 | 2.806 | 0.828 | 1.168 |
| LUSC | 0.318 | 2.559 | 0.127 | 1.721 | 1.778 | 0.032 | 0.000 | 0.089 | 1.139 | 0.371 | 0.554 |
| OV | 0.778 | 0.781 | 0.288 | 0.531 | 0.548 | 0.782 | 0.234 | 0.000 | 0.620 | 0.209 | 0.334 |
| X2NO | 0.007 | 0.008 | 0.271 | 0.265 | 0.269 | 0.935 | 0.714 | 0.713 | 0.000 | 0.525 | 0.275 |
| X6CA | 0.480 | 0.284 | 0.091 | 0.023 | 0.027 | 0.052 | 0.050 | 0.045 | 0.293 | 0.000 | 0.077 |
| X6C2N | 0.085 | 0.161 | 0.120 | 0.037 | 0.038 | 0.100 | 0.075 | 0.092 | 0.095 | 0.073 | 0.000 |

Except for the mixed data sets, the tissue with the lowest relative entropy for CGX in BRNO (normal breast tissue) is BRCA (breast cancer), and the tissue with the lowest relative entropy for CGX in CONO (normal colon tissue) is COAD (colon adenocarcinoma) and READ (rectal adenocarcinoma). In addition, the tissue with the lowest relative entropy for CGX of LUAD (lung adenocarcinoma) is LUSC (lung squamous cell carcinoma), which is another type of lung cancer. This tendency is shown in the dendrogram generated based on the relative entropy between tissues for CGX.

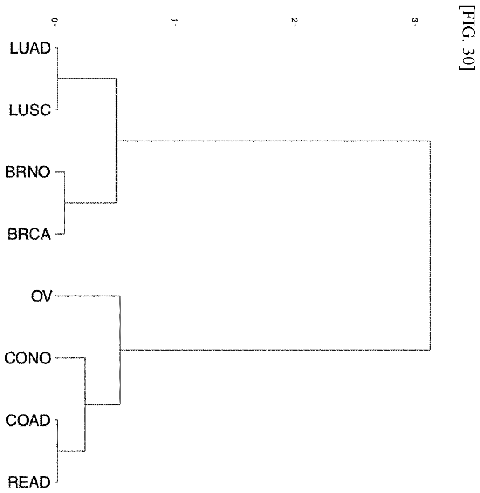

FIG. 30 is a clustering result based on the relative entropy between eight tissues for CGX. FIG. 30 is a dendrogram generated based on the relative entropy between eight tissues for CGX. The dendrogram of FIG. 30 does not distinguish between a tumor tissue and a normal tissue but classifies the tumor tissue and the origin normal tissue together for accurate classification according to the type of tissue.

The results of calculation of the relative entropy of CG between tissues are shown in Table 11.

[Table 11]

|        | BRNO  | CONO  | BRCA  | COAD  | READ  | LUAD  | LUSC  | OV    | X2NO   | X6CA   | X6C2N |
|--------|-------|-------|-------|-------|-------|-------|-------|-------|--------|--------|-------|
| BRNO   | 0.000 | 0.012 | 0.043 | 0.060 | 0.040 | 3.540 | 0.396 | 0.209 | 0.003  | 0.106  | 0.097 |
| CONO   | 0.014 | 0.000 | 0.045 | 0.062 | 0.030 | 0.041 | 0.386 | 0.205 | 0.004  | 0.103  | 0.091 |
| BRCA   | 0.107 | 0.111 | 0.000 | 0.041 | 0.061 | 0.035 | 0.268 | 0.123 | 0.097  | 0.031  | 0.043 |
| COAD   | 0.169 | 0.187 | 0.042 | 0.000 | 0.034 | 0.029 | 0.260 | 0.172 | 0.162  | 0.047  | 0.049 |
| READ   | 0.075 | 0.069 | 0.042 | 0.024 | 0.000 | 0.023 | 0.306 | 0.175 | 0.063  | 0.068  | 0.056 |
| LUAD   | 8.584 | 0.125 | 0.028 | 0.025 | 0.033 | 0.000 | 0.269 | 0.142 | 0.111  | 0.047  | 0.033 |
| LUSC   | 1.533 | 1.573 | 0.532 | 0.645 | 0.892 | 0.629 | 0.000 | 0.184 | 1.475  | 0.238  | 0.268 |
| OV     | 0.646 | 0.636 | 0.190 | 0.322 | 0.415 | 0.253 | 0.146 | 0.000 | 0.606  | 0.090  | 0.070 |
| X2NO   | 0.003 | 0.004 | 0.040 | 0.058 | 0.033 | 0.041 | 0.389 | 0.203 | 0.000  | 8.426  | 0.091 |
| X6CA   | 0.328 | 0.344 | 0.047 | 0.082 | 0.145 | 0.103 | 0.158 | 0.077 | 13.200 | 0.000  | 0.033 |
| X6C2N  | 0.301 | 0.306 | 0.059 | 0.087 | 0.127 | 0.052 | 0.171 | 0.060 | 0.281  | 0.025  | 0.000 |

Except for the mixed data sets, the tissue with the lowest relative entropy for CG in BRNO (normal breast tissue) is CONO (normal colon tissue) which is another normal tissue, and the tissue with the lowest relative entropy for CG in BRCA (breast cancer) is LUAD (lung adenocarcinoma), COAD (colon adenocarcinoma), and READ (rectal adenocarcinoma). In addition, LUSC (lung squamous cell carcinoma) and OV (ovarian cancer), which are highly prone to malignancy, had the lowest relative entropy for CG. This tendency is shown in a dendrogram generated based on the relative entropy between tissues for CG.

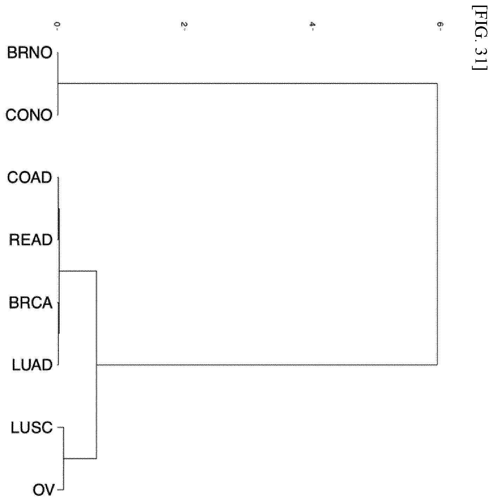

FIG. 31 is a result of clustering based on the relative entropy of 8 tissues for CG. FIG. 31 is a dendrogram generated based on the relative entropy between eight tissues for CG. The dendrogram of FIG. 31 accurately distinguishes normal tissue and tumor tissue and classifies the samples of tumor tissues according to the characteristics of the tumors.

Tumor tissue results in global phenotypic transformation in all cells, including tissues and tumor cells. Attempts have been made to explain such a transformation as a change in the expression level or mutation of a specific gene or gene group. However, conventional studies do not clearly distinguish between normal tissue and tumor tissue on the basis of a single factor.

In contrast, referring to FIGS. 30 and 31, the analysis of the kernel module (i.e., measurement of CG and CGX transformations) after the construction of the genomic module network enables distinguishment between tissue types as well as between normal tissues and tumor tissues. The functional location of the kernel module within the genomic system and the results of the study suggest that tumors begin with CG deviations and CGX transformations.

Hereinafter, whether the analysis based on the kernel module (CG and CGX) is meaningful will be described.

## 1. Breast Cancer-Associated Hormone Receptor)

In tumor tissue, the integrity of a genomic system composed of CG and CGX is related to malignant transformation of cells. Thus, CG and CGX may affect the overall normal function of cells. The researcher analyzed CG and CGX in breast cancer to verify whether this prediction was correct.

In general, a treatment method for breast cancer is determined using the expression of hormone receptors. Hormone therapy is not effective in the case of hormone receptor-negative breast cancer, and drugs for hormone therapy are known to be effective in the case of hormone receptor-positive breast cancer. However, it is problematic to apply these treatments to all breast cancer patients because the treatment effect varies from patient to patient in many cases.

The researcher analyzed the relationship between the integrity of CG and CGX and the expression of hormone receptors in breast cancer. In the analysis below, the researcher classified BRCA and BRNO samples related to breast cancer into a positive expression group for receptors and a negative expression group for receptors, and performed analysis on the groups.

For each of an estrogen receptor (ER), a progesterone receptor (PR), and a human epidermal growth factor receptor 2 (HER2), positive and negative sample groups were isolated for CG and CGX (n = 44, respectively), and the entropy for each sample was calculated. The entropies of CG were measured to be 0.1522, 0.1399, and 0.1986 bits in the positive groups for ER, PR, and HER2, respectively, and 0.4666, 0.3781, and 0.2604 bits in the negative groups for ER, PR and HER2, respectively. That is, the entropy of CG was lower in the positive group than in the negative group for each hormone receptor. The entropies of CGX were measured to be 0.2378, 0.2172, and 0.2708 bits in the positive groups for ER, PR, and HER2, respectively, and 0.2269, 0.2775, and 0.2417 bits in the negative groups for ER, PR, and HER2, respectively. That is, the difference between the entropy of CGX in the positive group and the entropy of CGX in the negative group was insignificant.

In addition, BRCA is a tumor tissue in which CG is isolated from CGX. The relationship between the degree of isolation and the expression state of each receptor was estimated as the relative entropy of CGX and CG. Looking at the relative entropy in BRNO, the relative entropy of CG with respect to CGX was 0.025 bits (n = 28), and the relative entropy of CGX with respect to CG was 0.062 bits (n = 28).

Among BRCA samples (n = 44), in the positive sample groups ER+, PR+, and HER2+, the relative entropies of CG to CGX were 1.4845, 1.5023, and 3.5093 bits, respectively, which were quite high. However, in the negative sample groups, the relative entropies were increased to 7.1480, 5.4585, and 3.0739 bits, respectively. On the other hand, the relative entropies of CGX to CG were 1.5616, 1.5307, and 4.0471 bits in the positive sample groups for the three receptors, respectively, and 6.4663, 5.2620, and 3.1832 bits in the negative sample groups, respectively. In the case of the receptors ER and PR, the relative entropy in the negative sample group was significantly higher than that in the positive sample group. However, in the case of the receptor HER2, the relative entropy in the negative sample group was lower than that in the positive sample group.

The relative entropy of CG to BRNO and the relative entropy of CGX to BRNO in the positive or negative sample groups for each receptor of BRCA were calculated and shown in Table 12 below.

[Table 12] [0408]

|      | $S(\rho_{CG}^{BRCA} \| \rho_{CG}^{BRNO})$ | | $S(\rho_{CGX}^{BRCA} \| \rho_{CGX}^{BRNO})$ | |
| --- | --- | --- | --- | --- |
|      | Negative | Positive | Negative | Positive |
| ER   | 0.3868 | 0.0594 | 0.3440 | 0.2684 |
| PR   | 0.2563 | 0.0556 | 0.3545 | 0.2446 |
| HER2 | 0.1323 | 0.0741 | 0.2758 | 0.3193 |

In general, the relative entropy of the genomic module of BRCA to the genomic module of BRNO means the degree of deviation from the normal state. In the case of the receptors ER and PR, when the degree of deviation of CG from the normal state was large, the receptors were not expressed. However, in the case of HER2, the expression and non-expression of the receptor HER2 was determined by a small difference in the degree of deviation of CGX. In the case of the receptor HER2, when CG is deviated from the normal state, the receptor expression was suppressed, but the deviation of CGX from the normal state promoted the receptor expression. That is, the expression of HER2 is increased when CG is slightly deviated from the normal state and CGX is significantly deviated from the normal state. On the other hand, ER and PR were expressed when both CG and CGX were slightly deviated from BRNO.

The MSP of each sample can be calculated based on CG and CGX. $MSP_{CG}$ and $MSP_{CGX}$ mean the MSPs of each sample calculated based on CG and CGX, respectively. FIG. 32 is a result of calculating $MSP_{CG}$ and $MSP_{CGX}$ for 248 BRCA samples and shows the distribution of $MSP_{CG}$'s and $MSP_{CGX}$'s according to the expression of the receptor. FIG. 32A is an example showing the distribution of $MSP_{CG}$'s according to receptor expression in the BRCA sample, FIG. 32B is an example showing the distribution of $MSP_{CGX}$'s according to the receptor expression in the BRCA sample.

In FIG. 32A, the distribution of $MSP_{CG}$'s showed a significant difference in the positive and negative sample groups for each of the three receptors, and the median value of $MSP_{CG}$'s in the positive sample group of each receptor was compared to the median value of $MSP_{CG}$'s in the negative sample group of each receptor was significantly larger. In FIG. 32B, $MSP_{CGX}$'s in the positive sample groups ER+ and PR+ were significantly larger than those in the negative sample groups. On the other hand, in the case of HER2, although not significant, $MSP_{CGX}$ was greater in the negative sample group than in the positive sample group.

On the other hand, in the case of triple negative breast cancer (TNBC) sample groups ER-, PR-, and HER2-, $MSP_{CG}$ and $MSP_{CGX}$ showed a significant difference from the rest of the sample groups (p-value < 0.005).

To further clearly confirm this, BRCA samples were divided according to the levels of $MSP_{CG}$ and $MSP_{CGX}$, and a significant difference in receptor expression was examined through a binomial test. Only when the $MSP_{CG}$ was less than 0.9932, the expression of ER was significantly reduced compared to all the modules of BRCA. When $MSP_{CGX}$ was greater than 0.9885, the expression of each of ER and PR was significantly increased compared to all the modules of BRCA. In the case of HER2, there was no notable change according to the level of $MSP_{CG}$ or $MSP_{CGX}$. In normal breast tissue, CG and CGX are included in the kernel module. Therefore, in breast cancer, there is no choice but to have a close relationship between the two genomic systems.

The researcher generated BRCA sample groups "hh", "hl", "lh", and "11" according to the levels of $MSP_{CG}$ and $MSP_{CGX}$ (See Table 13 below).
[Table 13]

| | $MSP_{CG} \geq Th_{CG}$ | $MSP_{CG} < Th_{CG}$ |
|---|---|---|
| $MSP_{CGX} \geq Th_{CGX}$ | *hh* | *hl* |
| $MSP_{CGX} < Th_{CGX}$ | *lh* | *ll* |

In Table 13, $Th_{CG}$ and $Th_{CGX}$ refer to reference points for dividing samples according to $MSP_{CG}$ and $MSP_{CGX}$. Hereinafter, the case where $Th_{CG} = 0.9932$ and $Th_{CGX} = 0.9885$ will be described.
Among the four BRCA sample groups, the group lh was excluded because the number of samples was only 11. After calculating the expression frequencies of ER, PR, and HER2 in each of the remaining three BRCA sample groups, the significance between the positive and negative groups of each receptor was verified by a binary test (Table 14 below).
[Table 14]

| | ER | | PH | | HER2 | |
|---|---|---|---|---|---|---|
| | negative | positive | negative | positive | negative | positive |
| *hh* | 9 | 50 | 18 | 41 | 45 | 10 |
| *hl* | 22 | 69 | 36 | 56 | 55 | 24* |
| *ll* | 33** | 49 | 42* | 40 | 66* | 8 |

*: $p < .05$, **: $p < .01$ in binomial test

A binary test for the positive and negative groups of ER and PR shows that, as expected, the frequency of the negative samples of each of receptors was significantly higher in the sample group 11, that is, the BRCA sample group with a low $MSP_{CG}$ and a low $MSP_{CGX}$.
On the other hand, in the case of HER2, the frequency of negative samples was significantly higher in the sample group 11, and the frequency of positive samples was significantly higher in the sample group hl, that is, the sample group with a high $MSP_{CG}$ and a low $MSP_{CGX}$. These results are consistent with those in Table 12 in which the relative entropy of CG to BRNO is relatively low and the relative entropy of CGX to BRNO is relatively high in the HER2-positive sample group compared to the HER2-negative sample group.
The expression of ER and PR depended on the degree of collapse of the genomic systems of CG and CGX. Particularly, the dependence of CG on the genomic system was high. In the BRCA sample group hl in which the expression of HER2 was significantly increased, the relative entropies of BRNO to CG and CGX were 0.0023 and 0.4018 bits, respectively. That is, in the HER2+ sample, the degree of collapse of CGX related to cell differentiation is large, but the integrity of CG, which is the starting point of tumorigenicity, is maintained. From this, it can be presumed that the samples exhibit the characteristics of an undifferentiated cell that has lost control of cell proliferation.
On the other hand, in the BRCA sample group 11, the relative entropies of BRNO to CG and CGX were increased to 0.6986 and 0.4357 bits, respectively, indicating that the genomic system capable of expressing HER2 is collapsed. Therefore, a decrease in HER2 expression may be seen in sample group 11. On the other hand, even in the BRCA sample group hh, that is, the sample group in which the genomic systems of both CG and CGX were well preserved and the characteristics of differentiated epithelial cells were well maintained, more HER2- samples than HER2+ samples were included (See Table 14). That is, HER2- samples are present in both sample groups hh and 11.
Therefore, the malignancy of breast cancer associated with HER2 expression is high in all the HER2+ samples and in the sample group 11 of the HER2- samples. On the other hand, since the expression of ER and PR in the sample group 11 is likely to be lowered, breast cancer patients with high malignancy have a high probability that all three receptors will eventually become triple negative.
Summarizing these results, the genomic systems of CG and CGX are involved in the degree of cell differentiation and tumorigenicity in breast cancer, thereby being related to the expression of ER, PR, and HER2.

## 2. Genomic Modules Associated with CG and CGX

The gene expression data BRNO of a normal breast tissue in TCGA consists of 28 samples. Tissues determined to be pathologically normal among tissues excised by surgery for breast cancer were used. The researcher calculated MSPs of CG and CGX for BRNO samples. $MSP_{CG}$s and $MSP_{CGX}$s were distributed in a range of 0.9911 to 0.9989 and in a range of 0.9921 to 0.9984, respectively. The lower value of the two probability distributions is smaller than the maximum value of breast cancer. This implies that the potential for transformation into a tumor in the genomic system is high although the sample are pathologically normal.

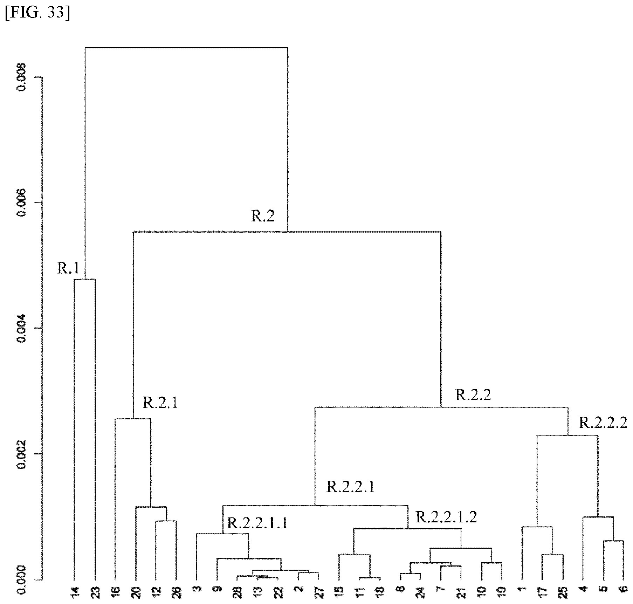

Therefore, hierarchical clustering was performed on the BRNO samples on the basis of $MSP_{CG}$ and $MSP_{CGX}$. FIG. 33 is a result of clustering based on $MSP_{CG}$ and $MSP_{CGX}$ for the BRNO samples. FIG. 33 is a dendrogram of a BRNO sample generated based on $MSP_{CG}$ and $MSP_{CGX}$ of the BRNO sample. The dendrogram of the BRNO sample suggests that the genomic systems of the BRNO sample may have heterogeneous properties with each other.

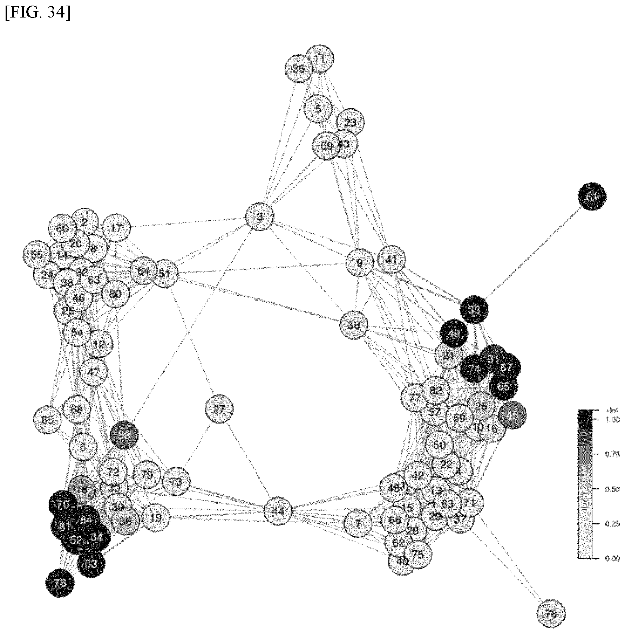

FIG. 34 illustrates an example of representing the relative entropy of each genomic module with respect to the kernel module in the intermodular network of BRNO. FIG. 34 is a graph showing the relative entropy of each genomic module with respect to the kernel module calculated from the data of the entire BRNO sample. In the BRNO intermodular network of FIG. 34, domains and modules belonging to the domains are shown in Table 15.

[Table 15

| Abbrv. | Domain | Module No. |
|---|---|---|
| *kn* | kernel | 2, 8, 14, 20, 26, 32, 38, 46, 54, 60 |
| *st* | stroma | 1, 7, 13, 15, 28, 29, 40, 42, 45, 48, 62, 66, 75 |
|  | angio | 4, 10, 16, 21, 22, 37, 50, 57, 59, 71, 77, 82, 83 |
|  | adipo | 25, 31, 33, 49, 61, 65, 67, 74 |
| *cc* | ccdr | 5, 11, 23, 35, 43, 69 |
| *pr* | epi | 34, 52, 53, 70, 76, 81, 84 |
|  | epi.base | 6, 12, 18, 19, 30, 39, 56, 58, 68, 72, 79, 85 |
| *meta* | meta.cc | 3, 17, 24, 51, 55, 63, 80 |
|  | meta. 1 | 9, 36, 41, 64 |
|  | meta.2 | 44, 47 |
|  | meta.3 | 27, 73, 78 |

In this BRNO genomic system, modules are around a kernel module. Here, meta means the meta domain as described above. In some modules of the adipose tissue-forming domain (adipo), the relative entropy increased to 0.9265 to 4.3553 bits, and in the modules included in the epithelial domain (epi), the relative entropy increased to 1.0170 to 1.9703 bits.

To verify that the modules of the epithelial domain (epi) deviate from the dominance of the kernel module, the relatively entropy of each module with respect to the kernel module was calculated on the basis of samples (sample indices of 15, 11, 18, 8, 24, 7, 21, 10 and 19) on branches (R.2.2.1.2) whose $MSP_{CG}$ and $MSP_{CGX}$ are close to 1 in the dendrogram of FIG. 33.

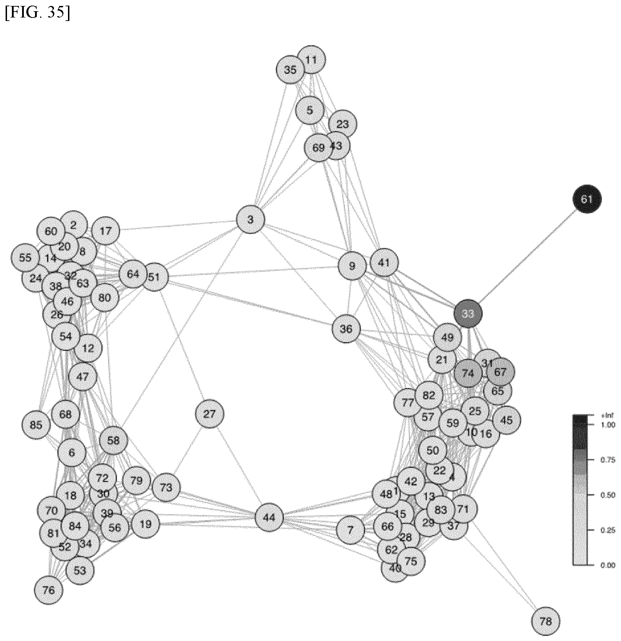

FIG. 35 illustrates an example of displaying the relative entropy of each genomic module with respect to a kernel module for some samples in the intermodular network of BRNO. FIG. 35 is a graph of the relative entropy of each genomic module with respect to the kernel module calculated based on the samples belonging to the branch R.2.2.1.2 among the branches of the BRNO sample dendrogram. As shown in FIG. 35, the relative entropy (0.0496 to 0.2131 bits) of each of the modules belonging to the epithelial domain (epi) was significantly reduced, and the relative entropy of each of some modules belonging to the adipose tissue-forming domain (adipo) was also reduced. This result suggests that, among normal breast samples, the kernel module dominates almost all modules of the genomic system in the case of samples in which the genomic system is estimated to be normal.

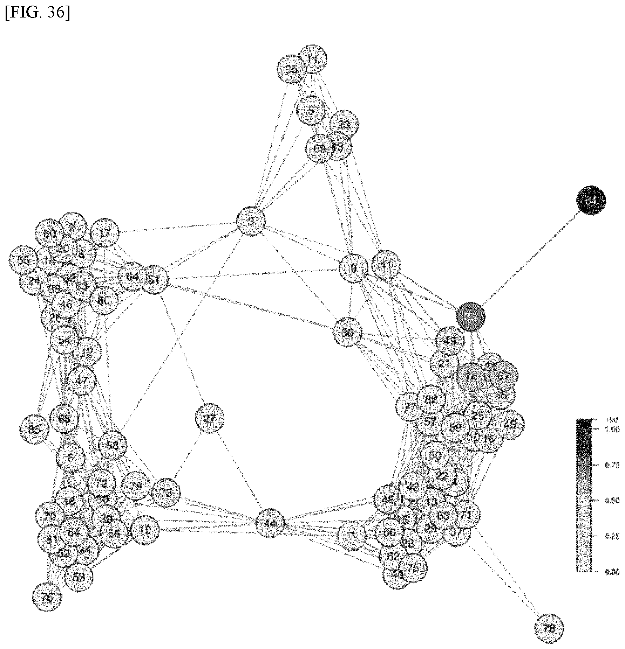

The researcher calculated the relative entropy of each of the BRNO modules with respect to CGX, which constitutes a significant part of the kernel module, using some of the BRNO samples. FIG. 36 is an example of displaying the relative entropy of each genomic module with respect to the CGX of the kernel module for some samples in the intermodular network of BRNO. FIG. 36 is a graph showing the relative entropy of each genomic module with respect to the CGX of the kernel module calculated with samples belonging to the branch R.2.2.1.2 among the branches of the BRNO sample dendrogram. Referring to FIG. 36, the relative entropy of FIG. 36 is similar to the relative entropy of each genomic module with respect to the entire kernel module of FIG. 35.

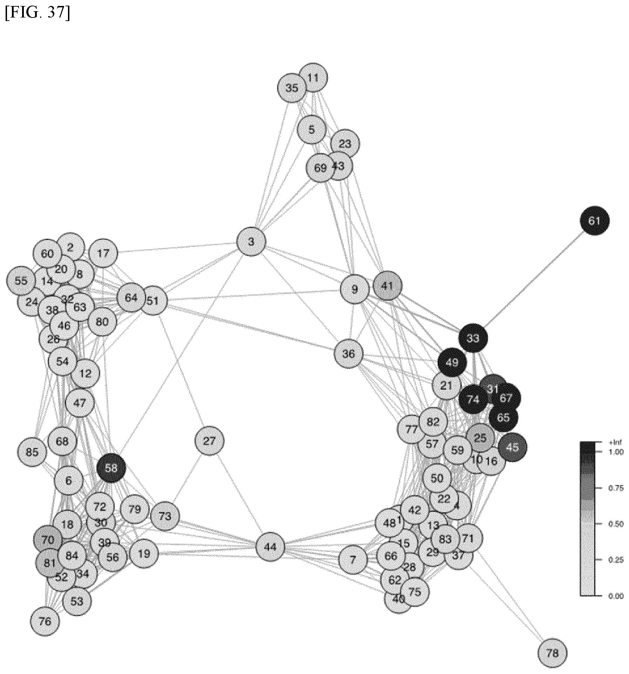

The researcher calculated the relative entropy of BRNO modules with respect to CG, which constitutes a significant part of the kernel module, using some of the BRNO samples. FIG. 37 is an example of displaying the relative entropy of each genomic module with respect to the CG of the kernel module, for a part of the samples in the intermodular network of BRNO. FIG. 37 is the relative entropy of each genomic module with respect to the CG of the kernel module calculated with samples belonging to the branch R.2.2.1.2 among the branches of the BRNO sample dendrogram. In all modules, the relative entropy to CG was greater than the entropy to CGX. Therefore, it is assumed that the CG is indirectly connected to the modules of the BRNO.

Among the branches of the BRNO sample dendrogram of FIG. 33, that is, the branches R.1, R.2.1, R.2.2.1.1, R.2.2.1.2, and R.2.2.2, the branch R.1 has only two samples. Therefore, the branch R.1 is not suitable for the calculation of entropy. Therefore, the relative entropy of CG to CGX for each branch except for the branch R.1 was calculated. The results show that the relative entropy of CG to CGX on the branch R.2.1 was the largest as 0.0196 bits.

The average value of the relative entropy $S(\rho_i\|\rho_2)$ of each genomic module to the kernel module on each of the sample branches R.2.1, R.2.2.1.1, R.2.2.1.2, and R.2.2.2 in the BRNO sample dendrogram was calculated. The average values were 0.2175, 0.3582, 0.0843, and 0.1094 bits, respectively. The average values of the branches R.2.2.1.1 and R.2.1 are relatively high. The standard deviations are 0.2943, 0.6219, 0.1485, and 0.1739, respectively. That is, the standard deviation is exceptionally large in the branch R.2.2.1.1. This is because the relative entropy of each of the modules of the epithelial domain (epi) and the adipose tissue-forming domain (adipo) is extraordinarily increased. Therefore, it is assumed that in the branch R.2.1, the dominance of CGX over each genomic module decreased overall due to the deviation of CG from the normal state, whereas in R.2.2.1.1, a more complex mechanism was presumed to be involved.

### 3. Predictive Analysis of Tumor Development on Normal Sample

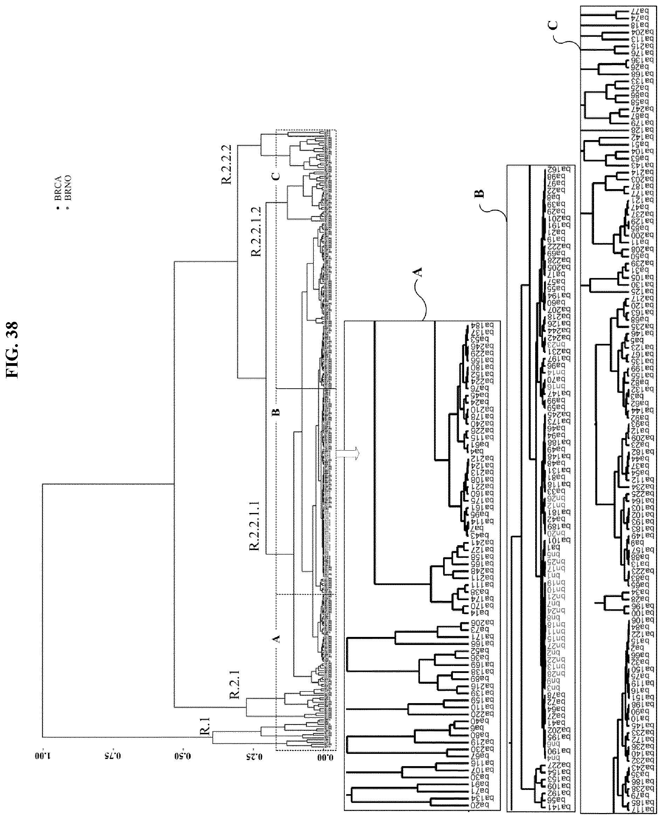

To clearly identify the characteristics of BRNO samples, BRNO samples were combined with breast cancer (BRCA) samples, the MSPs of BRNO to CG and CGX were calculated, and the hierarchical clustering was performed. FIG. 38 illustrates a result of clustering based on the MSPs of the BRNO samples and the BRCA samples, in which the MPSs were calculated with respect to CG and CGX. The lower part of FIG. 38 is an enlarged view showing the names of samples belonging to the classified branches.

Most of the BRNO samples (19 samples) were separated from the BRCA samples, but the samples #4, #5, and #6 included in the branch R.2.2.2 among the branches of the BRNO sample dendrogram were mixed with the BRCA samples in the immediately adjacent branches. The samples #12, #20 and #26 in the branch R.2.1 were separately present in more distant branches and mixed with the BRCA samples. The samples #14 and #23 of the branch R.1 and the sample #16 of the branch R.2.1 were separately present in quite distant branches and mixed with the BRCA samples. This suggests that transformations may proceed in the genomic system before pathologically distinguishing tumor cells. That is, the BRNO samples classified into the same branches as the BRCA samples may be regarded as samples in which transformation into tumor tissue has begun (potentially developed into tumor), unlike normal tissue.

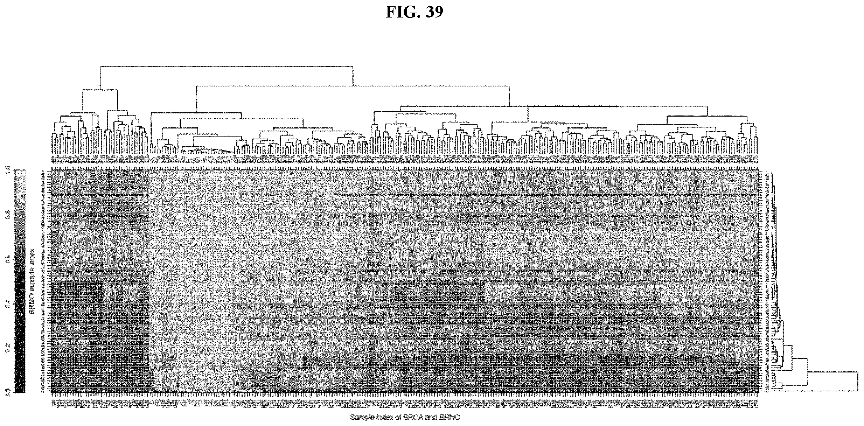

To check the transformation of the genomic system in the BRNO sample, the MSPs of the BRNO and BRCA samples for all the BRNO genomic modules were calculated and a level plot was prepared. FIG. 39 illustrates an example of a level plot clustering based on the MSPs of the BRNO and BRCA samples, calculated for all BRNO genomic modules. In FIG. 39, the horizontal axis represents the hierarchical clustering result of BRNO and BRCA samples, and the vertical axis represents the hierarchical clustering result of all BRNO genomic modules. As expected, the BRNO and BRCA samples except for a few samples were divided into different branches in the dendrogram. In the case of the samples 2 and 3 escaped from the branches of the BRNO samples, the MSPs thereof with respect to the modules of the epithelial domain (epi) were reduced to the MSP level of the BRCA samples. In the sample 9 among the BRNO samples, the MSPs thereof with respect to the modules 76 and 81 were as low as 0.3463. and 0.3118, respectively, and the MSPs thereof with respect to the other modules were in a range of 0.8 to 0.9 which are difficult to be considered normal. In addition, the MSPs of the samples 20, 15, and 22 among the BRNO samples indicate that in these samples, the module 61 associated with adipogenesis were collapsed and the modules 49 and 33 were partially collapsed. The sample 12 of the BRNO samples was not present in any branch of the BRNO samples but was included in the same branch as the BRCA samples. The MSP of the sample 12 of the BRNO samples exhibited the lowest value among 30 modules of a total of 85 BRNO samples, which is largest among the BRNO samples.

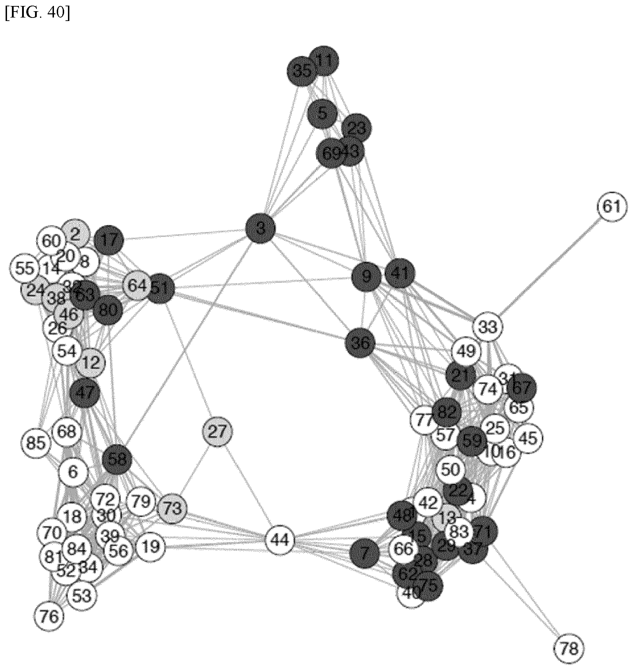

These modules 30 belong to domains related to the cell cycle, stroma, and angiogenesis. FIG. 40 illustrates an example in which the module with the lowest MSP among the MPSs of the BRNO samples 12 with respect to the BRNO genomic modules is an intermodular network of BRNO. FIG. 10 illustrates an example in which the MSP of the BRNO sample 12 has the lowest value in an intermodular network of the BRNO sample 12, constructed using all BRNO samples.

These results suggest that it is difficult to say that all BRNO samples of TCGA are in the normal range in terms of genomic system transformation. Particularly, in the case of the samples 2, 3, and 9, the modules of the epithelial domain (epi) are transformed. In general, an increase in relative entropy can occur through the collapse and transformation of modules. The collapse can be distinguished by an increase in entropy, and the transformation can be distinguished by the angular divergence of the eigenvectors. The results of calculating the angular divergence of each sample for each of the modules 34, 52, 53, 70, 76, 81, 84 of the epithelial domain (epi) are shown in Table 16 below.

[Table 16]

|  | sample | 34 | 52 | 53 | 70 | 76 | 81 | 84 |
|---|---|---|---|---|---|---|---|---|
| R.1 | 14 | -1.884 | -4.721 | -2.082 | -9.311 | -1.935 | -6.308 | -2.143 |
|  | 23 | -2.129 | -5.144 | -2.064 | -7.377 | -2.095 | -6.069 | -2.362 |
| R.2.1 | 16 | -2.234 | -5.424 | -2.202 | -8.798 | -2.252 | -7.249 | -2.499 |
|  | 20 | -2.107 | -4.625 | -1.936 | -6.415 | -2.072 | -5.543 | -2.401 |
|  | 12 | -2.513 | -6.615 | -2.494 | -10.149 | -2.542 | -8.812 | -3.032 |
|  | 26 | -2.431 | -6.695 | -2.671 | -9.805 | -2.481 | -8.726 | -2.791 |
| R.2.2.1.1 | 3 | -5.350 | -19.493 | -0.316 | -18.666 | 4.194 | -7.461 | -6.731 |
|  | 9 | -3.843 | -17.339 | -6.225 | -16.479 | -3.922 | -12.781 | -4.762 |
|  | 28 | -2.018 | -4.818 | -2.102 | -8.739 | -2.077 | -6.202 | -2.297 |
|  | 13 | -2.040 | -4.682 | -1.972 | -6.625 | -2.006 | -5.740 | -2.191 |
|  | 22 | -2.040 | -4.743 | -1.889 | -6.593 | -2.133 | -5.709 | -2.227 |
|  | 2 | -3.181 | -12.316 | 0.838 | -19.941 | 4.248 | -13.433 | -5.999 |
|  | 27 | -1.989 | -4.650 | -2.009 | -8.026 | -2.000 | -5.700 | -2.198 |
| R.2.2.1.2 | 15 | -2.177 | -5.283 | -2.224 | -7.809 | -2.158 | -6.112 | -2.243 |
|  | 11 | -2.078 | -4.892 | -1.993 | -7.252 | -2.137 | -6.053 | -2.231 |
|  | 18 | -2.152 | -4.783 | -2.110 | -6.797 | -2.112 | -5.768 | -2.188 |
|  | 8 | -2.291 | -5.435 | -2.386 | -8.064 | -2.294 | -6.731 | -2.436 |
|  | 24 | -2.132 | -5.473 | -2.204 | -8.022 | -2.126 | -7.194 | -2.385 |
|  | 7 | -2.188 | -5.660 | -2.432 | -8.783 | -2.307 | -7.249 | -2.403 |
|  | 21 | -2.151 | -5.024 | -2.084 | -7.454 | -2.142 | -6.247 | -2.363 |
|  | 10 | -2.096 | -4.821 | -2.036 | -6.671 | -2.037 | -6.246 | -2.309 |
|  | 19 | -2.074 | -5.024 | -2.137 | -7.377 | -2.086 | -6.182 | -2.199 |
| R.2.2.2 | 1 | -2.110 | -6.239 | -2.393 | -11.090 | -2.176 | -8.019 | -2.396 |
|  | 17 | -2.070 | -4.663 | -1.978 | -7.659 | -2.069 | -5.710 | -2.329 |
|  | 25 | -2.123 | -4.867 | -2.098 | -6.840 | -2.117 | -6.317 | -2.423 |
|  | 4 | -2.532 | -7.891 | -2.848 | -13.601 | -2.720 | -10.989 | -3.001 |
|  | 5 | -2.065 | -5.716 | -2.075 | -9.361 | -2.269 | -7.124 | -2.610 |
|  | 6 | -2.168 | -4.999 | -2.190 | -7.967 | -2.249 | -5.902 | -2.426 |

Although the angular divergence is large only in the samples 2, 3, and 9 among the samples in the branch R.2.2.1.1 of the dendrogram of the BRNO samples, the angular divergence was calculated for all samples belonging to the branch for entropy calculation. Entropy increased in the branch R.2.2.1.1. Therefore, it is assumed that the epithelial domain (epi) is transformed and collapsed in the samples of the branch R.2.2.1.1.

As described above, the connectivity between genomic modules can be estimated based on the relative entropy. Density matrices of two genomic modules are calculated using gene expression data obtained from a group of samples having the same property in a specific aspect, and the relative entropy is measured from these density matrices. Since the relative entropy is obtained in the sample space, it is possible to estimate the connectivity for the entire sample. However, with the relative entropy, it is impossible to estimate the connectivity between modules in an individual sample. To estimate the connectivity between modules in an individual sample, the researcher created an index called single sample modular connectivity (SSMC). The SSMC will be described first.

To estimate the connectivity between genomic modules in an individual sample, the researchers hypothesized a set of genes c shared by the two modules and sets of genes a and b unique to each module. The sample vector $|s_i\rangle$ of a sample i consists of sample vectors $|s^c_i\rangle$, $|s^a_i\rangle$, and $|s^b_i\rangle$. When the density matrices of the respective modules are

defined as $\rho_{c,a}$ and $\rho_{c,b}$, and the integrated density matrix of the two genomic modules is defined as p, the relationship between the entropies of the two modules is $S(\rho) \leq S(\rho_{s,a}) + (\rho_{s,b})$. At this time, if the relative entropy between the two modules is sufficiently low, $S(p) \ll S(\rho_{s,a}) + (\rho_{s,b})$ is established. In the opposite case, it approaches $S(\rho) = S(\rho_{s,a}) + (\rho_{s,b})$. Therefore, in the former case, $\rho$ is an ellipse with a large ratio of the eigenvalue of the major axis to the eigenvalue of the minor axis. In the latter case, as the relative entropy between the two modules increases, the ratio of the eigenvalue of the major axis to the eigenvalue of the minor axis decreases, thereby gradually approaching a circular shape. Therefore, the probability $p_i$ of the sample i for the integrated density matrix $\rho$ of the two genomic modules is $<S_i|\rho|S_i>$, and the probability of all samples decreases and regardless whether the probability for the modules a and b is high or low. On the other hand, the probability $p_i$ of the sample i for the combination of the two modules is expressed as Equation 17 below as a function of the gene sets a, b and the shared gene set c.

[Equation 17]

$$p_i = \frac{1}{\|s_i^c\|^2 + \|s_i^a\|^2 + \|s_i^b\|^2} \left(\langle s_i^c| \quad \langle s_i^a| \quad \langle s_i^b|\right) \rho \begin{pmatrix} |s_i^c\rangle \\ |s_i^a\rangle \\ |s_i^b\rangle \end{pmatrix}$$

Here, the integrated density matrix $\rho$ of the two genomic modules is composed of the density matrices $\rho_c$, $\rho_a$, and $\rho_b$ of the respective gene sets and asymmetric matrices $\rho_{ca}$, $\rho_{cb}$, $\rho_{ac}$, $\rho_{ab}$, and $\rho_{ac}$ generated between them as shown in Equation 18 below.

[Equation 18]

$$\rho = \begin{pmatrix} \gamma\rho_c & \rho_{ca} & \rho_{cb} \\ \rho_{ac} & \alpha\rho_a & \rho_{ab} \\ \rho_{bc} & \rho_{ba} & \beta\rho_b \end{pmatrix}$$

Here, $\gamma = \|s_i^c\|^2/(\|s_i^c\|^2 + \|s_i^a\|^2 + \|s_i^b\|^2)$, $\alpha = \|s_i^a\|^2/(\|s_i^c\|^2 + \|s_i^a\|^2 + \|s_i^b\|^2)$, and $\beta = \|s_i^b\|^2/(\|s_i^c\|^2 + \|s_i^a\|^2 + \|s_i^b\|^2)$ are established.

Accordingly, $p_i$ may be expressed as in Equation 19 shown below.

[Equation 19]

$$p_i = \frac{1}{\|s_i^c\|^2 + \|s_i^a\|^2 + \|s_i^b\|^2} (\gamma p_i^c + \alpha p_i^a + \beta p_i^b + 2\langle s_i^a|\rho_{ac}|s_i^c\rangle + 2\langle s_i^b|\rho_{bc}|s_i^c\rangle + 2\langle s_i^a|\rho_{ab}|s_i^b\rangle)$$

Here, $p_i^c = \langle s_i^c|\rho_c|s_i^c\rangle$, $p_i^a = \langle s_i^a|\rho_a|s_i^a\rangle$, and $p_i^b = \langle s_i^b|\rho_b|s_i^b\rangle$ are established, and all the terms except for $\gamma p_i^c$ and $\langle s_i^a|\rho_{ab}|s_i^b\rangle$ determines the characteristics of samples in each module. Therefore, the connectivity between the two modules in the sample i is irrelevant. The gene set c shared by the two modules increases the connectivity between the two modules. Particularly, the greater the $\gamma$, the greater the connectivity. The term $\langle s_i^a|\rho_{ab}|s_i^b\rangle$ indicates the connectivity formed by the genes that are not shared by the two modules and has significance when the number of genes shared by the modules is small. Therefore, the probability $p_i$ of the sample i for the case where the two modules are combined comprehensively indicates the integrity and connectivity of the two modules. Reference symbol $p_i$ denotes the SSMC index.

The SSMC was calculated in each BRNO sample to examine the relationship between the kernel module and the modules of the epithelial domain (epi). Table 14 below shows the SSMC between the BRNO module 6 and the modules of the epithelial domain (epi) in each sample of BRNO. Table 15 below shows the SSMC between the BRNO module 68 and the modules of the epithelial domain (epi) in each sample of BRNO.

Referring to Table 17 and Table 18, it is confirmed that the SSMCs of the BRNO samples 2, 3, and 9 belonging to the branch R.2.2.1.1 in the BRNO sample dendrogram are reduced in all modules of the epithelial domain (epi). This means that information exchange with that module has been cut off or reduced. The modules closest to the epithelial domain (epi) on the BRNO intermodular network are the modules 6 and 68.

[Table 17]

| sample | 34 | 52 | 53 | 70 | 76 | 81 | 84 |
|---|---|---|---|---|---|---|---|
| 1 | 0.965 | 0.956 | 0.950 | 0.946 | 0.946 | 0.950 | 0.954 |
| 2 | 0.290 | 0.717 | 0.234 | 0.866 | 0.049 | 0.810 | 0.586 |
| 3 | 0.414 | 0.737 | 0.232 | 0.852 | 0.066 | 0.741 | 0.652 |
| 4 | 0.957 | 0.925 | 0.931 | 0.932 | 0.921 | 0.924 | 0.943 |
| 5 | 0.965 | 0.959 | 0.954 | 0.953 | 0.937 | 0.956 | 0.953 |
| 6 | 0.969 | 0.957 | 0.960 | 0.961 | 0.952 | 0.954 | 0.964 |
| 7 | 0.971 | 0.967 | 0.964 | 0.969 | 0.954 | 0.964 | 0.965 |
| 8 | 0.970 | 0.962 | 0.962 | 0.969 | 0.954 | 0.963 | 0.965 |
| 9 | 0.824 | 0.806 | 0.691 | 0.909 | 0.481 | 0.798 | 0.796 |
| 10 | 0.971 | 0.960 | 0.962 | 0.957 | 0.951 | 0.956 | 0.965 |
| 11 | 0.973 | 0.965 | 0.962 | 0.966 | 0.950 | 0.963 | 0.968 |
| 12 | 0.957 | 0.946 | 0.945 | 0.942 | 0.934 | 0.938 | 0.939 |
| 13 | 0.974 | 0.965 | 0.965 | 0.965 | 0.958 | 0.962 | 0.969 |
| 14 | 0.960 | 0.948 | 0.940 | 0.942 | 0.941 | 0.949 | 0.955 |
| 15 | 0.968 | 0.959 | 0.950 | 0.963 | 0.952 | 0.957 | 0.960 |
| 16 | 0.971 | 0.962 | 0.963 | 0.966 | 0.955 | 0.962 | 0.966 |
| 17 | 0.972 | 0.960 | 0.960 | 0.967 | 0.957 | 0.957 | 0.968 |
| 18 | 0.967 | 0.965 | 0.956 | 0.964 | 0.953 | 0.960 | 0.964 |
| 19 | 0.972 | 0.966 | 0.964 | 0.967 | 0.955 | 0.963 | 0.965 |
| 20 | 0.963 | 0.955 | 0.959 | 0.950 | 0.949 | 0.950 | 0.957 |
| 21 | 0.973 | 0.960 | 0.963 | 0.961 | 0.955 | 0.957 | 0.965 |
| 22 | 0.971 | 0.965 | 0.963 | 0.964 | 0.943 | 0.960 | 0.967 |
| 23 | 0.965 | 0.942 | 0.955 | 0.943 | 0.949 | 0.935 | 0.958 |
| 24 | 0.972 | 0.961 | 0.962 | 0.963 | 0.955 | 0.955 | 0.963 |
| 25 | 0.972 | 0.962 | 0.956 | 0.962 | 0.955 | 0.961 | 0.961 |
| 26 | 0.963 | 0.950 | 0.946 | 0.958 | 0.939 | 0.944 | 0.952 |
| 27 | 0.969 | 0.958 | 0.959 | 0.962 | 0.953 | 0.954 | 0.966 |
| 28 | 0.972 | 0.964 | 0.962 | 0.963 | 0.956 | 0.964 | 0.965 |

[Table 18]

| sample | 34 | 52 | 53 | 70 | 76 | 81 | 84 |
|---|---|---|---|---|---|---|---|
| 1 | 0.960 | 0.960 | 0.947 | 0.957 | 0.940 | 0.958 | 0.953 |
| 2 | 0.426 | 0.816 | 0.384 | 0.911 | 0.189 | 0.877 | 0.659 |
| 3 | 0.502 | 0.798 | 0.371 | 0.873 | 0.214 | 0.810 | 0.682 |
| 4 | 0.950 | 0.935 | 0.926 | 0.942 | 0.918 | 0.936 | 0.941 |
| 5 | 0.962 | 0.966 | 0.952 | 0.965 | 0.937 | 0.966 | 0.955 |
| 6 | 0.961 | 0.956 | 0.952 | 0.963 | 0.944 | 0.955 | 0.959 |
| 7 | 0.967 | 0.970 | 0.959 | 0.974 | 0.949 | 0.969 | 0.965 |
| 8 | 0.964 | 0.964 | 0.955 | 0.970 | 0.947 | 0.965 | 0.962 |
| 9 | 0.827 | 0.854 | 0.711 | 0.921 | 0.567 | 0.857 | 0.808 |
| 10 | 0.965 | 0.961 | 0.955 | 0.962 | 0.944 | 0.960 | 0.961 |
| 11 | 0.967 | 0.968 | 0.958 | 0.971 | 0.945 | 0.968 | 0.966 |
| 12 | 0.952 | 0.950 | 0.941 | 0.951 | 0.929 | 0.947 | 0.941 |
| 13 | 0.967 | 0.966 | 0.959 | 0.968 | 0.950 | 0.965 | 0.966 |
| 14 | 0.948 | 0.947 | 0.934 | 0.951 | 0.929 | 0.951 | 0.946 |
| 15 | 0.964 | 0.962 | 0.949 | 0.967 | 0.946 | 0.961 | 0.957 |
| 16 | 0.964 | 0.962 | 0.956 | 0.967 | 0.947 | 0.964 | 0.962 |
| 17 | 0.965 | 0.960 | 0.954 | 0.966 | 0.948 | 0.959 | 0.963 |
| 18 | 0.960 | 0.963 | 0.950 | 0.964 | 0.945 | 0.961 | 0.960 |
| 19 | 0.966 | 0.965 | 0.958 | 0.967 | 0.947 | 0.964 | 0.961 |
| 20 | 0.958 | 0.956 | 0.951 | 0.955 | 0.942 | 0.954 | 0.955 |
| 21 | 0.967 | 0.963 | 0.957 | 0.967 | 0.949 | 0.963 | 0.963 |
| 22 | 0.963 | 0.962 | 0.956 | 0.964 | 0.937 | 0.960 | 0.962 |
| 23 | 0.956 | 0.945 | 0.946 | 0.948 | 0.938 | 0.942 | 0.952 |
| 24 | 0.966 | 0.964 | 0.957 | 0.967 | 0.948 | 0.961 | 0.961 |
| 25 | 0.965 | 0.960 | 0.950 | 0.962 | 0.947 | 0.961 | 0.958 |
| 26 | 0.958 | 0.955 | 0.941 | 0.962 | 0.935 | 0.953 | 0.950 |
| 27 | 0.960 | 0.957 | 0.950 | 0.966 | 0.942 | 0.957 | 0.958 |
| 28 | 0.964 | 0.961 | 0.955 | 0.965 | 0.947 | 0.962 | 0.960 |

The modules 6 and 68 in the BRNO samples 2, 3, and 9 experienced decreases in the SSMC with respect to the modules of the epithelial domain (epi). The results of the calculation of the SSMC between the kernel module and the modules 6 and 68 showed a slight decrease in connectivity in the sample 3 but showed no decrease in connectivity in the samples 2 and 9. This implies that another module influenced by the kernel module is mediated between the modules 6 and 68 and the epithelial domain (epi). When examining the relationship between several nearby modules and the kernel modules and between the several nearby modules and the modules 6 and 68, the module 18 was the most influential.

The SMC between the kernel module and the module 18 had an average value of 0.9811 $\pm$ 0.0050 when the samples 2, 3 and 9 were excluded. In the case of the samples 2, 3, and 9, the SSMCs were reduced to 0.9488, 0.9328, and 0.9460, respectively. When calculating the SSMC for CG, which is a set of genes constituting the kernel module in each of the samples 2, 3, and 9, the SSMCs were 0.8796, 0.8374, and 0.8762, respectively, and the average of the other SMCs were 0.9661 $\pm$ 0.0087. When the SSMC for CGX which is a set of genes constituting the kernel module was calculated, and the SSMCs for the samples 2, 3, and 9 were respectively 0.9397, 0.9205, and 0.9362 , and the average of the SMCs except for those SMCs was 0.9789 $\pm$ 0.0051. These results imply that that the CG transformation in the kernel module destroyed the connectivity between the kernel module and the module 18 and prevented the modules 6 and 68 from being connected to the epithelial domain (epi). It means that it can be transformed into other forms, whereby the functional properties of epithelial cells were lost, meaning that epithelial cells can be transformed into other forms.

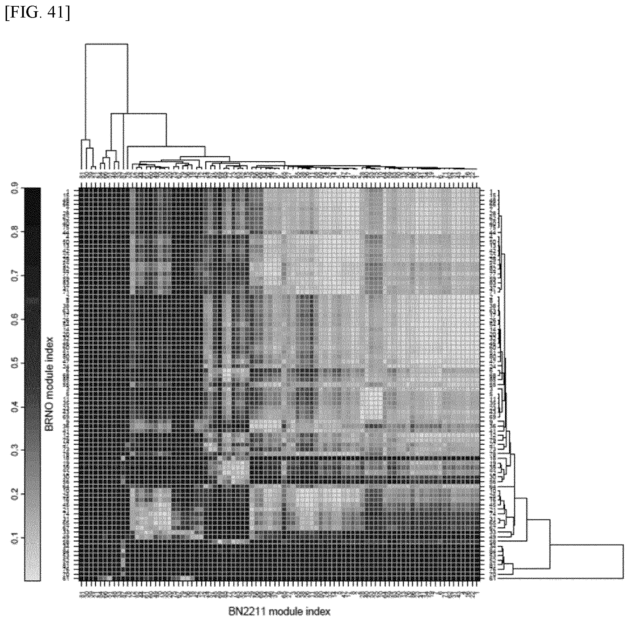

By additionally isolating BRNO samples (hereinafter, referred to as BN2211) of the branch R.2.2.1.1 in which the samples 2, 3, and 9 are included, the researcher obtained a total of 87 modules. To determine the biological functions of the modules obtained from BN2211, the modules of BRNO were mapped to the modules of BN2211, and the relative entropy of each of the modules of BRNO with respect to a corresponding one of the BN2211 modules was calculated. The BN2211 modules refer to modules belonging to an intermodular network constructed using the samples belonging to the BN2211. FIG. 41 illustrates an example of a level plot clustering based on the relative entropy of each of the BN2211 modules with respect to a corresponding one of the BRNO modules which are mapped to the BN2211 modules.

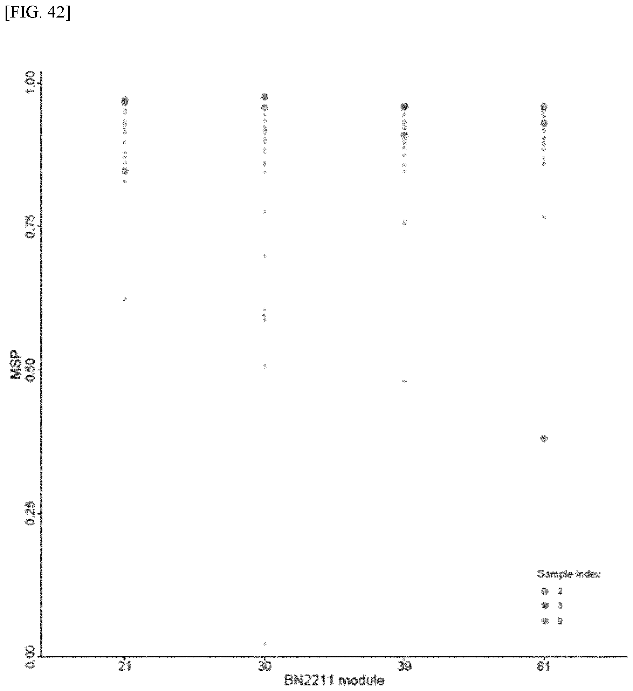

The modules 34, 52, 53, 70, 76, 81, and 84 belonging to the epithelial domain (epi) of BRNO had a relatively low entropy level only in the module 87 of BN2211. That is, it was confirmed that most of the modules of the epithelial domain (epi) were collapsed in BN2211. On the other hand, the modules 21, 30, 39 and 81 of BN2211 are not identified from the modules of BRNO. FIG. 42 is a distribution of MSPs of samples of BRNO for the modules 21, 30, 39 and 81 of BN2211. As shown in FIG. 42, in the BN2211 modules 21 and 81, the MSP of the sample 9 has a small value, but in other cases, the MSP has a relatively great large value. Accordingly, the modules 21, 30, 39 and 81 easily maintain integrity in the BRNO samples 2, 3, and9. On the other hand, when the SSMCs of each of the BRNO modules and each of the BN2211 modules in each BRNO sample were calculated, the connectivity was reduced in the samples 2, 3, and 9. Specifically, in the samples 2 and 3, the isolation of the modules was conspicuous. However, in the sample 9, the connectivity (SSMC: 0.03 to 0.88) with the kernel module and the CCDR domain was insufficient but the isolation was alleviated. In these samples, all the modules of BN 2211 except for the modules 21, 30, 39 and 81 had sufficient connectivity with the kernel module, and the connectivity with most other modules except for the epithelial domain (epi) was also high. To determine whether the biological functions of the modules 21, 39, 30, and 81 of the BN2211 are transformed or not, the first eigenvector $|v_i^1\rangle$ was calculated from each of the density matrices ( $\rho_i^{BRNO}$ , i = 21, 30, 39, 81) of the modules in all BRNO samples. The results of measuring the angle between the expression vector $|g_i^{BRNO}\rangle$ and $|v_i^1\rangle$ of the BRNO sample j (j = 1, ..., 28) in the genetic space of each BN2211 module are shown in Table 19.

[Table 19]

|  | Sample | 21 | 30 | 39 | 81 |
|---|---|---|---|---|---|
| R.1 | 14 | 162.2 | 142.3 | 164.3 | 164.2 |
|  | 23 | 173.6 | 168.8 | 170.1 | 171.8 |
| R.2.2 | 16 | 170.6 | 157.2 | 167.3 | 171.8 |
|  | 20 | 165.6 | 161.3 | 159.4 | 165.8 |
|  | 12 | 159.4 | 144.1 | 153.5 | 161.7 |
|  | 26 | 163.7 | 140.3 | 154.3 | 166.6 |
| R.2.2.1.1 | 3 | 9.9 | 8.6 | 9.8 | 14.5 |
|  | 9 | 24.0 | 13.5 | 18.3 | 52.8 |
|  | 28 | 171.4 | 163.8 | 167.6 | 171.2 |
|  | 13 | 174.1 | 170.3 | 170.8 | 172.6 |
|  | 22 | 172.0 | 168.5 | 167.8 | 169.5 |
|  | 2 | 7.7 | 8.5 | 10.5 | 9.5 |
|  | 27 | 173.9 | 169.8 | 171.8 | 174.2 |
| R.2.2.1.2 | 15 | 162.7 | 163.7 | 163.5 | 165.5 |
|  | 11 | 173.3 | 168.9 | 170.8 | 169.2 |
|  | 18 | 167.9 | 168.3 | 168.1 | 162.7 |
|  | 8 | 169.6 | 169.0 | 167.6 | 171.3 |
|  | 24 | 171.8 | 165.2 | 166.7 | 168.1 |
|  | 7 | 171.9 | 161.7 | 165.7 | 165.0 |
|  | 21 | 171.8 | 168.2 | 171.1 | 168.1 |
|  | 10 | 170.6 | 166.2 | 165.6 | 169.8 |
|  | 19 | 164.5 | 163.9 | 164.3 | 170.8 |
| R.2.2.2 | 1 | 159.1 | 151.4 | 155.3 | 164.4 |
|  | 17 | 170.1 | 165.0 | 168.2 | 167.6 |
|  | 25 | 168.0 | 163.0 | 162.4 | 166.7 |
|  | 4 | 146.0 | 102.5 | 137.4 | 155.8 |
|  | 5 | 164.5 | 143.5 | 154.0 | 170.1 |
|  | 6 | 170.8 | 166.0 | 169.3 | 164.2 |

In the BRNO samples 2, 3, and 9, the angle of the expression vector is significantly different from those of other samples, thereby indicating that the direction was significantly changed. In these samples of BRNO, the change in the biological property can be determined not by the magnitude (degree) of the phenotypes generated by the genomic modules but by the direction change.

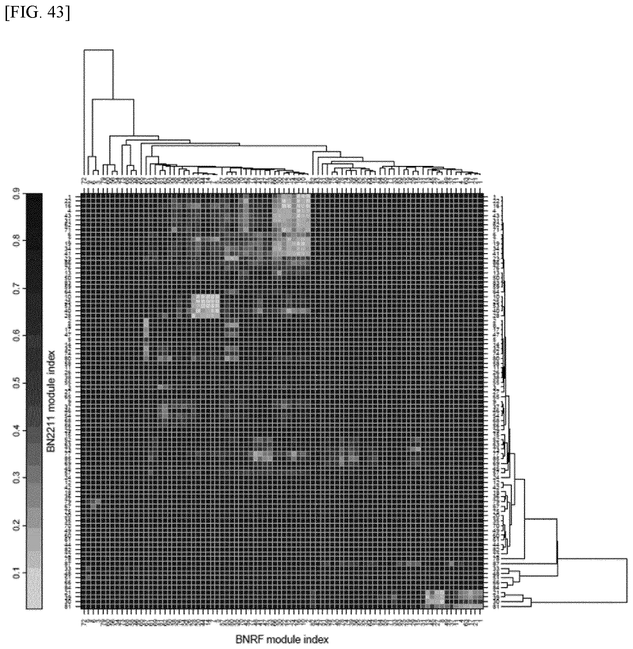

The modules 21, 30, 39 and 81 of BN2211 are free from direct or indirect control by the kernel module. BNRF is a genetic data set generated by removing the influence of the kernel module of the BRNO. BNRF is a result of filtering BRNO using the main eigenvector of the kernel module and the above-described filtering technique. Therefore, the modules that are not affected by the kernel module in BN2211 must be detected in BNRF. The modules of BN2211 were mapped to BNRF, and the relative entropy of each of the modules of BNRF was measured. The modules of BNRF mean modules belonging to an intermodular network constructed with the use of the genetic data set of BNRF. FIG. 43 illustrates an example of a level plot clustering based on the relative entropy of each of the BNRF modules with respect to a corresponding one of the BN2211 modules mapped to BNRF. The BNRF modules with a low entropy with respect to the BN2211 modules 21, 30, and 39 were the modules 8, 27, 45, and 13 (see Table 20 below).

[Table 20]

|  | Module | BNRF | | | |
|---|---|---|---|---|---|
|  |  | 8 | 27 | 45 | 13 |
| BN2211 | 21 | 0.135 | 0.120 | 0.283 | 0.185 |
|  | 30 | 0.138 | 0.150 | 0.155 | 0.214 |
|  | 39 | 0.182 | 0.219 | 0.262 | 0.129 |

With respect to the BN2211 module 81, the BNRF module 17 exhibited the lowest relative entropy of 0.139. Regarding the distribution of the angles of the sample vectors of BRNO with respect to the first eigenvector of the density matrix in the genetic space in which the BNRF modules are mapped into the BRNO, the distribution of the angles in each of the BRNO samples 2, 3, and 9 was significantly different from that in the other samples. This result implies that the modules of the BNRF and BN2211 are directly related to cell transformation.

Genes constituting the modules 21, 30, 39, and 81 of BN2211 were investigated to elucidate the transformation of biological properties of cells (see Table 21 below).

[Table 21]

| Module | Genes |
|---|---|
| 21 | ANXA8L2, BSPRY, TTC39A** (C1orf34), CDH3, CHMP4C, CHST9, CLDN4, CYP2J2, HSPC105, KIAA1324, KRT7, KRTCAP3*, LOC124220, OVOL2*, PPAP2C, PROM1*, PRSS16*, ROPN1, ROPN1B, SCNN1A, SHROOM3**, SLC27A6, SLC35F3, TACSTD2, TFAP2B, TFAP2C, TMEM125, TNS4*, TRIM29* |
| 30 | AGR2, C1orf172**, CGN, CLDN3, CLDN7, CRB3, DSP**, ELF3, ELMO3, EPN3*, FXYD3, GPR143, GRB7, KIAA0895, KIAA1909*, KRT19, LRRN1, MARVELD3, MB, MIA, PCBP3, PRSS8*, RAB25***, SH3KBP1, SPDEF, SUSD4, SYNGR1, TMC4, TMEM61 |
| 39 | AGR3, C10orf35, SYNE4 (C19orf46), SRARP* (C1orf64), CA8, CHMP4C, CHST9, CLDN7*, CREB3L4, DLK2**, FAM103A1, FLJ33534, GNA12*, GPC6**, GRHL2, GSTO2**, IGFALS, KIAA1324***, KLK6, KLK8, KRTCAP3*, LIX1L, LOC124220, ODZ3, PGBD2, PLEKHB1, PPAP2C, PRSS16*, RBM11, SCNN1A, SERPINB5, SLC4A3, SOX9*, SPINT1*, STYK1, TALDO1**, TFAP2C, TMEM125***, XBP1* |
| 81 | ARL2, C1orf210, CALML3, CHRNB2, DKK3, DSG3, FAM46B, KCNS1, KCTD14, KLHL13, KLK10*, KRT81, LCN2, LINCR, LIPH, MUC15, NEBL, NTF5*, PLCH2, PPP1R13L, PROM2*, PRRG2, RASGEF1C, RSPH1, SAMD12, SLPI, SOSTDC1*, TMPRSS13, TMPRSS2, TPPP2*, WFDC2*, ZNF594, ZNF627, ZNF750 |

*: $p < .05$, **: $p < .01$, ***: $p < .005$

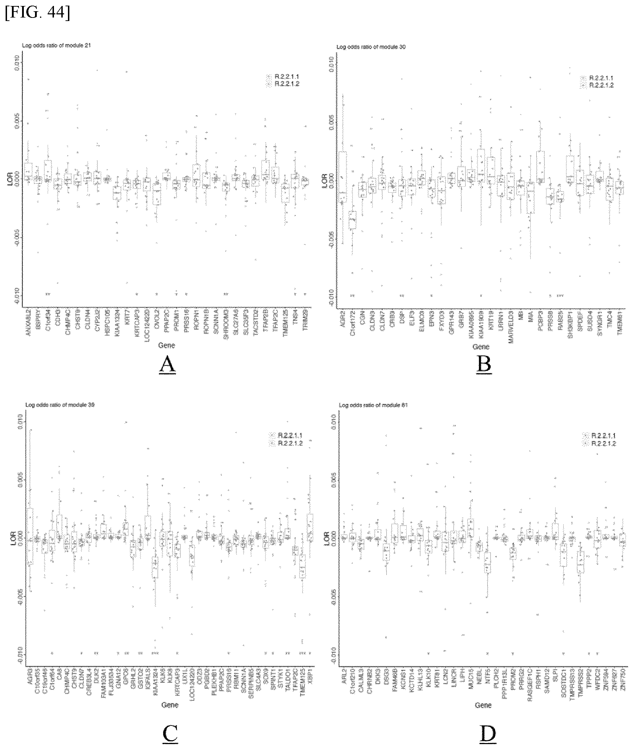

To analyze the degree of contribution of BN2211 to the transformation of modules 21, 30, 39 and 81 in R.2.2.1.1 including BRNO samples 2, 3 and 9, the LORs of genes included in each of the modules in R2.2.1.2, which are considered as the most normal genes, were calculated and compared. FIG. 44 illustrates the calculated LORs for the genes of the modules 21, 30, 39, and 81 of BN2211. FIG. 44 shows the significance of BRNO sample groups R.2.2.1.1 and R.2.2.1.2 for the modules of BN2211. FIG. 44A shows the LORs of the module 21 of BN2211, FIG. 44B shows the LORs of the module 30 of BN2211, FIG. 44C shows the LORs of the module 39 of BN2211, and FIG. 44D shows the LORs of the module 81 of BN2211. A number of genes showing statistically significant differences were included, and most of the genes were closely related to epithelial mesenchymal transition (EMT). Thus, the modules 21, 30, 39, and 81 of the BN2211 will be called "EMT modules" herein.

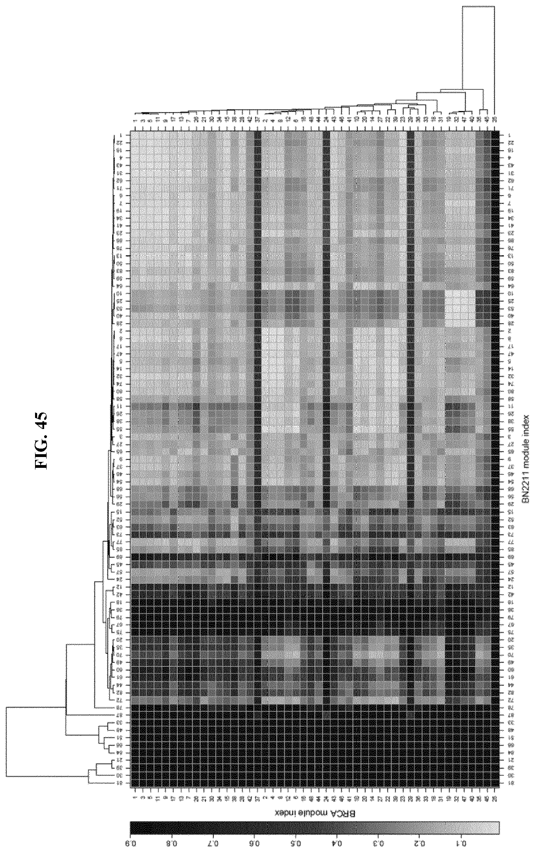

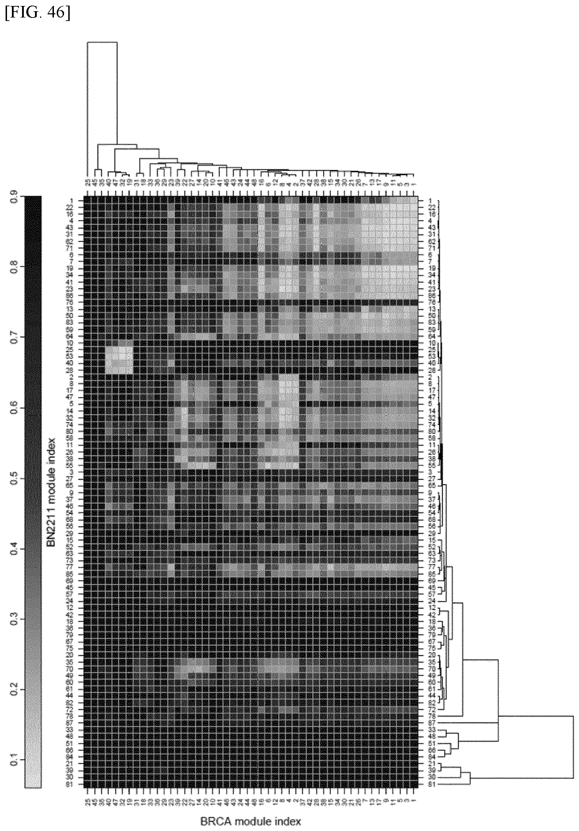

To keep track of the role of the EMT module on breast cancer, a level plot of the relative entropy of each of the modules of BN2211 versus the relative entropy of each of the modules of BRCA was created. FIG. 45 illustrates an example of a level plot clustering based on the relative entropy of each of the BN2211 modules with respect to each of the BRCA modules mapped to the BN2211 modules. Referring to FIG. 45, there was no BRCA module that can correspond to the modules 21, 30, 39 and 81 of BN2211. The relative entropy of the modules of BRCA with respect to the mapped modules of BN2211 was calculated. FIG. 46 illustrates an example of a level plot clustering based on the relative entropy of each of the modules of BRCA with respect to the modules of BN2211 mapped into BRCA. Even in this case, no BRCA modules corresponding to the modules 21, 30, 39 and 81 of BN2211 were found. This means that these modules are activated only in rare samples that are difficult to detect in BRCA or are activated only in some cells in a single sample.

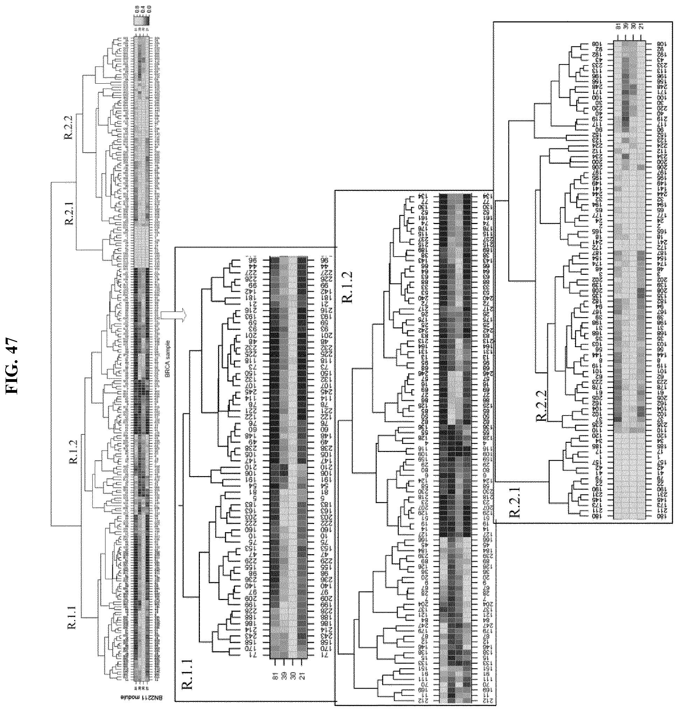

To determine this, the MSPs of the BRCA samples with respect to those modules of BN2211 were calculated, and a sample dendrogram was created. FIG. 47 illustrates the clustering of the MSPs of the BRCA samples with respect to some modules of BN2211. FIG. 47 illustrates the clustering of the MSPs of the BRCA samples with respect to the modules 21, 30, 39, and 81 of BN2211. As shown in FIG. 47, the BRCA samples belonging to R.2.2 have high MSPs. Therefore, it is reasonable to interpret that it is difficult to detect the genes not because the frequency of cells in which these modules are activated in a single sample is low but because the samples in which the genes are activated is limited. This conclusion is consistent with the notion that BRCA is a collection of various subtypes of breast cancer.

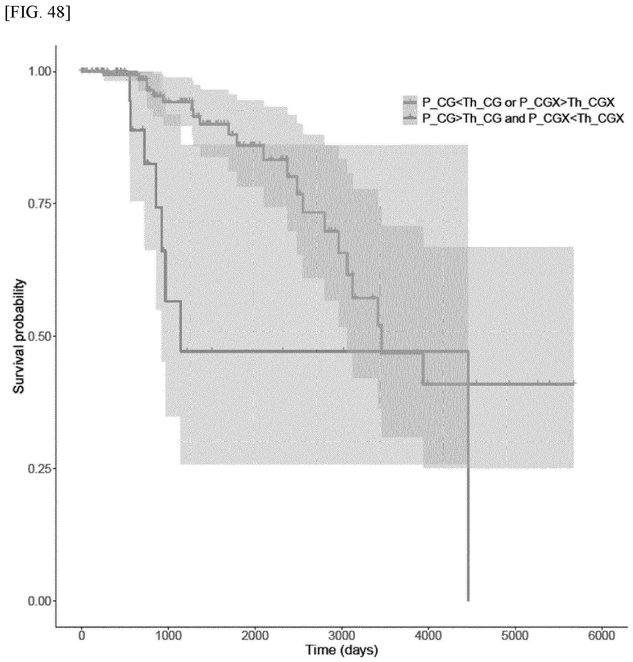

To detect the occurrence of EMT in BRCA, the samples of BRCA were grouped by the respective thresholds ($Th_{CG} = 0.9976$ and $Th_{CGX} = 0.9724$) of $MSP_{CG}$ and $MSP_{CGX}$. That is, the samples were divided into four groups BAHH, BAHL, BALH, and BALL. The BAHH group was higher in both $MSP_{CG}$ and $MSP_{CGX}$ than the respective threshold values, the BAHL group was higher in $MSP_{CG}$ and lower in $MSP_{CGX}$ than the respective threshold values, the BALH group was lower in $MSP_{CG}$ but higher in $MSP_{CGX}$ than the respective threshold values, and the BALL group was lower in both $MSP_{CG}$ and $MSP_{CGX}$ than the respective threshold values. The respective threshold values of $MSP_{CG}$ and $MSP_{CGX}$ were selected to maximize the hazard ratio of the Cox proportional hazards model survival analysis. FIG. 48 is a survival curve for the groups of BRCA samples classified by the of $MSP_{CG}$ and $MSP_{CGX}$. FIG. 48 illustrates an example of performing survival analysis on the sample group BAHL ($MSP_{CG} > Th_{CG}$ and $MSP_{CGX} < Th_{CGX}$) and the remaining samples.

In the sample group BAHL, the probability of survival decreased most sharply, and the hazard ratio in the samples of BAHL was significantly large (3.628 (p = 0002)). Most of the deaths died within 1,000 days. Compared with BALH, the probability of death within 3,000 days increased by 18.38 times (p = 0.0004).

The relative entropy of CG to CGX was 0.0251 bits in BRNO but was increased to 0.0782 bits in BAHL. Those values are exceptionally low compared to the relative entropy (4.6542 bits) of CG to CGX in all BRCA samples as shown in Table 9. Conversely, the relative entropy of CGX to CG in BRNO and the relative entropy of CGX to CG in BAHL significantly increased to 0.0616 bits and 1.5938 bits, respectively. A kernel module that was separated from BAHL included genes AHSG, APOA2, APOC3, APOH, ASGR2, C14orf1 15, CSAG1, CSAG3A, DCT, DPPA4, F2, GATA1, GDF3, HBE1, HBG1 , HEMGN, IGFBP1, LIN28 , MAGEA1, MAGEA10, MAGEA12, MAGEA3, MAGEA4, PASD1, PRODH2, RHAG, RHOXF2B, SERPINA7, SILV, TM4SF5, and TYR, including CG. The genes except for CG have a slightly different composition from that of CGX of BRNO, and the relative entropy of CG to those genes was exceptionally low as 0.0167 bits. Therefore, unlike general tumor tissues in which CGX and CG are separated from the kernel module and CG is collapsed, in BAHL, CG was well preserved and was not separated from CGX. Instead, CGX is transformed to enhance connectivity with CG. Thus, it could be found that the transformation of the kernel module had occurred.

To investigate the relationship between kernel module transformation and BRCA phenotype, the relationship with N stage, which means the degree of lymph node metastasis among breast cancer stages, was examined for four sample groups (Table 22 below).

[Table 22]

|  | N0 | N1 | N2 | N3 | sum |
|---|---|---|---|---|---|
| BAHH | 28 | 9 | 6 | 3 | 46 |
| BAHL | 7* | 12 | 5 | 4 | 28 |
| BALH | 36 | 27 | 15 | 2 | 80 |
| BALL | 43 | 30 | 12 | 4 | 89 |

It is found that N0 without lymph node metastasis is significantly reduced only in BAHL. Therefore, this result means that when $MSP_{CG}$ increases and $MSP_{CGX}$ decreases, tumor cell motility increase, resulting in EMT as in the case of BRNO.

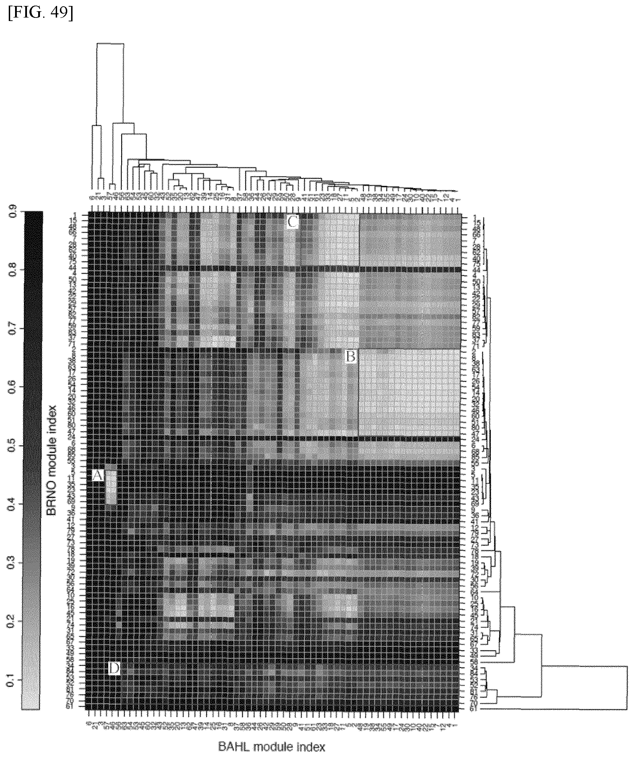

The researcher isolated genomic modules from the BAHL sample to investigate the genomic system that induces EMT of tumor cells. A total of 63 genomic modules were isolated, and the information on the genomic system thereof showed 0.7312 bits, which was lower than 1.0150 bits of BRCA. From the information, it was found that the degree of coexistence of breast cancer subtypes decreased. However, since the value was larger than 0.3712 bits of BRNO, it was found that subtypes were mixed or there was a collapse of the genomic system. The mapped entropy of the modules of BRNO to BAHL was $0.9760 \pm 0.5821$ bits, and the mapped entropy of the modules of BAHL to BRNO was $0.4234 \pm 0.3038$ bits. These results imply that the modules of BRNO collapse in BAHL, and it is inferred that the modules of BAHL are activated more or less in BRNO. To investigate the heterogeneity of BAHL with respect to BRNO, the relative entropy of BAHL modules with respect to BRNO modules was calculated. FIG. 49 illustrates an example of a level plot clustering based on the relative entropy of BAHL modules to BRNO modules mapped into BAHL. The CCDR domain of BRNO corresponds to the modules 46 and 57 (region A) in BAHL, and the kernel module domain (region B) and stroma domain (region C) are also clearly distinguished. On the other hand, the epithelial domain (epi) has a high relative entropy and is widely distributed in BAHL, without being clearly distinguished (region D). It means that epithelial cells have transformed into a tumor state in BAHL.

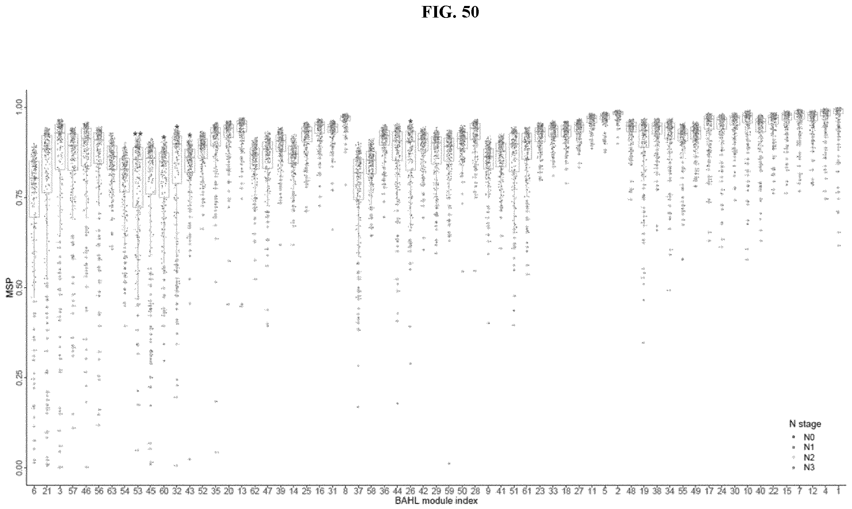

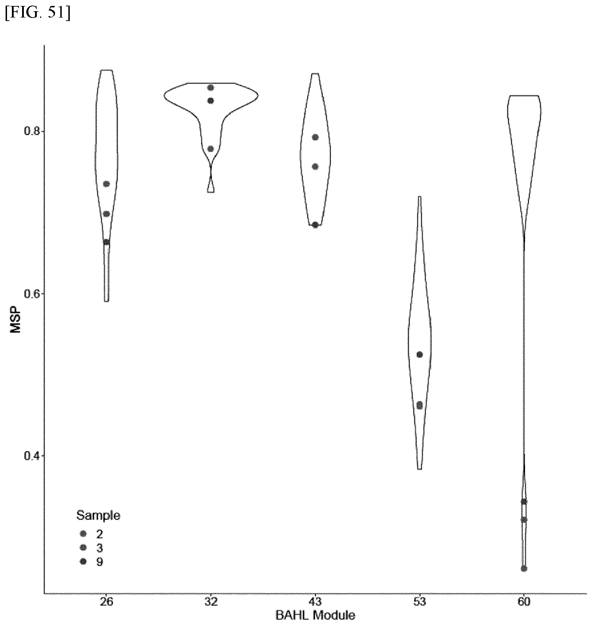

The researcher calculated the MSPs of BRCA samples with respect to the BAHL modules to elucidate the relationship between the module isolated from BAHL and the lymph node infiltration of tumor tissue cells. FIG. 50 illustrates an example a distribution of MSPs of the BRCA samples with respect to the BAHL modules. The BRCA samples are divided into an N0 group and an N1 or higher group of N stages, and the significance of the difference in MSP distribution in each module was verified by the Kruskal Wallis test. FIG. 50 shows that there is a significant difference between the MSP distribution of the N0 group and the MSP distribution of the N1 or higher group in BAHL modules 26, 32, 43, 53 and 60. The distribution of MSPs of BRNO samples with respect to the modules is shown in FIG. 51. FIG. 51 illustrates an example of the MSPs of BRNO samples with respect to BAHL modules 26, 32, 43, 53 and 60. As shown in FIG. 51, the MSPs of the BRNO samples in the BAHL module 60 are significantly lowered.

In BAHL's kernel module, the CGX part except for CG is transformed differently from BRNO's CGX. Therefore, in the BAHL sample, the module 60 may be controlled by the transformed CGX, have a high MSP, and experience node invasion acceleration. On the other hand, in the BRNO sample with the CGX in a steady state, a problem occurs when information exchange between the module 60 and the CGX is blocked. In fact, in BRNO, as to the SSMC of CGX and the module 60 of BAHL mapped to BRNO, the SSMCs in the samples except for the samples 2, 3, and 9 were 0.9374 ± 0.0117 on average, but the SSMCs in the three samples 2, 3, and 9 were decreased to 0.8124 ± 0.0195. In conclusion, in the samples 2, 3, and 9 of BRNO, the BAHL module 60 induced cell transformation by being free from the regulation due to the interruption of information exchange with normal CGX, whereas in BRCA, transformed CGX regulated the BAHL module 60, resulting in cell transformation.

Since the N0 group and the N1 or higher group of BRCA have different genomic systems, the two sample groups also have differences in the distribution of LORs of the genes constituting each module (Table 23 below). PIM2 of the module 26 continuously activates STAT3, thereby inducing EMT in breast cancer cells. NT5E (CD73) is also closely related to the activity of EMT.

[Table 23]

| Module | Genes |
|---|---|
| 26 | ANGPT1, C6orf105, CD1D, CD3D, CR1L***, CYP19A1, EREG, FAM112B, FOLR3, GFI1, GRAP2, HAS2, IGF2BP2, IRF4, KIAA1754L, LOC129293, LOC91461, MAL*, MYH6, NT5E**, PHF19, PIM2***, PLA2G4A, PLAC8, PRKCA, PRKCB1, PRSS21, PTX3, RGS20, SAA4, ST3GAL6, STARD4, TCF7*, TLR9, TNFRSF8, TNNI2, TOX, UNC93A |
| 32 | ADD2, CALB1***, CDCA7, CDK6, CDT1, CHEK1, CNTNAP3, COL9A3, CXCL1, F5, FOXA2*, FOXG1*, GAL, GLT1D1, hCG_25371*, KLK1, LCT, LYAR, NASP, NT5DC2, ODC1, ORC1L*, PHACTR1, PHC1, POLD1, PRKCQ, PSAT1, RGS20*, RNF182, RPRM, SACS*, SEC14L4, SKP2*, SLC10A4, SLC6A15*, TEX10, TMEFF2*, UQCRH* |
| 43 | ABI3*, ALDH3A1, APOB48R**, APOL3, ATP6V0E1, C13orf31, CAPG, CD36*, DDX58, DTX3L, EHD4, HLA-B, HLA-C, HLA-DRB5, HLA-E, HLA-F, IFI44L, IFIT3*, IRF7, IRX3, LGALS9, LOC654346**, NOD2, NUPR1*, OAS2, OSBPL5, PDGFRL, PRAM1***, SEL1L, SIGLEC1, TN-FSF12, TREM2, TRIM6-TRIM34, VWA1, ZNF613 |
| 53 | ANKS6, BAIAP2L2, BCL11B, C1orf67, C20orf42, C2orf55, C6orf117*, C9orf40*, CCDC18, CNTNAP3, DCBLD2**, DZIP1, FOXF2*, FSCN1, HDAC4, HMGCS1, IGF2BP3*, ITM2C, KLK11, LBR, MAP7D3, MET*, PCOLCE2, PDE1C, PHACTR1*, POLR3G, PRKCA*, PRPF38A, RNF145, ROBO3, SERPINE2***, SLC6A14, SOX21*, SPRY2, ST3GAL6, STK32A, TJP3, TNNI2***, TOX, TRIT1, YBX1, ZNF179 |
| 60 | ADD2, ALDH3A1*, B3GALT4, BCL3*, C20orf134, CAMKV***, CMTM8, CRIP1, DDC*, ELMO3, ELMOD2*, ENTPD7**, ERBB2, FAM102A, FOXA2**, FXYD3, GALNT6, GLT1D1, HCP5, IFIT5, IRX5, KLF8, KLK1, LIPH, LOC124220, MID2, NINJ1, OAS1, ONECUT1*, P2RY2*, PARP9, PERLD1, PHC1, PHYHIPL**, PPP2R5A, PRKCD***, PRSS22, RARA, RARRES3, RASL10B*, RNF182, SERF2, SERTAD3, SIX6**, SKP2*, SLC6A15, SP110, SQRDL*, TMC4, TMEFF2*, TMEM87B, VAMP8* |

*: $p < .05$, **: $p < .01$, ***: $p < .005$

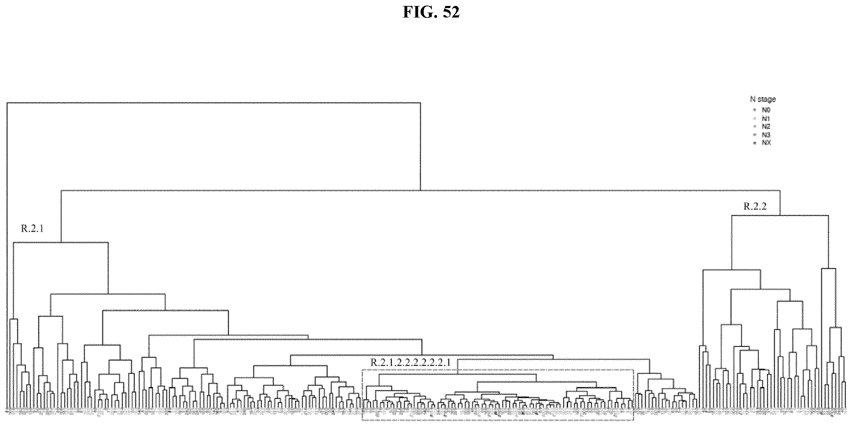

The relationship between the N-stage and the MSP was investigated by constructing a dendrogram with the MSPs of the BRCA samples with respect to the modules of the BAHL. FIG. 52 shows the clustering based on the MSPs of BRCA samples with respect to BAHL modules 26, 32, 43, 53 and 60. In FIG. 52, the BRCA samples whose N stage is N0 are concentrated in R.2.2. On the other hand, the ratio of the samples of N0 stage in R.2.1.2.2.2.2.2.2.2.1 (red box) is low (35.4%, p = 00427), and the proportion of N2 stage (26.6%, p = 0.0122) is large, thereby indicating that fluctuations in these modules were involved in lymph node infiltration of tumor tissue cells. In addition, 22 BAHL samples out of 28 BAHL samples were included in the group, thereby suggesting that transformation in the kernel modules ($MSP_{CG}$ rise and $MSP_{CGX}$ decline) are related to transformations in these modules.

**4. DNA Damage in Breast Cancer**

Mutations in genes most strongly presumed to be the cause of oncogenesis are widespread in tumors. In the BRCA data of TCGA, various mutations occurred at different degrees depending on samples. The researcher investigated whether the transformation of the kernel module was related to the occurrence of mutation and examined the related genomic system.

The causes of mutations are remarkably diverse and depend on the internal and external environment of the tissues. Therefore, the occurrence of mutations inevitably varies from samples to sample. An average of 155 mutations occurred in one sample of BRCA, with a standard deviation of 181.5. Mutations are presumed to be caused by a combination of several factors. Therefore, it is not easy to find the primary factor. As described above, since the tumorigenicity performance begins with the separation of CG and CGX from the kernel module transformation, the relationship between the mutations and the MSPs of the BRCA sample with respect to BRNO CG and CGX will be elucidated.

First, looking at the distribution of the frequency of occurrence of mutations with $MSP_{CG}$ as a reference point, when $MSP_{CG}$ is greater than the median value, the frequency of mutations was $188.1 \pm 229.0$, whereas when and when $MSP_{CG}$ is smaller than the median value, the frequency of mutations was $121.6 \pm 107.2$ (Kruskal-Wallis test, p = 0.0006; anova test, p = 00037) and was significantly increased. On the other hand, when $MSP_{CGX}$ was greater than the median value, the frequency was $188.7 \pm 232.9$, whereas when $MSP_{CGX}$ was smaller than the median value, the frequency was significantly increased to $121.0 \pm 98.0$ (Kruskal-Wallis test p = 0.0005; anova test, p = 00031). Transformation of the kernel module is the starting point of tumor tissue, showing that MSP of CG and MSP of CGX are associated with mutations in each sample. In addition, for the cases where the SSMC showing the connectivity between CG and CGX in each sample were larger than and smaller than the median, the frequency of mutations was investigated. When the SSMC was larger than the median value, the frequency of occurrence of mutations was as low as $115.6 \pm 94.2$, whereas when the SSMC was smaller than the median value, the frequency of occurrence of mutations was as high as $194.1 \pm 232.8$. Despite the large standard deviation, this difference was statistically significant (Kruskal-Wallis test, p = $1.0 \times 10^{-5}$; anova test, p = 00006).

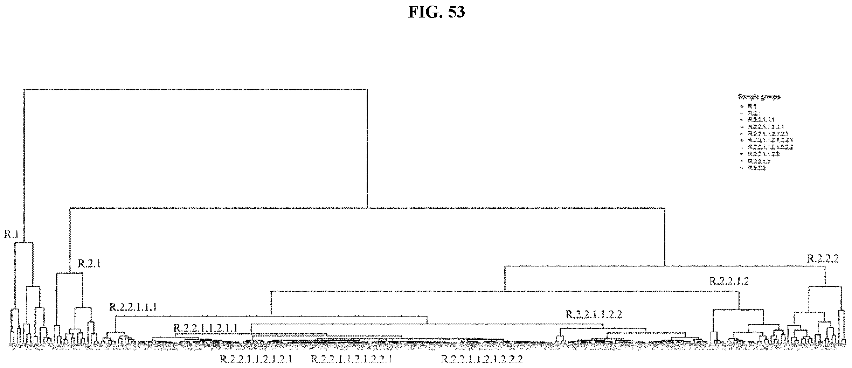

In order to more precisely analyze the relationship between the frequency of occurrence of mutations and the transformation of the kernel module, the researcher created the dendrogram of the samples with $MSP_{CG}$ and $MSP_{CGX}$ of BRCA, and classified the samples into 10 groups. FIG. 53 illustrates the clustering of BRCA samples according to $MSP_{CG}$ and $MSP_{CGX}$.

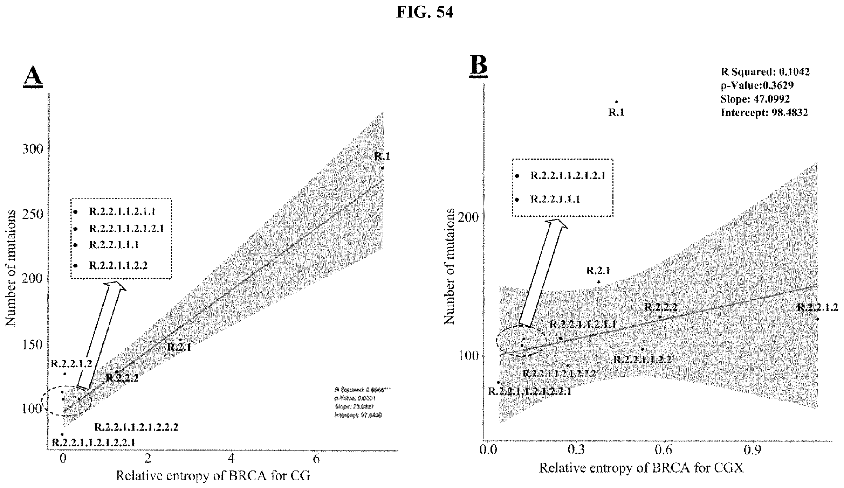

The relationship between the relative entropy indicating the degree of deviation of CG and CGX of BRCA from BRNO and the median value of the frequencies of occurrence of mutations of the respective samples groups was investigated. FIG. 54 illustrates the results of a linear regression analysis on the relationship between the relative entropy of CG and CGX of BRCA to CG and CGX of BRNO and the median value of the frequency of occurrence of mutation of each BRCA sample. FIG. 54A illustrates the results of linear regression analysis on the relationship between the median value of frequency of occurrence of mutation of the BRCA sample group of FIG. 53 and the relative entropy of CG of BRCA to CG of BRNO. FIG. 54B illustrates the results of linear regression analysis on the relationship between the median value of frequency of occurrence of mutation of the BRCA sample group of FIG. 53 and the relative entropy of CGX of BRCA to CGX of BRNO. In the linear regression of the mutation frequency of the BRCA sample with respect to the relative entropy of CG, $r^2$ was 0.87, but in the case of CGX, $r^2$ was as small as 0.10. In the case of dual linear regression of the mutation frequency against the relative entropy of CG and CGX, $r^2$ was increased to 0.93 (p = $8.0 \times 10^{-5}$). Therefore, of the transformations of the kernel module, the deviation of CG is significantly related to the occurrence of mutations, while the transformation of CGX also has some influence.

Considering the constitutive genes of the kernel module, it is not possible to conclude that the deviation of CG itself is the direct cause of the mutation. Therefore, it would be reasonable to search for those that can induce mutations from the modules affected by transformation of CG and CGX. In order to search for modules related to mutations, it is reasonable to compare the degree of deviation of CG from the normal state and the frequency of occurrence of mutations.

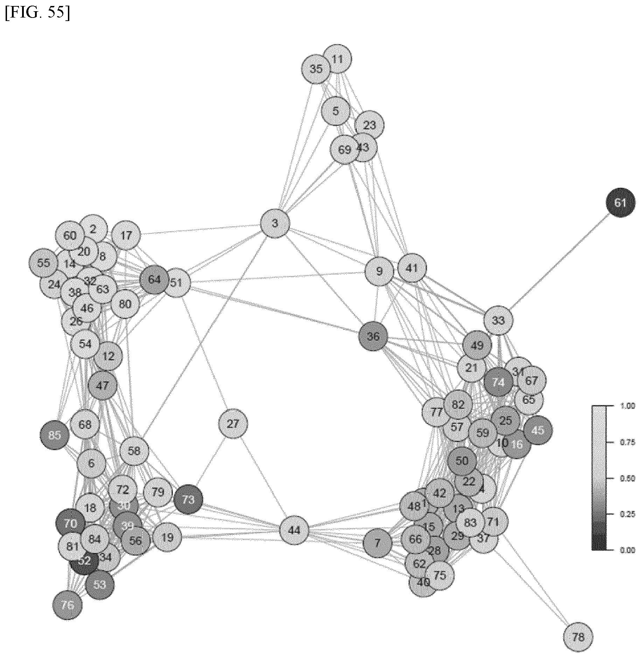

Therefore, the relative entropy of BRCA with respect to BRNO modules, i.e., $S_{ij} = S\left(\rho_i^{BRCA_j} \middle\| \rho_i^{BRNO}\right)$ was calculated for a BRCA sample group j, and the median value $m_j$ of the frequency of occurrence of mutations of the BRCA sample group j was calculated. Linear regression analysis was performed on $S_{ij}$ for $m_j$ in a module i, and $r^2_i$ was marked on an intermodular network of BRNO. FIG. 55 is an example of a BRNO intermodular network on which the results of linear regression analysis on the mutation frequency of the BRCA sample group in each module of the BRNO intermodular network are depicted. FIG. 55 illustrates an example of a BRNO intermodular network on which $r^2$ of each module is depicted after performing the linear regression analysis on the mutation frequency of the BRCA sample group of FIG. 53 in each module of BRNO. The frequency of mutations and the module 2, which is the kernel module, have a close correlation ($r^2 = 0.90$, p = $3 \times 10^{-5}$), similarly to the case of CG and CGX. The module 51 not belonging to the kernel module domain has the second largest $r^2$ of 0.89 (p = $4.3 \times 10^{-5}$).

To further investigate modules related to mutations, multiple linear regression analysis was performed on multiple modules including the module 51. When the module 51, the module 44, the module 58 ware used for analysis, the $r^2$ and p-value were 0.99 and $2.4 \times 10^{-6}$, respectively. To estimate the influence of CG and CGX on the three modules, linear regression analysis was performed on the relative entropy of the three modules to BRNO modules in the BRCA sample group of FIG. 52 against the relative entropy of CG and CGX, and $r^2$ was obtained (Table 24 below).

[Table 24]

|  | CG+CGX | CG | CGX |
|---|---|---|---|
| 51 | 0.92*** | 0.82*** | 0.14 |
| 58 | 0.63* | 0.49* | 0.17 |
| 44 | 0.54 | 0.27 | 0.30 |

*: $p < .05$, **: $p < .01$, ***: $p < .005$

The relative entropy, which is the degree of deviation from the normal state, of the module 51, which is most correlated with mutation, has dependency of 92% on the linear combination of the degrees of deviation of CG and CGX from the normal state. On the other hand, the relative entropies of the modules 58 and 44 have dependency of 63% and 54%, respectively, while $r^2$ for the frequency of mutations are 0.70 and 0.44, respectively, and the p-values are 0.003 and 0.036 which are both significant values, respectively.

To search for genes that are highly related to mutations, the probability of genes in the sample space was calculated. Ten sample spaces were separated based on the dendrogram generated with $MSP_{CG}$ and $MSP_{CGX}$ of BRCA, and the probability $P_{ij}^{BRCA}$ of a gene i in a sample space j was calculated. In addition, the probability ($P_i^{BRNO}$) in each of the sample spaces of BRNO was calculated, and the odds ratio of the gene i, i.e., $R_{ij} = P_{ij}^{BRCA}/P_i^{BRNO}$ was obtained in the sample space j of BRCA. A linear regression analysis on the median mutation frequency $m_j$ was performed on $R_{ij}$ in the sample space j of the BRCA. Significant (p value < 005) genes in the BRNO module 51 were 20 (MAGEA12, MAGEA1, CAD, MKRN3, PROC, ASFIB, RELL2, C21orf125, PFKFB4, SPAG4, C9orf100, C8G, MCM7, E2F1, ORC1L, NY-SAR-48, DLL3, SERPINF2, MAGEA4, DUSP9). The gene MAGEA12 exhibited the largest $r^2$ value of 0.93 and the gene DUSP9 exhibited the smallest $r^2$ value of 0.40. In the module 58, the mutation frequency showed a significant linear relationship with the odds ratio (LO) of 18 genes, and the gene NME5 exhibited the largest $r^2$ of 0.57.

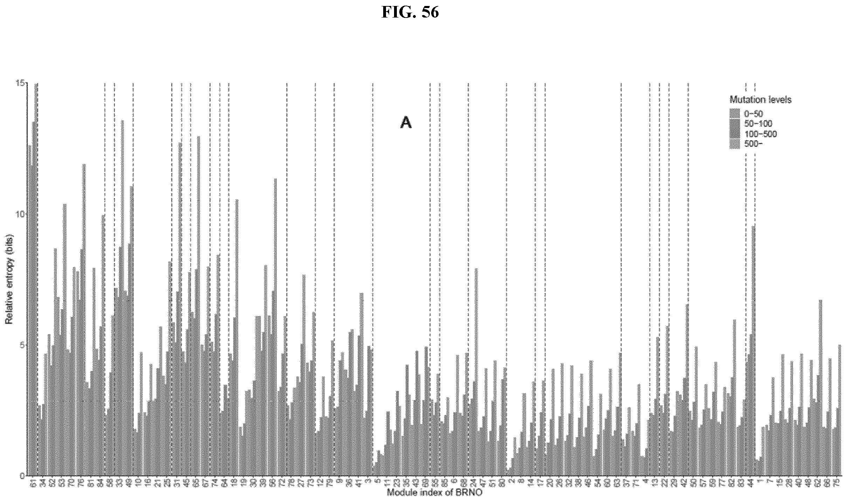

In investigating the genomic system, it is necessary to separately investigate the characteristics of the parts involved in DNA damage response (DDR) and the characteristics of the parts involved in the DNA repair. The genomic system related to DDR may have a wide range. BRCA samples were classified into four groups according to the frequency of occurrence of mutations, and the relative entropy of each sample group with respect to each BRNO module was calculated. FIG. 56 shows the relative entropy of each of the four BRCA sample groups with respect to each BRNO module. Section A is the CCDR domain of BRNO and contains genes involved in DNA damage repair.

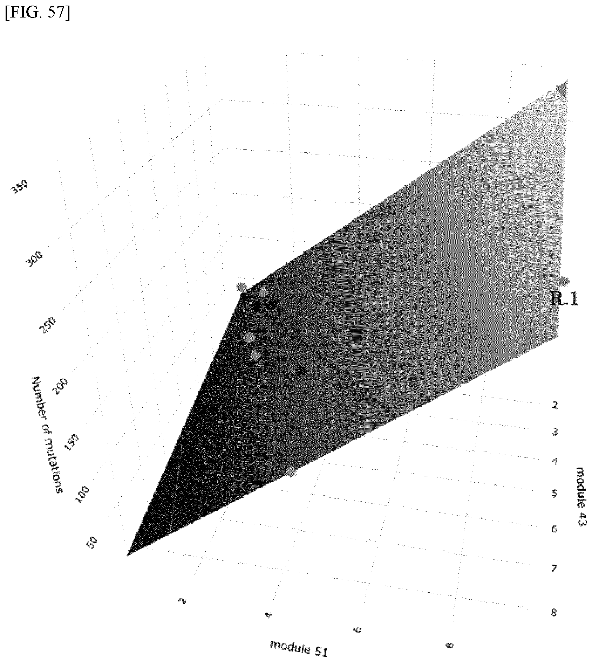

However, only in modules of section A and module 3, the relative entropy of the sample group with a mutation occurrence frequency of 500 or more was the second highest, but in other modules, the relative entropy of the sample group was the highest. The relative entropy of each of the modules 51, 58 and 44 as described earlier has a linear relationship with the frequency of mutations, and the $r^2$ of each of the modules was close to 1. Therefore, the modules are suitable for DDR, and the functions of the genes are far removed from the repair of DNA damage. Taken the results together, the frequency of mutations is determined by the integrity of the genomic system operating the DNA damage response and the integrity of the genomic system operating the DNA damage repair system. When the mutation frequency is 500 or more, the integrity of the DNA damage repair system is good, whereas the collapse of the DDR operating system is severe. The relative entropy was more greatly increased in all modules except for those in the CCDR domain. The frequency of occurrence of mutations is dependent on DDR and DNA damage repair system. In this study, a linear combination between the frequency of mutations and the module 51 and any one of the modules in the CCDR domain was attempted. FIG. 57 illustrates the result of the linear combination of the module 43 in the CCDR domain and the module 51 corresponding to the DDR system exhibited an $r^2$ of 0.93 and a p-value of $8.0 \times 10^{-5}$. FIG. 57 illustrates the result of linear regression analysis of the mutation frequency and the relative entropy of BRCA with respect to the BRNO modules 43 and 51. In the BRCA sample groups classified based on $MSP_{CG}$ and $MSP_{CGX}$, R.1, which has the highest mutation frequency, is highly dependent on the module 51, unlike other sample groups, as shown in FIG. 57. Therefore, when the mutation frequency is extremely high, it is due to the mutation of the DDR system rather than the DNA damage repair system.

As previously described, transformations in the kernel module induce transformations in the linked genomic system, resulting in DNA mutations. Therefore, it is of great significance to identify the mechanism by which the module 51, which is the most sensitive module among DDRs widely distributed in the genomic system, and the module 43 of the DNA damage repair system are associated with the kernel module. The researcher investigated the relationship between the relative entropy of each of CG and CGX and the relative entropy of each of the modules 43 and 51 in the BRCA sample groups (Table 25 below).

[Table 25]

| Module | $RE_{CG}{}^{a}$ | $RE_{CGX}{}^{b}$ | $RE_{CG\|CGX}{}^{c}$ | $RE_{CGX\|CG}{}^{d}$ | $RE_{CG}+RE_{CGX}$ |
|---|---|---|---|---|---|
| 43 | 0.33 | 0.34 | 0.75** | 0.79*** | 0.63 |
| 51 | 0.82*** | 0.14 | 0.74** | 0.52* | 0.92*** |

a $S(\rho_{CG}^{BRCA}\|\rho_{CG}^{BRNO})$

b $S(\rho_{CGX}^{BRCA}\|\rho_{CGX}^{BRNO})$

c $S(\rho_{CG}^{BRCA}\|\rho_{CGX}^{BRCA})$

d $S(\rho_{CGX}^{BRCA}\|\rho_{CG}^{BRCA})$

*: $p < 0.05$, **: $p < 0.01$, ***: $p < 0.001$

The module 43 depends on the relative entropy of CG and CGX in the sample space of BRCA, but the module 51 not only depends on the relative entropy between CG and CGX but also mainly on the relative entropy of BRCA with respect to CG of BRNO. That is, different kernel module transformations have different effects on the disruption of the DNA damage response (DDR) and the DNA damage repair system, resulting in double mutation.

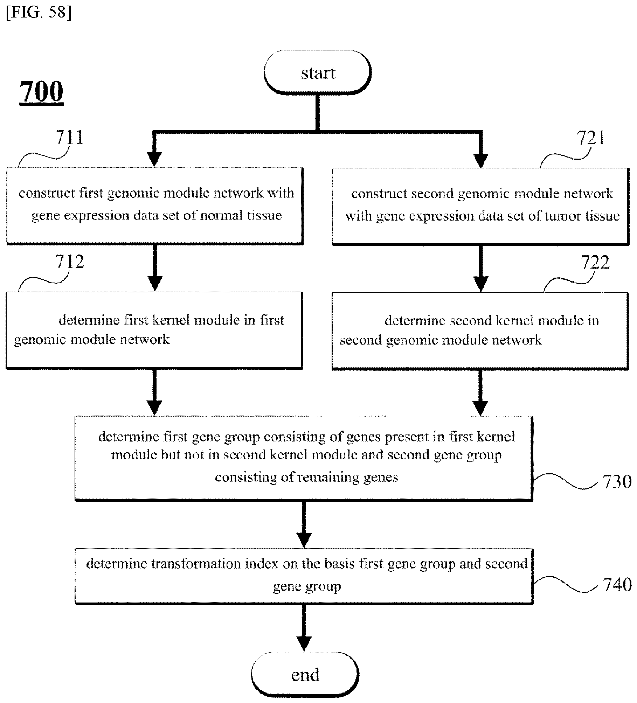

Herein after, the above-described genomic module network, and the analysis method using the kernel module of the genomic module network will be summarized. FIG. 58 illustrates an example of a process 700 of analyzing a kernel module of a genomic module network. FIG. 58 illustrates the process of determining a kernel module using gene expression data of normal tissue and gene expression data of tumor tissue, and of then generating a certain index based on differences between genes belonging to the kernel module.

An analysis device constructs a first genomic module network using gene expression data set for normal tissue (711). The analysis device determines a kernel module (a first kernel module) of the first genomic module network (712). In addition, the analysis device constructs a second genomic module network using gene expression data set for tumor tissue (721). The analysis device determines a kernel module (a second kernel module) of the second genomic module network **(722).**

As described above, the kernel module is a module having a lower entropy than the other modules in the intermodular network. The analysis device may determine a module having a lower entropy level than a predetermined reference value as a kernel module. In this case, an appropriate reference value may vary depending on the type of tissue, the type of tumor, the characteristics of the gene expression data set, and the like. In addition, the analysis device may calculate entropy for each module of the intermodular network and determine the kernel module using a low-entropy module group that is lower in entropy by a reference value or more than a high-entropy module group. In this case, since the kernel module needs to be remarkably low in entropy than other modules, a module group having a clearly distinguishable entropy value will be selected as the kernel module.

The analysis device determines a first gene group (CG) consisting of genes that are present in the first kernel module (normal tissue) but not present in the second kernel module (tumor tissue) and a second gene group (CGX) consisting of the remaining genes belonging to the kernel module **(730).**

Next, the analysis device may determine various transformation indices based on the first gene group CG and the second gene group CGX **(740).** The transformation indices may include the relative entropy of CG and CGX, MSP with respect to CG, MSP with respect to CGX, and the like. Furthermore, the analysis device may determine the connectivity (SSMC) between the kernel module (CG and/or CGX) and each of the other modules. Various indices and analysis examples are the same as described above.

The analysis device may store kernel module information, CG gene information, and CGX gene information which are generated during the analysis of the normal tissue and the tumor tissue in a separate reference DB.

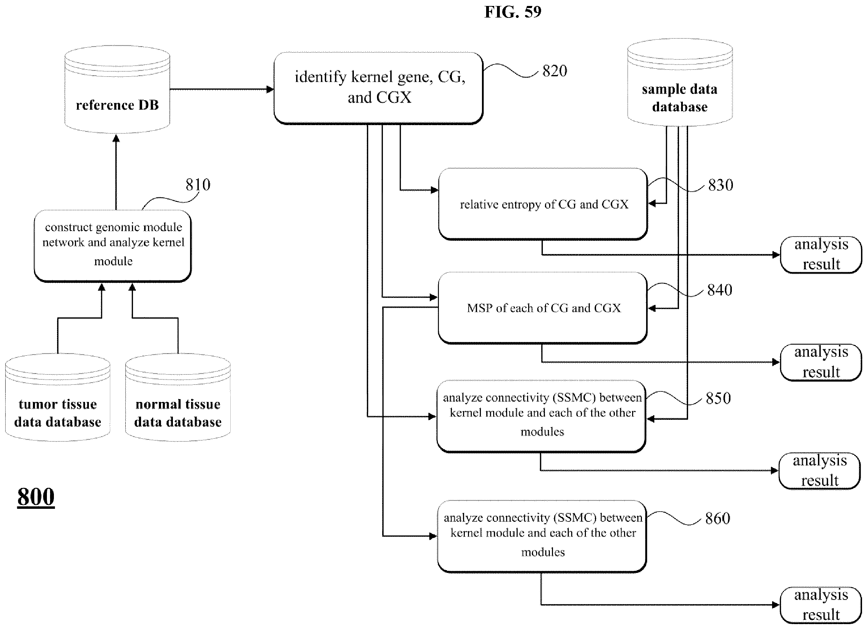

FIG. 59 illustrates an example of a process 800 of analyzing a sample using a kernel module of a genomic module network. First, a reference DB may be constructed similarly to the process described with reference to FIG. 58.

The analysis device acquires a first gene expression data set for tumor tissue from a tumor tissue data DB and acquires a second gene expression data set for intestinal tissue from a normal tissue data DB. The analysis device may construct genomic module networks using the first gene expression data set and the second gene expression data set, respectively, and analyzes the kernel modules to obtain information on gene, CG, CGX, etc. included in the kernel module as described above **(810).** The reference DB may store the result generated through the analysis of the kernel module. The reference DB may store gene expression data sets of normal and tumor tissues, genomic module network configuration information of normal and tumor tissues, kernel configuration information of normal and tumor tissues, CG gene information, and CGX gene information.

A sample data DB holds gene expression data of an analysis target. The analysis target may be tissues isolated from patients, healthy people, or people who can be categorized into healthy people but have a potential for developing a tumor.

The analysis device identifies kernel genes, CG genes, and CGX genes belonging to the kernel module, using the information stored in the reference DB **(820).**

The analysis device may perform various tests on the sample. Hereinafter, it is assumed that the analyzer identifies gene expression data of normal tissues, gene expression data of tumor tissues, and gene expression data of samples.

(1) The analysis device may construct a genomic module network based on the gene expression data of a sample. The analysis device may determine a kernel module in the genomic module network of the sample. The analysis device may determine whether the sample belongings to a normal category or a tumor category based on whether CG is present in the kernel module of the sample.

(2) The analysis device may combine the gene expression data of the sample with the gene expression data set of the normal tissue and construct a genomic module network based on the combined data. The analysis device may analyze the sample in a manner that compares the sample with the normal tissue. For example, the analysis device may compare the relative entropy of CG and the relatively entropy of CGX **(830),** may compare the MSPs of CG and CGX **(840),** may compare the connectivity of the kernel module with other modules between the normal tissue and the tumor tissue **(850),** may compare the normal tissue and the tumor tissue through the MSP-based clustering **(860),** or may compare the normal tissue and the tumor tissue on the basis of the LORs of the clustered groups **(860).**

(3) The analysis device may combine the gene expression data of the sample with the gene expression data set of the tumor tissue and construct a genomic module network based on the combined data. The analysis data may analyze the sample in a manner that compares the sample with the tumor tissue. For example, the analysis device may compare the relative entropy of CG and CGX **(830),** may compare the MSPs of CG and CGX **(840),** may compare the connectivity of the kernel module with other modules **(850),** may compare the MSP-based clustering between the tumor tissue and the sample **(860),** or compare the LORs of the clustered group between the tumor tissue and the sample (860).

(4) The analysis device may combine gene expression data of normal tissue, a gene expression data set of tumor tissue, and gene expression data of a sample and construct a genomic module network based on the combined data. The analysis device may analyze the sample in a manner that compares the sample with the tumor tissue. For example, the analysis device may compare the relative entropy of CG and CGX **(830),** may compare the MSPs of CG and CGX **(840),** may compare the connectivity of the kernel module with other modules **(850),** may compare the MSP-based clustering between the normal/tumor tissue and the sample, and may compare the LORs between the normal/tumor tissue and the sample **(860).**

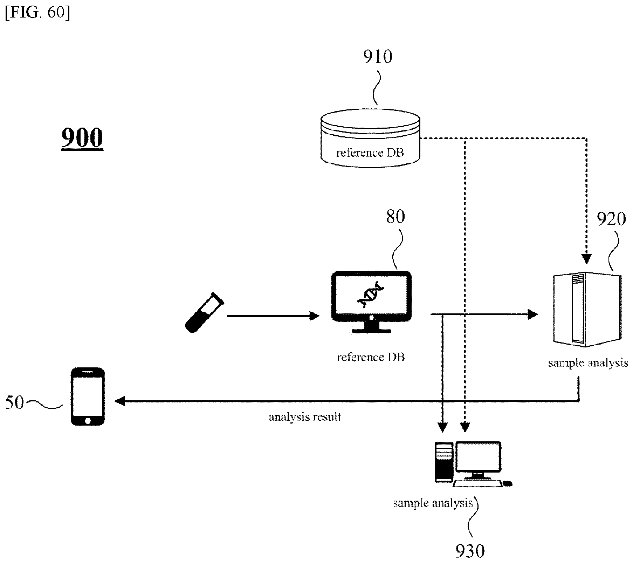

FIG. 60 is a diagram illustrating an example of a system 900 for analyzing a sample on the basis of a kernel module in a genomic module network. The system illustrated in FIG. 60 corresponds to a system that provides a service of analyzing a sample according to a kernel module-based sample analysis method. The sample analysis method may be at least one of various methods described with reference to FIG. 59.

The analysis system 900 includes a reference DB 910 and an analysis device 920 or 930. Alternatively, the analysis system 900 may include the reference DB 910, the analysis device 920, and the analysis device 930. The analysis device 920 corresponds to a networked analysis server, and the analysis device 930 corresponds to a private computer. The computer may be implemented in various forms such as a personal computer, a smart device, and the like.

A gene data generating device 80 corresponds to a device analyzing a sample of a test target to generate gene expression data. The gene expression data can be obtained with the use of a microarray. Alternatively, the gene expression data may be prepared through NGS analysis.

The analysis device 920 may receive gene expression data of a sample through a network. In addition, the analysis device 920 receive gene expression data sets of a normal tissue and a tumor tissue, genomic module network configuration information of the normal tissue and the tumor tissue, kernel configuration information of the normal tissue and the tumor tissue, CG genetic information, and CGX genetic information from the reference DB 910 through a network.

The analysis device 920 may construct a genomic module network using the reference data and the gene expression data of the sample and analyze the sample, using the kernel module as described above. The analysis device 920 may transmit the analysis result to the user terminal 50.

The analysis device 930 may acquire gene expression data of a sample through a network or from a storage medium (USB, SD card, hard disk, etc.). In addition, the analysis device 930 may receive, through a network, gene expression data sets of normal tissue and tumor tissue from a reference DB 910, genomic module network configuration information of normal tissue and tumor tissue, kernel configuration information of normal tissue and tumor tissue, CG genetic information, and CGX genetic information. Alternatively, unlike FIG. 60, the analysis device 930 may be connected to the reference DB by wire, or the analysis device 930 may contain the reference DB in a storage medium thereof. The analysis device 930 may construct a genomic module network using the reference data and the gene expression data of the sample and analyze the sample, using the kernel module as described above. The analysis device 930 may output the analysis result on the screen. Alternatively, the analysis device 930 may transmit the analysis result to the user terminal 50.

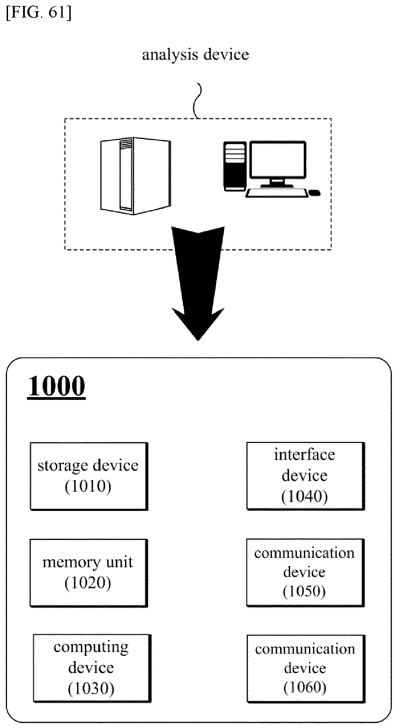

FIG. 61 is a diagram illustrating an example of an analysis device 1000 that analyzes a sample on the basis of a kernel module in a genomic module network. FIG. 61 may be a diagram illustrating the construction of the analysis device 920 or 930.

The analysis device 1000 may use a sample analysis program to generate health information about a sample. The analysis device 1000 may be physically implemented in various forms. For example, the analysis device 1000 may have the form of a personal computer, a smart device, a computer, a network server, a chipset dedicated to data processing, or the like.

The analysis device 1000 include a storage device 1010, a memory unit1020, a computing device 1030, an interface device 1040, a communication device 1050, and an output device 1060.

The storage device 1010 may store a genomic module network construction program and/or a sample analysis program for analyzing a sample using a kernel module in a genomic module network.

The storage device 1010 may store the input gene expression data.

The storage device 1010 may store reference data obtained by analyzing normal tissue and tumor tissue. The reference data may include gene expression data sets of normal tissue and tumor tissue, genomic module network configuration information of normal tissue and tumor tissue, kernel configuration information of normal tissue and tumor tissue, CG gene information, and CGX gene information.

The memory unit 1020 may store data necessary for the data processing process of the analysis apparatus 1000 and temporary data generated during the data processing.

The interface device 1040 is a device that receives predetermined commands and data from the outside. The interface device 1040 may receive gene expression data and/or reference data of a sample from a physically connected input device or an external storage device. The interface device 1040 may receive a program for data processing.

The communication device 1050 refers to a component for receiving and transmitting certain information through a wired or wireless network. The communication device 1050 may receive gene expression data and/or reference data of a sample from an external object. The communication device 1050 may receive a program and data for data processing. The communication device 1050 may receive reference data by communicating with a reference DB existing on a network. The communication device 1050 may transmit the analysis result of the sample to the outside.

The communication device 1050 and the interface device 1040 are devices that receive predetermined data or commands from the outside. The communication device 1050 or the interface device 1040 may be referred to as input devices.

The output device 1060 is a device that outputs certain information. The output device 1060 may display an interface necessary for a data processing process and output an analysis result, and the like.

The computing device 1030 constructs a genomic module network using gene expression data. The computing device 1030 may construct a genomic module network for each of normal tissue, tumor tissue, and a sample.

The computing device 1030 constructs a genomic module network using the gene expression data of a sample and analyzes the sample by comparing the kernel module information of normal tissue (or tumor tissue) with the kernel module information of the sample to analyze the sample.

The computing device 1030 constructs a genomic module network using the gene expression data of normal tissue (and/or tumor tissue) and the gene expression data of a sample performs sample analysis using the sample analysis method (index, clustering, etc.) described with reference to FIG. 58. The computing device 130 derives the normal state, tumor development state, tumor development probability, and customized treatment method for a diagnosed tumor as analysis results for the sample.

The computing device 1030 refers to a processor, application processor, or program-embedded chip for processing data and specific computation.

The sample analysis method based on the genomic module network, the intermodular network, the kernel module, and/or the genes of the kernel module may be implemented as a program (or application) including an algorithm executable on a computer. The program may be stored and provided in a non-transitory computer-readable medium.

Th non-transitory computer-readable medium does not refer to a medium that temporarily stores data such as a resistor, a cache, and a memory but refers to a medium that semi-permanently stores data and that is readable by a device. Specifically, the above-described various applications and programs may be provided while being stored in a non-transitory computer-readable medium such as a compact disc (CD), a digital versatile disc (DVD), a hard disk, a Blu-ray disc, a universal serial bus (USB), a memory card, a read-only memory (ROM), a programmable read only memory (PROM), an erasable PROM (EPROM), an electrically erasable PROM (EEPROM), a flash memory, etc.

Th transitory computer-readable medium refers to various RAMs such as a static RAM (RAM), a dynamic RAM (DRAM), a synchronous DRAM (SDRAM), a double data rate SDRAM (DDR SDRAM), an enhanced SDRAM (ESDRAM), a sync link DRAM (SLDRAM), and a direct Rambus RAM (DRRAM).

The embodiments described above and the accompanying drawings are presented only to clearly illustrate a portion of the technical spirit of the present disclosure, and it will be apparent that all modifications and specific embodiments that can be easily inferred by those skilled in the art without departing from the scope of the technical idea included in the specification and drawings may fall within the scope of the above-described technology.

**Claims**

1. A sample analysis method based on a kernel module of a genomic module network, the method comprising:

   by an analysis device, constructing a sample genomic module network based on entropy of a sample; and
   by the analysis device, analyzing the sample, using a reference kernel module in a reference genomic module network and a sample kernel module in the sample genomic module network,
   wherein the reference genomic module network is constructed, in advance, using at least one gene expression data set selected from among a gene expression data set of a normal tissue and a gene expression data set of a tumor tissue,
   the sample kernel module is a module that is lower in entropy by a reference value or greater than other modules, in the sample genomic module network, and
   the entropy indicates relations between each of a plurality of genes, based on probabilities of transcriptional states of the plurality of genes.

2. The sample analysis method according to claim 1, wherein the analysis device constructs the sample genomic module network including a plurality of genomic modules using the gene expression data of the sample,
   the distinguishing of the plurality of genomic modules comprises:

   classifying the plurality of genes into a plurality of gene sets and adjusting the entropy of each gene set to be smaller than a threshold value by removing genes, one by one, from each of the plurality of gene sets; and
   adding, to each of the gene sets, genes that do not belong to any of the plurality of gene sets, provided that the entropy of each of the plurality of gene sets is equal to or less than the threshold value and a change in principal eigenvector is equal to or less than a reference value.

3. The sample analysis method according to claim 1, wherein the analysis device compares the reference kernel module and the sample kernel module, based on at least one gene group among a first gene group consisting of genes that are not present in a kernel module of the tumor tissue but are present in a kernel module of the normal tissue and a second gene group consisting of the remaining genes in the kernel module.

4. The sample analysis method according to claim 2, wherein the analysis device compares the reference kernel module and the sample kernel module, in terms of relative entropy between the first gene group and the second gene group and the degree of transformation of at least one of the first gene group and the second gene group.

5. The sample analysis method according to claim 3, wherein the analysis device classifies the reference kernel module and the sample kernel module on the basis of the relative entropy between the first gene group and the second gene group and the degree of transformation of at least one of the first gene group and the second gene group, and the analysis device calculates a log odds ratio (LOR) of each of the reference kernel module and the sample kernel module in classified groups.

6. The sample analysis method according to claim 1, wherein the analysis device compares connectivity between the reference kernel module and the other modules in the reference genomic module network with connectivity between the sample kernel module and the other modules in the sample genomic module network.

7. A sample analysis method based on a kernel module in a genomic module network, the method comprising:

by an analysis device, constructing a genomic module network, using gene expression data in which reference gene expression data and sample gene expression data are combined, based on entropy; and
by the analysis device, analyzing a sample, using a kernel module in the genomic module network,
wherein the reference gene expression data comprises at least one of a normal tissue gene expression data set and a tumor tissue gene expression data set,
the kernel module is a module that is lower in entropy by a reference value or greater than each of the other modules in the genomic module network, and
the entropy represents relations between each of a plurality of genes, based on probabilities of transcriptional states for the plurality of genes.

8. The sample analysis method according to claim 7, wherein the analysis device constructs the genomic module network including a plurality of genomic modules using the gene expression data, and
the distinguishing of the plurality of genomic modules comprises:

classifying the plurality of genes into a plurality of gene sets and adjusting the entropy of each gene set to be smaller than a threshold value by removing genes, one by one, from each of the plurality of gene sets; and
adding, to each of the plurality of gene sets, genes that do not belong to any one of the plurality of gene sets, provided that the entropy of the gene set to which the genes are to be added is equal to or smaller than the threshold value and a principal eigenvector of the gene set to which the genes are to be added is equal to or smaller than a reference value.

9. The sample analysis method according to claim 7, wherein the analysis device compares a kernel module of the reference gene expression data and a kernel module of the sample gene expression data on the basis of at least one gene group selected from among a first gene group consisting of one or more genes that are not present in a kernel module of a tumor tissue but are present in a kernel module of a normal tissue and a second gene group consisting of the remaining genes in the kernel module.

10. The sample analysis method according to claim 9, wherein the analysis device compares the kernel module of the reference gene expression data and the kernel module of the sample gene expression data on the basis of relative entropy between the first gene group and the second gene group and the degree of transformation of at least one of the first gene group and the second gene group.

11. The sample analysis method according to claim 9, wherein the analysis device classifies the reference kernel module and the sample kernel module on the basis of relative entropy between the first gene group and the second gene group and the degree of transformation of at least one of the first gene group and the second gene group and calculates a log odds ratio (LOR) for each of the reference gene expression data and the sample gene expression data that are classified.

12. The sample analysis method according to claim 7, wherein the analysis device compares the reference gene expression data and the sample gene expression data on the basis of connectivity between the kernel module and the other modules in the genomic module network.

13. An analysis device comprising:

an input device configured to receive reference data of a reference and gene expression data of a sample;
a storage device configured to store a data analysis program for analyzing data using a kernel module in a genomic module network constructed with a gene expression data set; and
a computing device configured to construct a genomic module network using the program and the gene expression data of the sample and to analyze the sample on the basis of information on genes constituting the kernel module of the constructed genomic module network,
wherein the reference data is at least one of a tumor tissue gene expression data set and a normal tissue gene expression data set, or data of a reference genomic module network constructed using the at least one of the normal tissue gene expression data set and the tumor tissue gene expression data set,
the kernel module is a module that is lower in entropy by a reference value or greater than other modules in the genomic module network, and
the entropy represents relations between each of a plurality of genes on the basis of probabilities of transcriptional states of the plurality of genes.

14. The analysis device according to claim 13, wherein the computing device compares the reference and the sample on the basis of at least one gene group selected from among a first gene group consisting of one or more genes that are present in the kernel module of the normal tissue but not in the kernel module of the tumor tissue and a second gene group consisting of the remaining genes in the kernel module.

[FIG. 1]

EP 3 970 606 A1

[FIG. 2]

| g₁ | g₂ | vector |
|---|---|---|
| 1 | 1 | $|11\rangle$ |
| 1 | 0 | $|10\rangle$ |
| 0 | 1 | $|01\rangle$ |
| 0 | 0 | $|00\rangle$ |

$|t_1\rangle$

$|t_2\rangle$

55

[FIG. 3]

[FIG. 4]

FIG. 5

[FIG. 6]

$$l_{ij} = log \frac{p_{j\backslash i}}{p_j}$$

[FIG. 7]

(A)

(B)

[FIG. 8]

FIG. 9

cutoff 4.0

cutoff 2.5

cutoff 2.0

cutoff 1.0

cutoff 1.5

cutoff 0.7

[FIG. 10]

A
BRNO ↦ CONO

B
BRNO ↦ BRCA

C
BRNO ↦ LUAD

D
BRNO ↦ LUSC

FIG. 11

FIG. 12

[FIG. 13]

[FIG. 14]

**200**

```
        ( start )
            │
            ▼
┌───────────────────────────────┐
│    input gene expression data │ ──── 210
└───────────────────────────────┘
            │
            ▼
┌───────────────────────────────┐
│        modulize genome        │ ──── 220
└───────────────────────────────┘
            │
            ▼
┌──────────────┐   250   ┌───────────────────────────────┐
│perform genome│ ◄────── │ construct intermodular network│ ──── 230
│analysis at all levels  └───────────────────────────────┘
└──────────────┘   260
            │
            ▼
┌──────────────┐         ┌───────────────────────────────┐
│perform genome│ ◄────── │    construct genetic network  │ ──── 240
│analysis at gene level  └───────────────────────────────┘
└──────────────┘
            │
            ▼
        ( end )
```

**FIG. 15**

FIG. 16

EP 3 970 606 A1

[FIG. 17]

A

B

FIG. 18

FIG. 19

[FIG. 20]

**FIG. 21**

FIG. 22

[FIG. 23]

**400**

```
┌─────────────┐
│    start    │
└─────────────┘
```

construct first genomic module network with first gene
expression data for specific tissue　　　　　　　　　　　　　410

filter first gene expression data and second gene expression data
of sample tissue, using specific module in first genomic module network　　420

construct second genomic module network with filtered first gene
expression　　　　　　　　　　　　　430

performing mapping on second gene expression data filtered based on
second genomic module network　　　　　　　　　　　　　440

perform analysis by comparing first gene expression data and
second gene expression data, using module of second genomic module
network　　　　　　　　　　　　　450

```
┌─────────────┐
│     end     │
└─────────────┘
```

FIG. 24

[FIG. 25]

$|s_j\rangle$

$|g_i\rangle$

$n$ genes

$m$ samples

gene expression data DB

gene expression data set — 610

calculate SVD for all gene expression data sets — 620

construct genomic module network — 630

calculate DVD for specific module (kernel) — 640

derive principal eigenvector — 650

U and S

$V_1$

perform filtration — 660

**600**

filtered gene expression data set — 670

**FIG. 26**

Estimated modules of Tumor Infiltrating Lymphocyte

☐ BREAST CANCER BASE MODULE DISCOVERED FROM BRX

○ GENOMIC MODULE PRESENT IN CONVENTIONAL BRCA

FIG. 27

**High MSP @ BRX#2**
**(entropy in BRCA: 0.02026 bits)**

A

**Low MSP @ BRX#2**
**(entropy in BRCA: 0.05368 bits)**

B

EP 3 970 606 A1

FIG. 28

**High MSP @ BRX#9**
**(entropy in BRCA: 0.3164 bits)**

A

**Low MSP @ BRX#9**
**(entropy in BRCA: 0.7255 bits)**

B

[FIG. 29]

[FIG. 30]

[FIG. 31]

FIG. 32

[FIG. 33]

[FIG. 34]

[FIG. 35]

[FIG. 36]

[FIG. 37]

**FIG. 38**

FIG. 39

Sample index of BRCA and BRNO

[FIG. 40]

[FIG. 41]

BN2211 module index

[FIG. 42]

[FIG. 43]

[FIG. 44]

A

B

C

D

FIG. 45

[FIG. 46]

**FIG. 47**

[FIG. 48]

[FIG. 49]

FIG. 50

[FIG. 51]

FIG. 52

EP 3 970 606 A1

**FIG. 53**

106

FIG. 54

[FIG. 55]

FIG. 56

[FIG. 57]

[FIG. 58]

**700**

start

711 — construct first genomic module network with gene expression data set of normal tissue

721 — construct second genomic module network with gene expression data set of tumor tissue

712 — determine first kernel module in first genomic module network

722 — determine second kernel module in second genomic module network

determine first gene group consisting of genes present in first kernel module but not in second kernel module and second gene group consisting of remaining genes — 730

determine transformation index on the basis first gene group and second gene group — 740

end

FIG. 59

EP 3 970 606 A1

[FIG. 60]

**900**

910
reference DB

80
reference DB

920
sample analysis

50

analysis result

930
sample analysis

[FIG. 61]

analysis device

**1000**

| storage device (1010) | interface device (1040) |
| memory unit (1020) | communication device (1050) |
| computing device (1030) | communication device (1060) |

## INTERNATIONAL SEARCH REPORT

| International application No. |
|---|
| **PCT/KR2020/006305** |

**A. CLASSIFICATION OF SUBJECT MATTER**

*A61B 5/00(2006.01)i, G16B 25/10(2019.01)i, G16B 40/00(2019.01)i, G16B 50/00(2019.01)i, G16H 50/20(2018.01)i*

According to International Patent Classification (IPC) or to both national classification and IPC

**B. FIELDS SEARCHED**

Minimum documentation searched (classification system followed by classification symbols)
A61B 5/00; C12N 15/09; C12N 5/079; G06F 19/18; G06F 19/22; G06F 19/26; G16B 5/00; G16B 25/10; G16B 40/00; G16B 50/00; G16H 50/20

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: gene expression, entropy, genome, network

**C. DOCUMENTS CONSIDERED TO BE RELEVANT**

| Category* | Citation of document, with indication, where appropriate, of the relevant passages | Relevant to claim No. |
|---|---|---|
| A | KR 10-2019-0032847 A (EWHA UNIVERSITY-INDUSTRY COLLABORATION FOUNDATION) 28 March 2019<br>See claims 1 and 2; and figure 3. | 1-14 |
| A | HAN, J. et al. The effects of variations in genomic modules on breast cancer phenotype. Animal Cells and Systems. 2014, 18.5: 296-303.<br>See pages 296 and 297; and paragraphs [2.1] and [2.2]. | 1-14 |
| A | KR 10-2017-0090093 A (INDUSTRY-ACADEMIC COOPERATION FOUNDATION, YONSEI UNIVERSITY) 07 August 2017<br>See the entire document. | 1-14 |
| A | JP 2018-181290 A (DALIAN UNIV.) 15 November 2018<br>See the entire document. | 1-14 |
| A | US 2017-0002319 A1 (WHITEHEAD INSTITUTE FOR BIOMEDICAL RESEARCH) 05 January 2017<br>See the entire document. | 1-14 |
| A | KR 10-2017-0047037 A (KOREA ADVANCED INSTITUTE OF SCIENCE AND TECHNOLOGY) 04 May 2017<br>See the entire document. | 1-14 |

☐ Further documents are listed in the continuation of Box C.  ☒ See patent family annex.

| | | |
|---|---|---|
| * Special categories of cited documents: | "T" | later document published after the international filing date or priority date and not in conflict with the application but cited to understand the principle or theory underlying the invention |
| "A" document defining the general state of the art which is not considered to be of particular relevance | | |
| "E" earlier application or patent but published on or after the international filing date | "X" | document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" document referring to an oral disclosure, use, exhibition or other means | | |
| "P" document published prior to the international filing date but later than the priority date claimed | "&" | document member of the same patent family |

| Date of the actual completion of the international search | Date of mailing of the international search report |
|---|---|
| 16 SEPTEMBER 2020 (16.09.2020) | **16 SEPTEMBER 2020 (16.09.2020)** |

| Name and mailing address of the ISA/KR | Authorized officer |
|---|---|
| Korean Intellectual Property Office<br>Government Complex Daejeon Building 4, 189, Cheongsa-ro, Seo-gu, Daejeon, 35208, Republic of Korea<br>Facsimile No. +82-42-481-8578 | Telephone No. |

Form PCT/ISA/210 (second sheet) (January 2015)

**INTERNATIONAL SEARCH REPORT**
Information on patent family members

International application No.

**PCT/KR2020/006305**

| Patent document cited in search report | Publication date | Patent family member | Publication date |
|---|---|---|---|
| KR 10-2019-0032847 A | 28/03/2019 | KR 10-2000832 B1 | 16/07/2019 |
| KR 10-2017-0090093 A | 07/08/2017 | None | |
| JP 2018-181290 A | 15/11/2018 | JP 6240804 B1 | 10/11/2017 |
| US 2017-0002319 A1 | 05/01/2017 | None | |
| KR 10-2017-0047037 A | 04/05/2017 | None | |

Form PCT/ISA/210 (patent family annex) (January 2015)